# EUROPEAN PATENT APPLICATION

(11) **EP 2 348 018 A1**
(43) Date of publication of application: **27.07.2011**
(21) Application number: 09816160.7
(22) Date of filing: 24.09.2009
(51) Int. Cl.: C07D 215/26, A61K 31/437, A61K 31/4709, A61K 31/4725, A61K 31/498, A61K 31/517, A61K 31/536, A61K 31/5377, A61K 31/538, A61P 1/00, A61P 1/16, A61P 3/04, A61P 3/06, A61P 3/10, A61P 7/02, A61P 9/04, A61P 9/10, A61P 9/12, A61P 11/00, A61P 11/02, A61P 11/06

(54) **HETEROCYCLIC BIARYL DERIVATIVE, AND PDE INHIBITOR COMPRISING SAME AS ACTIVE INGREDIENT**

(30) Priority: 25.09.2008 JP 2008246611
(71) Applicant: Kyorin Pharmaceutical Co., Ltd., Tokyo 101-8311 (JP)
(72) Inventor: KOHNO Yasushi, Shimotsuga-gun Tochigi 329-0114 (JP); SUMIYA Tatsunobu, Shimotsuga-gun Tochigi 329-0114 (JP); TAKITA Satoshi, Shimotsuga-gun Tochigi 329-0114 (JP); KOJIMA Akihiko, Shimotsuga-gun Tochigi 329-0114 (JP)
(74) Representative: Vossius & Partner
(86) International application number: PCT/JP2009/066524
(87) International publication number: WO 2010/035745

(57) **Abstract**

Novel heterocyclic biaryl derivatives were disclosed which are useful as pharmaceutical agents and which exhibit a phosphodiesterase-inhibitory action.

The heterocyclic biaryl derivatives are represented by the following general formula (1): 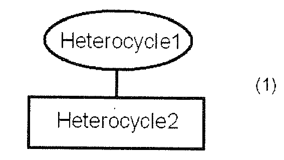 wherein the Heterocycle 1 and the Heterocycle 2 are directly bonded together.

## Description

### Technical Field:

The present invention relates to a heterocyclic biaryl derivative, or a salt or hydrate thereof, which is useful as a phosphodiesterase (PDE) inhibitor.

### Background Art

Phosphodiesterase (PDE) is an enzyme for decomposing cyclic AMP (cAMP) and cyclic GMP (cGMP) which are secondary messengers present in the living bodies. Up to now, 1 to 11 types of PDEs have been found. Which of cAMP or cGMP is decomposed or both which are decomposed is different depending on the types of PDEs. There is observed difference in the kind of tissues in which PDEs are distributed. It has been considered that the cellular reactions are controlled by various types of PDEs depending on the kinds of internal organs.

Up to now, there have been developed and known a number of PDE inhibitors. For instance, the PDE3 inhibitor has been expected to be an agent for the treatment of, for instance, angina, heart failure and hypertension or a platelet aggregation inhibitor or an antasthmatic agent; and the PDE4 inhibitor has been expected to be an agent for treating, for instance, bronchial asthma, chronic obstructive pulmonary diseases (COPD), interstitial pneumonia, interstitial cystitis, allergic conjunctivitis, allergic rhinitis, atopic dermatitis, osteoporosis, osteoarthritis of knee, rheumatoid arthritis, non-alcoholic fatty liver, multiple sclerosis, Crohn's disease, inflammatory colitis, Alzheimer's disease, dementia, Parkinson's disease, and depression.
The PDE5 inhibitor has already been clinically used as an agent for the treatment of the male impotence. Moreover, there has recently been reported that minocycline is active as a PDE10A modulator after it was tried in patients suffering from Huntington' s disease (Patent Document 1). There have also publicly been opened patent publications which disclose that the PDE10 inhibitor is effective as an agent for the treatment of a variety of mental disorders such as Huntington' s disease, Alzheimer's disease, dementia, Parkinson's disease, and schizophrenia (Patent Document 2). However, the compound disclosed in Patent Document 1 is related to a compound having, as the basic skeleton, an indoledione, while the compound disclosed in Patent Document 2 is related to a compound in which the group directly linked to the isoquinoline ring is not a hetero ring, but a phenyl group.

In addition, the pamphlet of an international publication was published in the latest day, which shows that it is also effective for obesity and metabolic syndromes (Patent Document 3). However, the compound disclosed in Patent Document 3 is related to one in which the hetero ring is linked to the isoquinoline ring through a C-N bond.
There has been reported, as a PDE inhibitor, a compound having a hetero bicyclic structure (Patent Document 4). In the compound disclosed in Patent Document 4, however, the substituent on the 2-position (located between neighboring two nitrogen atoms) of a quinazoline ring is an amino group or a nitrogen atom-containing group and therefore, the compound is different, in the structure, from that of the present invention.

In addition, there have been reported hetero bicyclic quinolone derivatives (Patent Documents 5 to 7) and hetero bicyclic quinazoline derivatives, hetero bicyclic benzoxazinone derivatives and the like (Patent Documents 8 to 11), although these compounds do not serve as PDE inhibitors.
However, the compounds disclosed in Patent Documents 5 and 6 differ, in the substituents on the Het ring, from the compound according to the present invention. Moreover, in the compound disclosed in Patent Document 7, a phosphorus atom-containing group is located at the 3-position on a quinolone ring and therefore, the former is different from the compound of the present invention.

Furthermore, in the compound disclosed in Patent Document 8, the substituent R4 corresponding to that locating at the 7-position, at which a Heterocycle 1 of the compound according to the present invention is bonded, is not a hetero ring and therefore, the former is different from the compound of the present invention.
The compound disclosed in Patent Document 9 is linked to a heteroaryl group through a nitrogen atom-containing ring and accordingly, the former differs from the compound of the present invention.
In the compound disclosed in Patent Document 10, the substituent R1 of the benzene ring constituting the quinolinone ring of the compound differs from that of the compound according to the present invention even if the basic skeleton (ZZ') of the formula 1 is a quinolinone ring.
In the compound disclosed in Patent Document 11, it is different from the compound of the present invention in the position at which the basic skeleton represented by the formula I is linked to the hetero ring corresponding to the Heterocycle 1 of the compound of the present invention, even if the basic skeleton corresponds to the Heterocycle 2.

### Prior Art References:

### Patent Document

Patent Document 1: WO 01024781 Pamphlet;
Patent Document 2: JP-A-2002-363103;
Patent Document 3: WO 2005/120514 Pamphlet;
Patent Document 4: WO 2005/087749 Pamphlet;
Patent Document 5: JP-A-63-280078;
Patent Document 6: EP 0290153;
Patent Document 7: JP-A-2001-278890;
Patent Document 8: WO 2006/015259 Pamphlet;
Patent Document 9: WO 2002/020488 Pamphlet;
Patent Document 10: WO 2007/076092 Pamphlet;
Patent Document 11: WO 2006/039718 Pamphlet

### Summary of the Invention:

### Subject to be Attained by the Invention:

It is an object of the present invention to provide a heterocyclic biaryl derivative which has an excellent phosphodiesterase inhibitory action and which shows almost no side effect.

### Means for Attaining the Subject:

The inventors of this invention have conducted intensive studies to develop a compound which has a phosphodiesterase-inhibitory activity and which is highly safe, and as a result, have found that a novel heterocyclic biaryl derivative having a structure different from those of the conventionally known PDE inhibitors shows a PDE-inhibitory action and have thus completed the present invention. More specifically, the present invention relates to:
1) A heterocyclic biaryl derivative which is represented by the following general formula (1) and in which the Heterocycle 1 and the Heterocycle 2 are directly bonded together, or an optically active derivative thereof or a pharmaceutically acceptable salt or hydrate thereof:

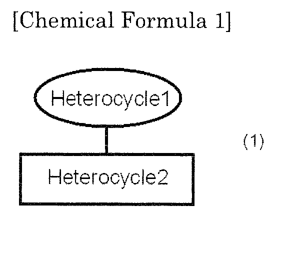

[wherein the Heterocycle 1 is a hetero ring represented by the following general formula (2):

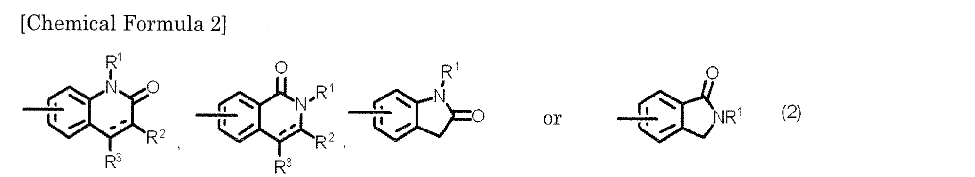

(in the formula (2), R¹ represents a hydrogen atom or an alkyl group having 1 to 6 carbon atoms which may have a substituent; R² and R³ may be the same or different and each represents a hydrogen atom, an alkyl group having 1 to 6 carbon atoms or a halogen atom; and

[Chemical Formula 3] ------

represents a single bond or a double bond); and the Heterocycle 2 is a hetero ring represented by the following general formula (3):

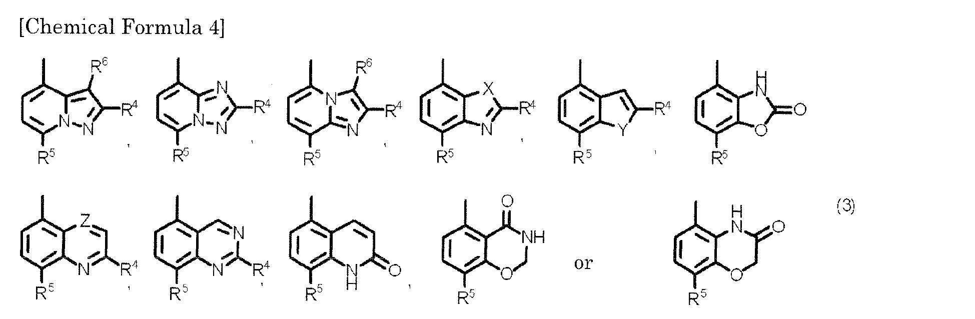

(in the formula (3), R⁴ represents a hydrogen atom, an alkyl group having 1 to 6 carbon atoms which may be substituted with a halogen atom or a cycloalkyl group having 3 to 8 carbon atoms, R⁵ represents an alkoxy group having 1 to 6 carbon atoms, an amino group or an alkylamino group having 1 to 6 carbon atoms, R⁶ represents a hydrogen atom or a halogen atom, X represents NH, O or S, Y represents O or S, and Z represents CH or N)].

2) The heterocyclic biaryl derivative, an optically active derivative thereof or a pharmaceutically acceptable salt or hydrate thereof as set forth in the foregoing item 1), wherein the Heterocycle 1 of the compound represented by the general formula (1) is a group represented by the following general formula (2a):

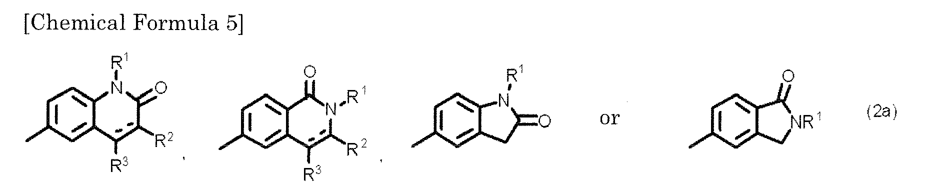

[Wherein R¹, R², R³ and

[Chemical Formula 6] **------**

are the same as those defined above].
3) The heterocyclic biaryl derivative, an optically active derivative thereof or a pharmaceutically acceptable salt or hydrate thereof as set forth in the foregoing item 1), wherein the Heterocycle 1 of the compound represented by the general formula (1) is a group represented by the following general formula:

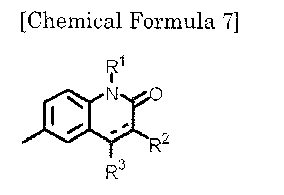

[wherein R¹, R², R³ and

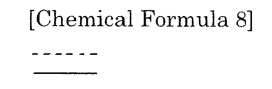

are the same as those defined above].
4) The heterocyclic biaryl derivative, an optically active derivative thereof or a pharmaceutically acceptable salt or hydrate thereof as set forth in the foregoing item 1), wherein the Heterocycle 1 of the compound represented by the general formula (1) is a group represented by the following general formula:

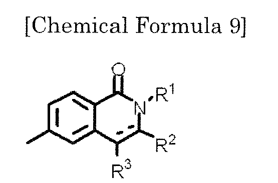

[wherein R¹, R², R³ and

[Chemical Formula 10]

------

are the same as those defined above].
5) The heterocyclic biaryl derivative, an optically active derivative thereof or a pharmaceutically acceptable salt or hydrate thereof as set forth in the foregoing item 1), wherein the Heterocycle 1 of the compound represented by the general formula (1) is a group represented by the following general formula:

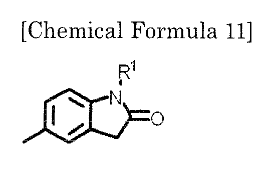

[wherein R¹ is the same as that defined above].
6) The heterocyclic biaryl derivative, an optically active derivative thereof or a pharmaceutically acceptable salt or hydrate thereof as set forth in the foregoing item 1), wherein the Heterocycle 1 of the compound represented by the general formula (1) is a group represented by the following general formula:

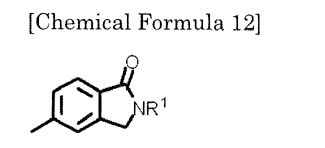

[wherein R¹ is the same as that defined above].
7) The heterocyclic biaryl derivative, an optically active derivative thereof or a pharmaceutically acceptable salt or hydrate thereof as set forth in any one of the foregoing items 1) to 6), wherein the Heterocycle 2 of the compound represented by the general formula (1) is a hetero ring represented by the following general formula (3a):

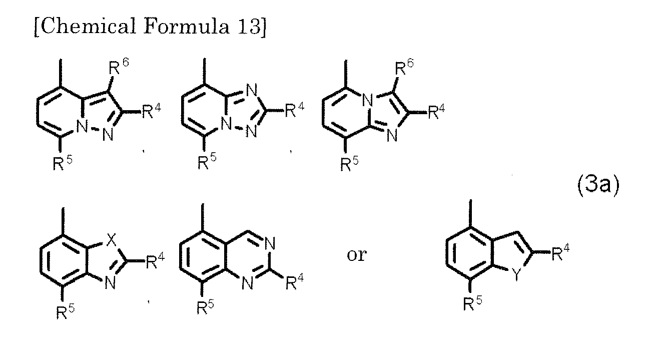

(wherein R⁴, R⁵, R⁶, X and Y are the same as those defined above).
8) The heterocyclic biaryl derivative, an optically active derivative thereof or a pharmaceutically acceptable salt or hydrate thereof as set forth in the foregoing item 1), wherein the compound of the formula (1) is one represented by the following general formula:

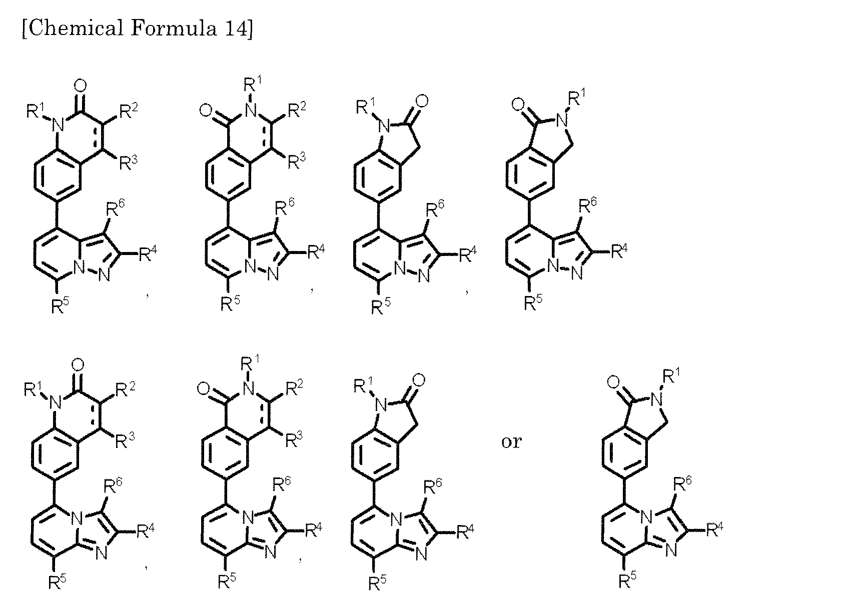

[in the formula, R¹, R², R³, R⁴, R⁵, R⁶, and

[Chemical Formula 15] - - - - - -

are the same as those defined above].
9) The heterocyclic biaryl derivative, an optically active derivative thereof or a pharmaceutically acceptable salt or hydrate thereof as set forth in any one of the foregoing items 1) to 8), wherein the substituent R⁵ of the compound represented by the general formula (1) is an alkoxy group having 1 to 6 carbon atoms.
10) The heterocyclic biaryl derivative, an optically active derivative thereof or a pharmaceutically acceptable salt or hydrate thereof as set forth in any one of the foregoing items 1) to 8), wherein the substituent R⁵ of the compound represented by the general formula (1) is an alkylamino group having 1 to 6 carbon atoms.
11) The heterocyclic biaryl derivative, an optically active derivative thereof or a pharmaceutically acceptable salt or hydrate thereof as set forth in the foregoing item 1), wherein the compound of the formula (1) is
- 6-(7-Methoxy-2-trifluoromethylpyrazolo[1,5-a]pyridin-4-yl)quinolin-2(1H)-one;
- 6-(7-Methoxy-2-trifluoromethylpyrazolo[1,5-a]pyridin-4-yl)-3,4-dihydroisoquinolin-1(2H)-one;
- 5-(7-Methoxy-2-trifluoromethylpyrazolo[1,5-a]pyridin-4-yl)lsoindolin- 1 -one;
- 4-Chloro-6-(7-methoxy-2-trifluoromethylpyrazolo[1,5-a]pyridin-4-yl)isoquinolin-1(2H)-one;

- 6-(3-Chloro-7-methoxy-2-trifluoromethylpyrazolo[1,5-a]pyridin-4-yl)-3,4-dihydro isoquinolin-1(2H)-one;
- 6-(7-Methylamino-2-trifluoromethylpyrazolo[1,5-a]pyridin-4-yl)-3,4-dihydroiso quinolin-1(2H)-one;
- 6-(7-Methylamino-2-trifluoromethylpyrazolo[1,5-a]pyridin-4-yl)quinolin-2(1H)- one;
- 6-(3-Chloro-8-methoxy-2-trifluoromethylimidazo[1,2-a]pyridin-5-yl)quinolin-2(1H)-one;
- 6-(7-Methoxy-2-trifluoromethylpyrazolo[1,5-a]pyridin-4-yl)-1-methyl-3,4-dihydroquinolin-2(1H)-one;
- 6-(3-Chloro-7-methoxy-2-trifluoromethylpyrazolo[1,5-a]pyridin-4-yl)-1-methylquinolin-2(1H)-one;

- 6-(2-Cyclopropyl-7-methoxypyrazolo[1,5-a]pyridin-4-yl)-3,4-dihydroisoquinolin-1(2H)-one;
- 6-(3-Chloro-(8-methylamino-2-trifluoromethylimidazo[1,2-a]pyridin-5-yl) quinolin-2(1H)-one; or
- 6-(3-Chloro-(8-methylamino-2-trifluoromethylimidazo[1,2-a]pyridin-5-yl)-3,4-di hydroisoquinolin-1 (2H)-one.

12) A phosphodiesterase (PDE) inhibitor comprising, as an effective component, a heterocyclic biaryl derivative, an optically active derivative thereof, or a pharmaceutically acceptable salt or hydrate thereof as set forth in any one of the foregoing items 1) to 11).
13) A pharmaceutical agent comprising, as an effective component, a heterocyclic biaryl derivative, an optically active derivative thereof, or a pharmaceutically acceptable salt or hydrate thereof as set forth in any one of the foregoing items 1) to 11).
14) The pharmaceutical agent as set forth in the foregoing item 13), wherein it is an agent for preventing or treating angina, heart failure, hypertension, bronchial asthma, chronic obstructive pulmonary diseases (COPD), interstitial pneumonia, interstitial cystitis, allergic conjunctivitis, allergic rhinitis, atopic dermatitis, osteoporosis, rheumatoid arthritis, osteoarthritis of knee, non-alcoholic fatty liver, multiple sclerosis, Crohn's disease, inflammatory colitis, Huntington's disease, Alzheimer's disease, dementia, Parkinson's disease, depression, schizophrenia, obesity and metabolic syndromes.

### Effect of the Invention:

According to the present invention, it has been found that a novel heterocyclic biaryl derivative and an addition salt thereof show excellent PDE- inhibitory action. Such a compound having a PDE-inhibitory action is useful as an agent for treating angina, heart failure and hypertension, a platelet aggregation inhibitor, or an agent for preventing or treating bronchial asthma, chronic obstructive pulmonary diseases (COPD), interstitial pneumonia, interstitial cystitis, allergic conjunctivitis, allergic rhinitis, atopic dermatitis, osteoporosis, rheumatoid arthritis, osteoarthritis of knee, non-alcoholic fatty liver, multiple sclerosis, Crohn's disease, inflammatory colitis, a variety of mental disorders such as Huntington' s disease, Alzheimer's disease, dementia, Parkinson's disease, depression and schizophrenia, obesity and metabolic syndromes as well as an agent for the treatment of the male impotence.

### Mode for Carrying out the Invention:

In the present invention, the term "alkyl group having 1 to 6 carbon atoms" means a linear or branched alkyl group having 1 to 6 carbon atoms and it preferably represents an alkyl group having 1 to 4 carbon atoms. Specific examples thereof include methyl group, ethyl group, propyl group, isopropyl group, butyl group, isobutyl group, sec-butyl group, tert-butyl group and the like.
The term "alkyl group which may be substituted with a halogen atom" herein used means an alkyl group having 1 to 6 carbon atoms which is unsubstituted or substituted with a halogen atom, preferably an alkyl group having 1 to 6 carbon atoms, in which all of the hydrogen atoms are substituted with fluorine atoms, more preferably an alkyl group having 1 to 4 carbon atoms, in which all of the hydrogen atoms are substituted with fluorine atoms, and particularly preferably a trifluoromethyl group.
The term "cycloalkyl group having 3 to 8 carbon atoms" herein used means cyclopropyl group, cyclobutyl group, cyclopentyl group, cyclohexyl group, cycloheptyl group, and cyclooctyl group, with cyclopropyl group being preferably used herein.
The term "alkoxy group having 1 to 6 carbon atoms" herein used means a linear or branched alkoxy group having 1 to 6 carbon atoms and preferably an alkoxy group having 1 to 4 carbon atoms. Specific examples thereof are methoxy group, ethoxy group, propoxyl group, isopropoxy group, butoxy group, isobutoxy group, sec-butoxy group, tert-butoxy group and the like.

The term "alkylamino group having 1 to 6 carbon atoms" herein used means a linear or branched alkylamino group having 1 to 6 carbon atoms and preferably an alkylamino group having 1 to 4 carbon atoms. Specific examples thereof include methylamino group, ethylamino group, propylamino group, isopropylamino group, butylamino group, isobutylamino group, sec-butylamino group, tert-butylamino group, dimethylamino group, methyl(ethyl)amino group and the like.
The term "halogen atom" herein used means fluorine, chlorine, bromine or iodine atoms.
The substituents for the "substituted or unsubstituted alkyl group having 1 to 6 carbon atoms" of R¹ may be alkoxycarbonyl groups each having 1 to 6 carbon atoms, carboxyl group, phenyl group, halogen atoms, alkylamino group having 1 to 6 carbon atoms, N-morpholino group or alkylammonium group whose total carbon atom number ranges from 3 to 18. Examples thereof usable herein are methoxycarbonylmethyl group, carboxymethyl group, phenylmethyl group, 3-bromopropyl group, 3-(ethylamino)propyl group, 3-(dimethylamino)propyl group, 3-morpholinopropyl group and 3-trimethylammonium propyl group.
As the pharmaceutically acceptable salts of it, there may be listed, for instance, acid-addition salts such as hydrochloride, hydrobromide, acetate, trifluoroacetate, methanesulfonate, citrate, and tartarate.
From the viewpoint of the selective inhibition of PDE4, preferred are the compounds in which the Heterocycle 2 is a hetero ring represented by the following general formula (3a):

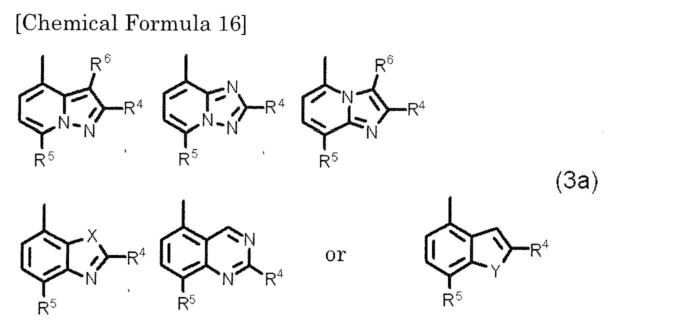

(in the formula (3a), R⁴, R⁵, R⁶, X and Y are the same as those defined above).
The compound of the present invention represented by Formula (1) can be prepared according to, for instance, the synthetic route scheme A specified below:

### [Synthetic Route Scheme A]

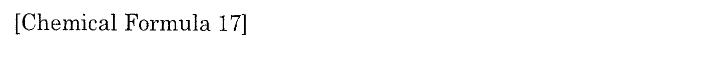

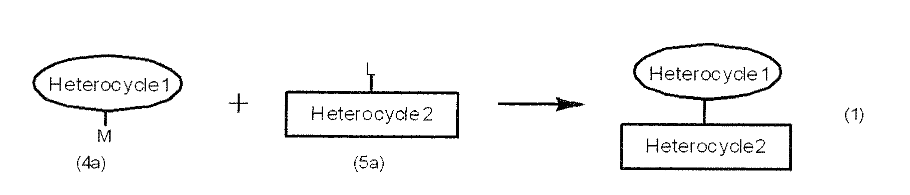

In the synthetic route scheme A, the compound of Formula (1) can be prepared by subjecting, to the Still cross-coupling reaction or the Suzuki-Miyaura cross-coupling reaction, a compound represented by the following general formula (4a) and a compound represented by the following general formula (5a):

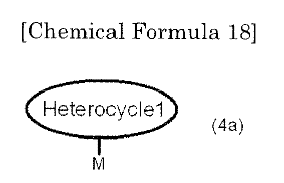

[wherein M represents -SnBu₃ (Bu represents a butyl group), -SnMe₃ (Me represents a methyl group), -B(OH)₂, -B(OMe)₂ or a group:

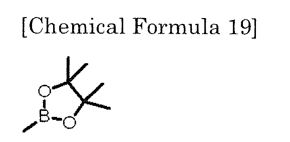

and Heterocycle 1 is the same as that described above];

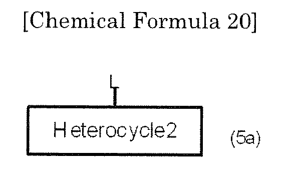

[in the formula (5a), L represents a halogen atom or a trifluoromethanesulfonyloxy group and Heterocycle 2 is the same as that described above].
In case of the Still coupling, the reaction can be carried out at a temperature ranging from ordinary temperature to the refluxing temperature of the system, while using a solvent such as toluene, N-methylpyrrolidone, dimethylformamide (DMF), tetrahydrofuran (THF) or 1,4-dioxane and likewise, optionally in the presence of a base such as diisopropylamine, using, as a catalyst, a Pd(0) complex such as tetrakis(triphenylphosphine) palladium [Pd(PPh₃)₄]. Alternatively, the compound of Formula (1) can likewise be prepared according to a method in which a ligand such as triphenylphosphine (PPh₃) or 1,1'-bis(diphenylphosphino)ferrocene (dppf) (wherein "dppf' represents bis(diphenylphosphino)ferrocene) is added to a Pd(0) complex such as bis(dibenzylideneacetone)palladium (0) [Pd(dba)₂] (wherein "dba" represents dibenzylideneacetone); or a method which makes use of a Pd(II) complex such as palladium acetate [Pd(OAc)₂], bis(acetonitrile) dichloropalladium (II) [PdCl₂(MeCN)₂], dichlorobis(triphenylphosphine)palladium (II) [PdCl₂(PPh₃)₂], [1,1'-bis(diphenylphosphino)ferrocene] dichloropalladium (II) [PdCl₂(dppf)] or the like.
In case of the Suzuki-Miyaura cross-coupling reaction which uses an organic boron compound, the reaction can be carried out at a temperature ranging from ordinary temperature to the refluxing temperature of the system, in the presence of a base such as potassium carbonate, cesium carbonate, sodium hydroxide, potassium hydroxide, potassium phosphate or diisopropylamine, while using, as a solvent, dimethoxyethane, DMF, THF, acetonitrile or 1,4-dioxane and using a palladium compound such as Pd(PPh₃)₄ as a catalyst. In this respect, it is also possible to use, as such a catalyst, those used in the foregoing Still cross-coupling reaction, in addition to Pd(PPh₃)₄.

Moreover, the compound represented by Formula (1) can also be prepared according to the following synthetic route scheme B:

### [Synthetic Route Scheme B]

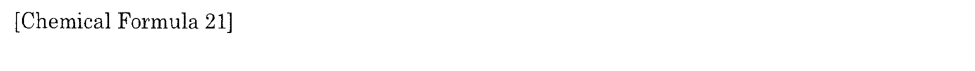

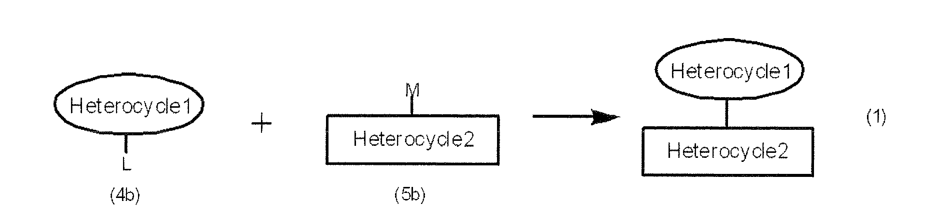

In the synthetic route scheme B, the compound of Formula (1) can be prepared by subjecting, to the Still cross-coupling reaction or the Suzuki-Miyaura cross-coupling reaction, a compound represented by the following general formula (4b) and a compound represented by the following general formula (5b):

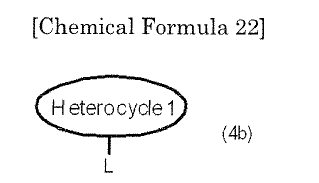

[wherein Heterocycle 1 and L are the same as those defined above];

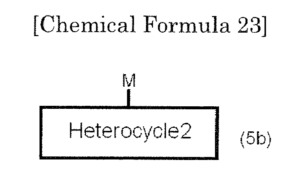

[wherein Heterocycle 2 and M are the same as those defined above].
The reaction can be carried out according to the same procedures used in the synthetic route scheme A.
In the synthetic route schemes A and B, the compounds having a pyrazolo pyridine as Heterocycle 2 among those represented by the general formula (5a) and the general formula (5b) can be prepared according to the following synthetic route scheme C or C'. In this connection, the substituents D¹ and D² will be detailed later.

### [Synthetic Route Scheme C]

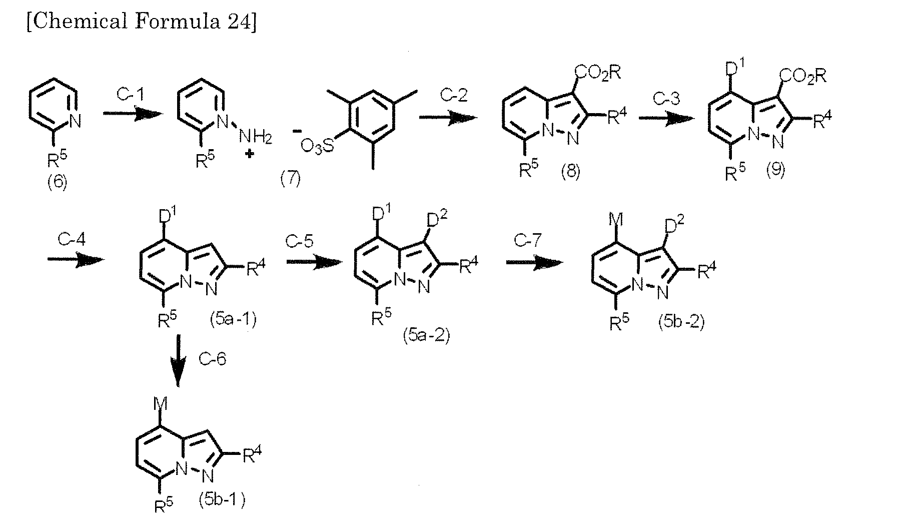

In the synthetic route scheme C, the compound represented by the general formula (7):

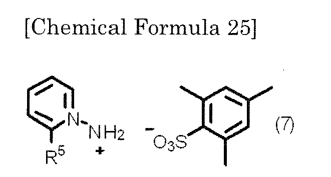

[in the formula (7), R⁵ is the same as that defined above], can be prepared by acting O-mesitylenesulfonyl hydroxylamine (hereunder referred to as "MSH") on a compound represented by the following general formula (6):

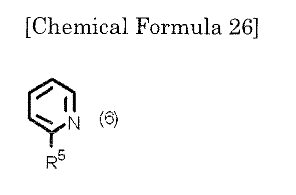

[in the formula (6), R⁵ is the same as that defined above] (Step C-1).
In the reaction, it is preferred that the compound of Formula (6) is dissolved in methylene chloride and then a solution of MSH in methylene chloride is acted on the compound of Formula (6) at a temperature ranging from 0°C to ordinary temperature.
In the synthetic route scheme C, the compound represented by the general formula (8):

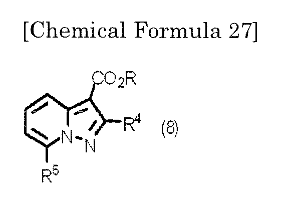

[in the formula (8), R represents an alkyl group having 1 to 6 carbon atoms or a benzyl group, R⁴ and R⁵ are the same as those defined above], can be prepared by acting a compound represented by the following general formula (10) on a compound represented by the formula (7):

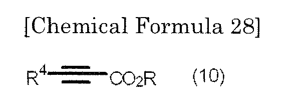

[in the formula (10), R and R⁴ are the same as those defined above], in the presence of a base (Step C-2).
The reaction can be carried out at a temperature ranging from 0°C to ordinary temperature and preferably ordinary temperature, in the presence of an inorganic base such as sodium hydrogen carbonate, sodium carbonate, potassium hydrogen carbonate or potassium carbonate, or an organic base such as triethylamine and preferably in the presence of potassium carbonate, while using a reaction solvent such as methanol, ethanol, 1,4-dioxane, dimethyl sulfoxide (DMSO), DMF, THF, toluene, benzene, cyclohexane, cyclopentene, methylene chloride, chloroform or acetonitrile and preferably DMF.
In the synthetic route scheme C, the compound represented by the following general formula (9):

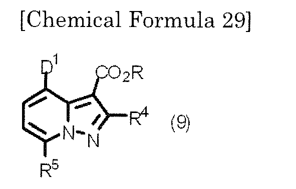

[in the formula (9), D¹ represents a halogen atom, and R, R⁴ and R⁵ are the same as those defined above], can be prepared by halogenating a compound of Formula (8) (Step C-3).
This reaction can be carried out at a temperature ranging from 0°C to 80°C, while using N-chlorosuccinimide (NCS), N-bromosuccinimide (NBS), N-iodosuccinimide (NIS), bromine or iodine and using, as a reaction solvent, acetic acid, DMF, methylene chloride, chloroform or acetonitrile, preferably acetonitrile.
In the synthetic route scheme C, the compound represented by the formula (5a-1):

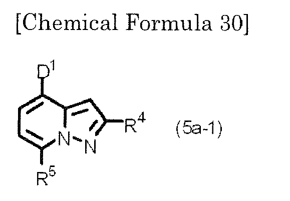

[in the formula (5a-1), R⁴, R⁵ and D¹ are the same as those defined above], can be prepared by hydrolyzing a compound represented by the formula (9) and then subjecting the resulting product to decarboxylation (Step C-4).
The reaction can be carried out in hydrobromic acid or hydrogen bromide-containing acetic acid, while refluxing the reaction system with heating. Alternatively, it is hydrolyzed, into the corresponding carboxylic acid, in methanol, ethanol, THF, DMSO, DMF or 1,4-dioxane as a solvent while acting, a sodium hydroxide aqueous solution, a potassium hydroxide aqueous solution or a lithium hydroxide aqueous solution at a temperature ranging from ordinary temperature to the refluxing temperature, and then further reacting the reaction system at a temperature ranging from 140 to 160°Cin benzene, chlorobenzene, dichlorobenzene, bromobenzene, toluene or xylene, or reacting the reaction system through the addition of a 2 to 10% sulfuric acid aqueous solution at a temperature of 100°C, in ethanol or 1,4-dioxane. The reaction can likewise be carried out by heating, with stirring, the reaction system to 100°C in a 50% sulfuric acid.
In the synthetic route scheme C, the compound represented by the formula (5a-2):

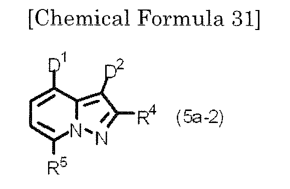

[wherein D² represents a halogen atom, and R⁴, R⁵ and D¹ are the same as those defined above], can be prepared by the halogenation of a compound of Formula (5a-1) (Step C-5).
The reaction can be carried out by the same method used in the step C-3.
In the synthetic route scheme C, the compound represented by the formula (5b-1):

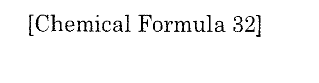

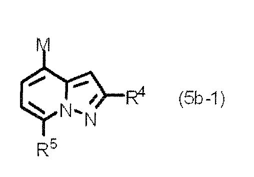

[wherein R⁴, R⁵ and M are the same as those defined above], can be prepared by converting the corresponding compound of Formula (5a-1) to a tin- or boron-containing derivative thereof (Step C-6).
When M represents -SnBu₃ or -SnMe₃, the reaction can be carried out by acting, on the compound of Formula (5a-1), an organometal reagent such as n-butyl lithium, s-butyl lithium, t-butyl lithium or isopropyl magnesium chloride in a solvent such as toluene, THF, diethyl ether or dioxane and then acting, on the resulting product, a tin-containing reagent such as tributyl tin chloride or trimethyl tin chloride at a temperature ranging from -78°C to ordinary temperature. Alternatively, it is also possible to prepare the objective compound by acting, on the compound of Formula (5a-1), bis(tributyl tin) or bis(trimethyl tin) at a temperature ranging from ordinary temperature to 140°C, in the presence of a palladium catalyst such as Pd(PPh₃)₄ or PdCl₂(dppf), in a solvent such as toluene, THF, dioxane, DMF or DMSO.

In case where M represents -B(OH)₂, the desired compound can be prepared by acting, on the corresponding compound of Formula (5a-1), an organometal reagent such as n-butyl lithium, s-butyl lithium, t-butyl lithium or isopropyl magnesium chloride, preferably n-butyl lithium, in a solvent such as toluene, THF, diethyl ether or dioxane, subjecting the resulting product to a reaction with a borane reagent such as trimethoxy borane or triisopropoxy borane at a temperature ranging from -78°C to ordinary temperature and then subjecting the product to acid hydrolysis using an acid such as hydrochloric acid. When M is -B(OMe)₂, the desired compound can be prepared by acting, on the corresponding compound of Formula (5a-1), an organometal reagent such as n-butyl lithium, s-butyl lithium, t-butyl lithium or isopropyl magnesium chloride, preferably n-butyl lithium, in a solvent such as toluene, THF, diethyl ether or dioxane, and then subjecting the resulting product to a reaction with trimethoxy borane at a temperature ranging from -78°C to ordinary temperature. When M represents a group represented by the following formula:

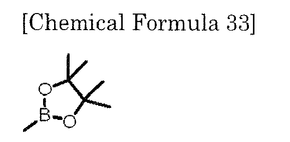

the desired compound can be prepared by acting bis(pinacolate) diboron or pinacol borane at a temperature ranging from ordinary temperature to 140°C, in a solvent such as toluene, THF, dioxane, DMF or DMSO and preferably DMSO, in the presence of a palladium catalyst such as Pd(PPh₃)₄ or PdCl₂(dppf), while using a base such as triethylamine, potassium acetate, sodium acetate or potassium 2-ethylhexanoate. In addition, when M represents a group represented by the following formula:

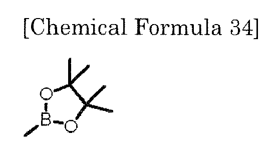

the desired compound can be prepared by acting pinacol on a compound in which M represents -B(OH)₂ or -B(OMe)₂. Moreover, when M represents -B(OH)₂, a desired compound can be prepared by subjecting a compound in which M represents a group represented by the following formula:

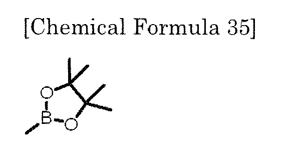

to a hydrolysis reaction using a sodium hydroxide aqueous solution or hydrochloric acid.
In the synthetic route scheme C, the compound represented by the following general formula (5b-2):

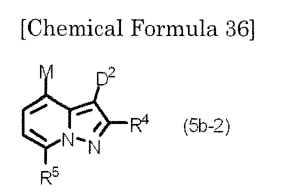

[wherein R⁴, R⁵, D² and M are the same as those defined above], can be prepared by converting the corresponding compound of Formula (5a-2) to a tin- or boron-containing derivative thereof (Step C-7).
The reaction can be carried out by the same method used in the step C-6.

### [Synthetic Route Scheme C']

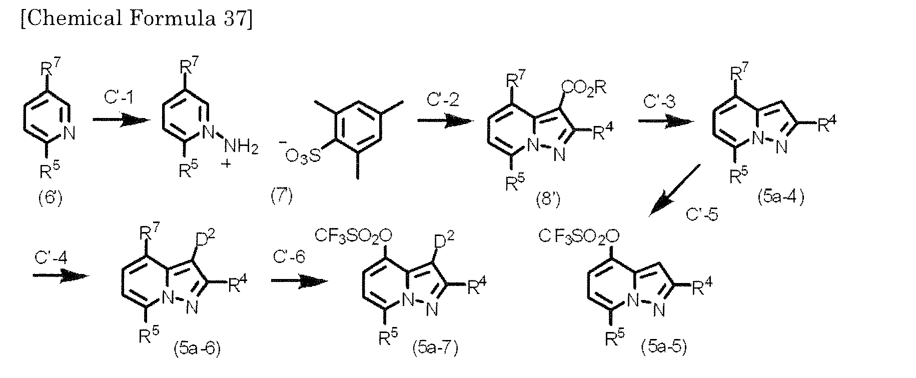

In the synthetic route scheme C', the compound represented by the following general formula (7'):

### [Chemical Formula 38]

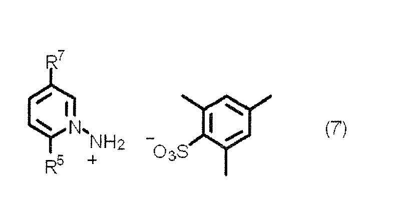

[wherein R⁷ represents a halogen atom or a hydroxyl group carrying a protecting group (P¹) (P¹ represents a protecting group such as methoxymethyl group, methoxyethoxymethyl group, tetrahydropyranyl group, benzyl group, p-methoxybenzyl group, t-butyldimethylsilyl group, t-butyldiphenylsilyl group or triisopropylsilyl group), and R⁵ is the same as that defined above], can be prepared by acting MSH on a compound represented by the following general formula (6'):

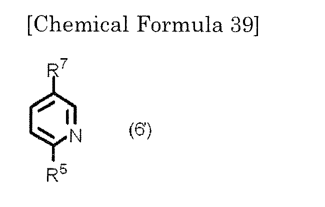

[wherein R⁵ and R⁷ are the same as those defined above] (Step C'-1).
The reaction can be carried out by the same method used in the step C-1.
In the synthetic route scheme C', the compound represented by the following general formula (8'):

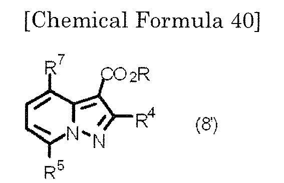

[wherein R⁴, R⁵, R⁷ and R are the same as those defined above], can be prepared by acting a compound of Formula (7') on a compound of Formula (10) in the presence of a base (Step C'-2).
The reaction can be carried out by the same method used in the step C-2.
In the synthetic route scheme C', the compound represented by the following general formula (5a-4):

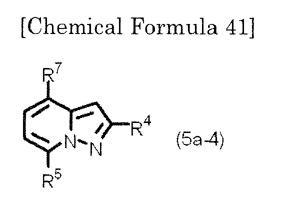

[wherein R⁴, R⁵ and R⁷ are the same as those defined above], can be prepared by hydrolyzing a compound represented by Formula (8'), followed by decarboxylation reaction (Step C'-3).
When R⁷ represents a halogen atom, the reaction can be carried out according to the same procedures used in the step C-4; when R⁷ represents a hydroxyl group carrying a protecting group, the reaction can be carried out by acting a sodium hydroxide aqueous solution, a potassium hydroxide aqueous solution or a lithium hydroxide aqueous solution, in a solvent such as methanol, ethanol, THF, DMSO, DMF or 1,4-dioxane at a temperature ranging from ordinary temperature to the reflux temperature of the reaction system to thus hydrolyze the compound into the corresponding carboxylic acid and then subjecting the system to a reaction at a temperature ranging from 80 to 160°C in a solvent such as benzene, chlorobenzene, dichlorobenzene, bromobenzene, toluene or xylene.
In the synthetic route scheme C', the compound represented by the following general formula (5a-6):

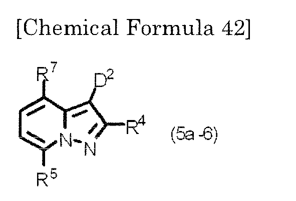

[wherein R⁴, R⁵, R⁷ and D² are the same as those defined above], can be prepared by halogenating a compound represented by Formula (5a-4) (Step C'-4).
The reaction can be carried out by the same method used in the step C-3.
In the synthetic route scheme C', the compound represented by the following general formula (5a-5):

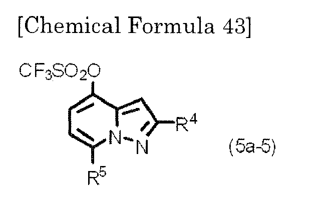

[wherein R⁴ and R⁵ are the same as those defined above], can be prepared by removing the protecting group of the hydroxyl group attached to a compound represented by Formula (5a-4) in which R⁷ represents a hydroxyl group carrying a protecting group and then replacing the resulting hydroxyl group with a trifluoro methanesulfonyl group (Step C'-5).

The deprotecting reaction can be carried out using hydrogen chloride-containing methanol, ethanol, ethyl acetate or diethyl ether at a temperature ranging from 0°C to ordinary temperature when the protecting group is a methoxymethyl group, a methoxyethoxymethyl group or a tetrahydropyranyl group. The deprotecting reaction can be carried out according to the catalytic reduction technique when the protecting group is a benzyl group or a p-methoxybenzyl group. In addition, the deprotecting reaction can be carried out by reacting a protected compound with, for instance, 2,3-dichloro-5,6-dicyano-p-benzoquinone (DDQ) or ceric ammonium nitrate (CAN) at a temperature ranging from 0°C to ordinary temperature in a solvent such as dichloromethane or acetonitrile, when the protecting group is a p-methoxybenzyl group. When the protecting group is a silyl protecting group such as a t-butyldimethylsilyl group, a t-butyldiphenylsilyl group or a triisopropylsilyl group, the deprotecting reaction can be carried out in a solvent such as acetonitrile or THF at a temperature ranging from 0°C to ordinary temperature, while using potassium fluoride, cesium fluoride or tetrabutyl ammonium fluoride.

The conversion of the hydroxyl group into trifluoromethanesulfonyl group can be carried out by acting trifluoromethanesulfonyl chloride or trifluoromethanesulfonic acid anhydride at a temperature ranging from -78°C to ordinary temperature, in the presence of a base such as triethylamine or diisopropylethylamine in a solvent such as dichloromethane.
In the synthetic route scheme C', the compound represented by the following general formula (5a-7):

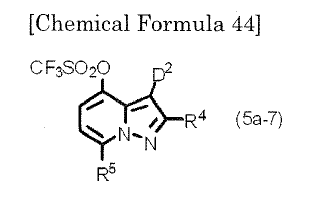

[in the formula, R⁴, R⁵ and D² are the same as those defined above], can be prepared by removing the protecting group on the hydroxyl group of a compound of Formula (5a-6) and then converting the resulting hydroxyl group into trifluoromethane sulfonyl group (Step C'-6).
The reaction can be carried out by the same method used in the step C'-5.
In the synthetic route scheme C', the compounds among those represented by the formula (5a-1) or (5a-2), in which R⁵ represents an amino group or an alkyl group having 1 to 6 carbon atoms, in other words, each of the compounds represented by the following general formula (5a-1-1) or (5a-2-1):

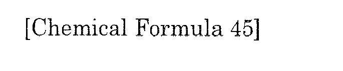

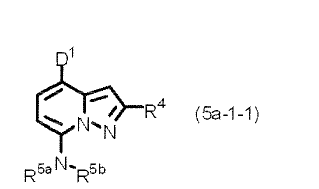

[in the formula, R^{5a} and R^{5b} each represent a hydrogen atom or an alkyl group having 1 to 6 carbon atoms, and R⁴ and D¹ are the same as those defined above], or

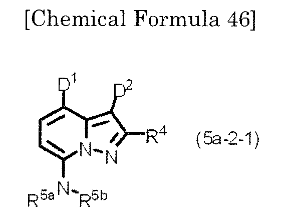

[in the formulas, R⁴, R^{5a}, R^{5b}, D¹ and D² are the same as those defined above], can be prepared according to the following synthetic route scheme C":

### [Synthetic Route Scheme C"]

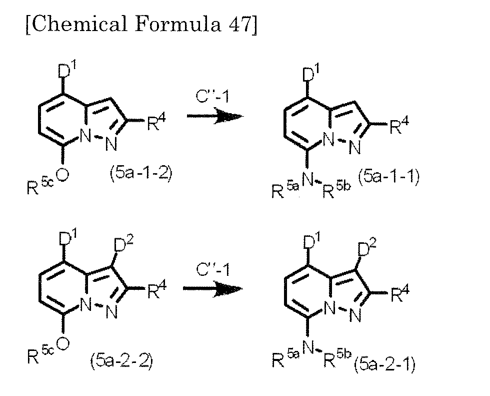

Regarding the preparation method, in the synthetic route scheme C, it can be prepared by acting, on the compounds among those represented by the formula (5a-1) or (5a-2), in which R⁵ represents an alkoxy group having 1 to 6 carbon atoms, in other words, each of the compounds represented by the following general formulas (5a-1-2) or (5a-2-2):

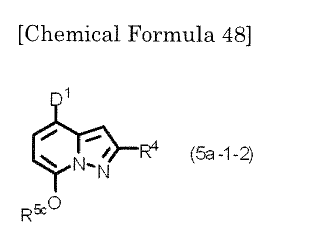

[in the formula, R^{5c} represents an alkyl group having 1 to 6 carbon atoms, and R⁴ and D¹ are the same as those defined above]; or

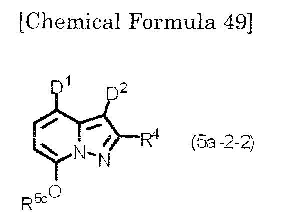

[in the formula, R⁴, R^{5c}, D¹ and D² are the same as those defined above], a compound represented by the following general formula (11):

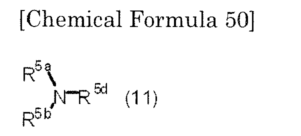

[in the formula, R^{5d} represents a hydrogen atom or a formyl group, and R^{5a} and R^{5b} are the same as those defined above] (Step C"-1).
This reaction can be carried out at a temperature ranging from ordinary temperature to 100°C, in a solvent such as THF, DMF or DMSO, in the presence or absence of a base such as lithium hydride, sodium hydride, potassium hydride, n-butyl lithium, s-butyl lithium, t-butyl lithium or potassium t-butoxide.
In the synthetic route schemes A and B, the compounds each having a triazolopyridine residue as the Heterocycle 2 moiety, among those represented by the general formula (5a) and (5b) can be prepared according to the following synthetic route scheme D:

### [Synthetic Route Scheme D]

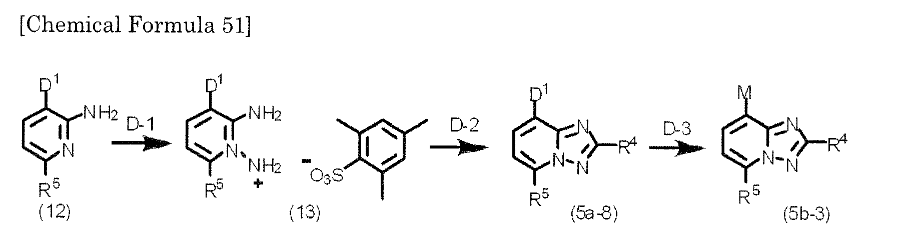

In the synthetic route scheme D, the compound represented by the following general formula (13):

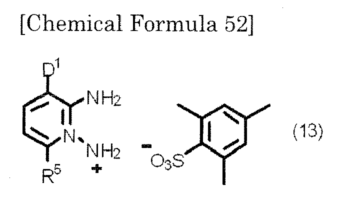

[in the formula, R⁵ and D¹ are the same as those defined above] can be prepared by acting MSH on a compound represented by the following general formula (12):

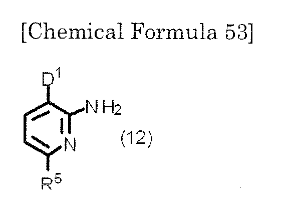

[in the formula, R⁵ and D¹ are the same as those defined above] (Step D-1).
The reaction can be carried out by the same method used in the step C-1.
In the synthetic route scheme D, the compound represented by the following general formula (5a-8):

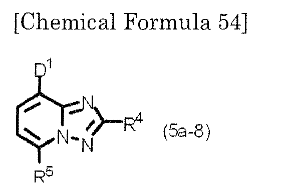

[in the formula, R⁴, R⁵ and D¹ are the same as those defined above], can be prepared by acting, on a compound represented by the formula (13), a compound represented by the following general formula (14):

[Chemical Formula 55] (R⁴CO)₂O (14)

[in the formula, R⁴ is the same as that defined above], in the presence of a base (Step D-2).
This reaction can be carried out at a temperature ranging from ordinary temperature to the refluxing temperature in the presence of a base such as triethylamine, sodium hydroxide, potassium hydroxide or potassium carbonate, preferably triethylamine while using a solvent such as benzene, toluene, xylene, methanol or ethanol.
In the synthetic route scheme D, the compound represented by the following general formula (5b-3):

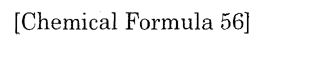

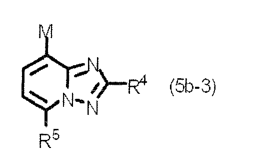

[in the formula, R⁴, R⁵ and M are the same as those defined above], can be prepared by stannylation or boration of a compound of Formula (5a-8) (Step D-3).
The reaction can be carried out by the same method used in the step C-6.
In the synthetic route schemes A and B, the compounds each having a imidazopyridine residue as the Heterocycle 2 moiety, among those represented by the general formula (5a) and (5b) can be prepared according to the following synthetic route scheme E:

### [Synthetic Route Scheme E]

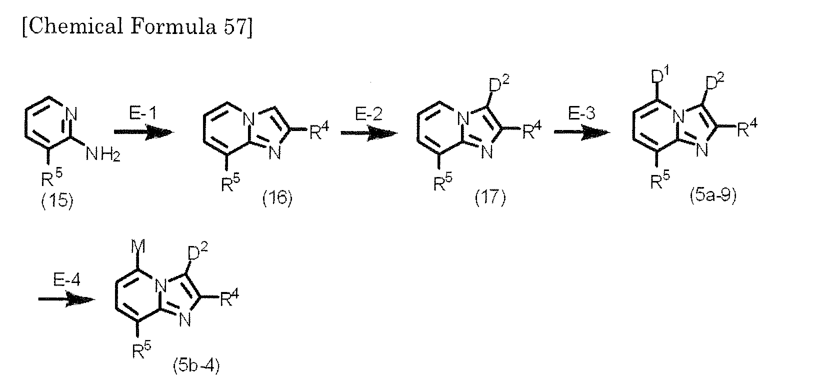

In the synthetic route scheme E, the compound represented by the following general formula (16):

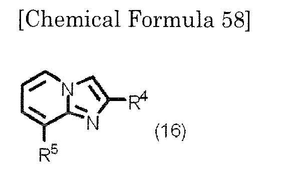

[in the formula, R⁴ and R⁵ are the same as those defined above], can be prepared by acting, on a compound represented by the following general formula (15):

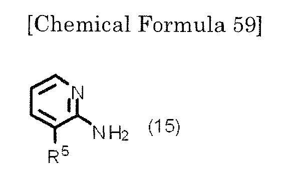

[in the formula, R⁵ is the same as that defined above], a compound represented by the following general formula (18):

[Chemical Formula 60] R⁴COCH₂X¹ (18)

[in the formula, X¹ represents a chlorine, bromine or iodine atom and R⁴ is the same as that defined above], in the presence or absence of a base (Step E-1).
This reaction can be performed at a temperature ranging from ordinary temperature to the refluxing temperature, in a solvent such as benzene, toluene, xylene, methanol or ethanol, optionally in the presence of a base such as triethylamine, sodium hydrogen carbonate, sodium carbonate, sodium hydroxide, potassium hydrogen carbonate, potassium carbonate or potassium hydroxide.
In the synthetic route scheme E, the compound represented by the following general formula (17):

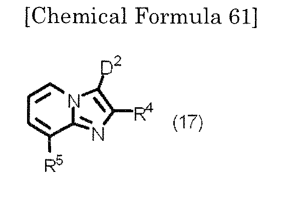

[in the formula, R⁴, R⁵ and D² are the same as those defined above], can be prepared by the halogenation of a compound represented by the general formula (16) (Step E-2).
The reaction can be carried out by the same method used in the step C-3.
In the synthetic route scheme E, the compound represented by the following general formula (5a-9):

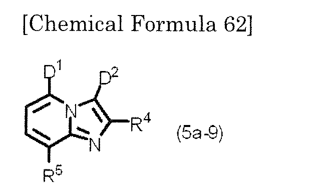

[in the formula, R⁴, R⁵, D¹ and D² are the same as those defined above], can be prepared by the halogenation of a compound represented by the general formula (17) (Step E-3).
The reaction can be carried out by the same method used in the step C-3.
In the synthetic route scheme E, the compound represented by the following general formula (5b-4):

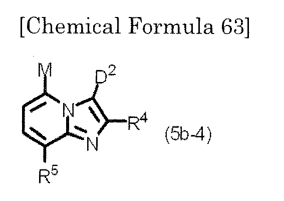

[in the formula, R⁴, R⁵, D² and M are the same as those defined above], can be prepared by stannylation or boration of the corresponding compound of Formula (5a-9) (Step E-4).
The reaction can be carried out by the same method used in the step C-6.
In the synthetic route schemes A and B, the compounds each having a benzothiazole residue as the Heterocycle 2 moiety, among those represented by the general formula (5a) and (5b) can be prepared according to the following synthetic route scheme F:

### [Synthetic Route Scheme F]

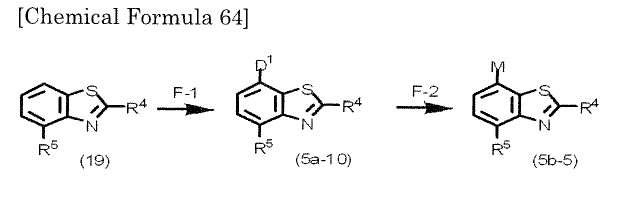

In the synthetic route scheme F, the compound represented by the following general formula (5a-10):

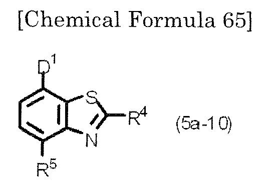

[in the formula, R⁴, R⁵ and D¹ are the same as those defined above], can be prepared by halogenating a compound represented by the following general formula (19):

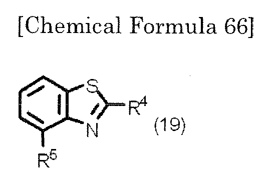

[in the formula, R⁴ and R⁵ are the same as those defined above], (Step F-1).
The reaction can be carried out by the same method used in the step C-3.
In the synthetic route scheme F, the compound represented by the following general formula (5b-5):

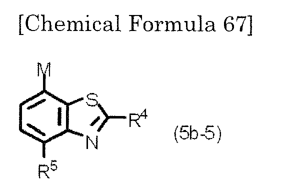

[in the formula, R⁴, R⁵ and M are the same as those defined above], can be prepared by stannylation or boration of the corresponding compound of Formula (5a-10) (Step F-2).
The reaction can be carried out by the same method used in the step C-6.
In the synthetic route schemes A and B, the compounds each having a benzofuran or benzothiophene residue as the Heterocycle 2 moiety, among those represented by the general formula (5a) and (5b) can be prepared according to the following synthetic route scheme G:

### [Synthetic Route Scheme G]

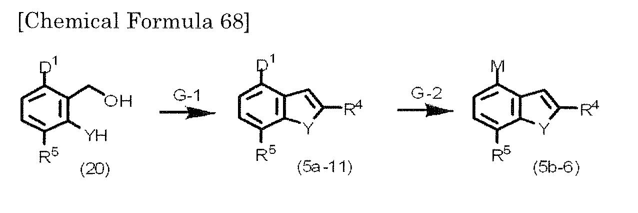

In the synthetic route scheme G, the compound represented by the following general formula (5a-11):

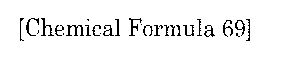

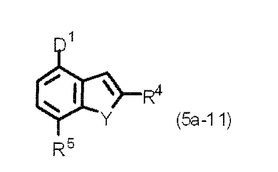

[in the formula, R⁴, R⁵, D¹ and Y are the same as those defined above], can be prepared by reacting a compound represented by the following general formula (20) with triphenylphosphonium bromide:

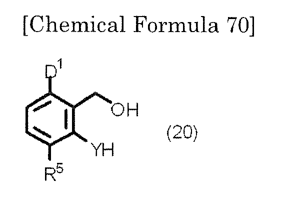

[in the formula, R⁵, D¹ and Y are the same as those defined above], and then subjecting the resulting product to a reaction with a compound of Formula (14) (Step G-1).
In this reaction, it is preferred that the compound (20) is reacted with triphenylphosphonium bromide while heating the reaction mixture with refluxing in a solvent such as acetonitrile, THF, 1,4-dioxane or ethyl acetate, preferably acetonitrile, followed by the replacement of the reaction solvent with toluene, benzene or xylene, preferably toluene, the addition of triethylamine and a compound of Formula (14), and then reacting them with heating and refluxing.
In the synthetic route scheme G, the compound represented by the following general formula (5b-6):

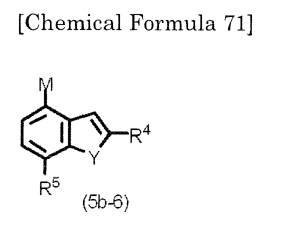

[in the formula, R⁴, R⁵, M and Y are the same as those defined above], can be prepared by stannylation or boration of the corresponding compound of Formula (5a-11) (Step G-2).
The reaction can be carried out by the same method used in the step C-6.
In the synthetic route schemes A and B, the compounds each having a benzoxazolone residue as the Heterocycle 2 moiety, among those represented by the general formula (5a) and (5b) can be prepared according to the following synthetic route scheme H:

### [Synthetic Route Scheme H]

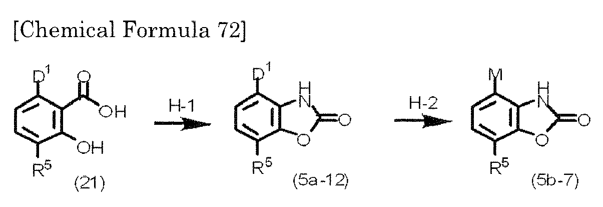

In the synthetic route scheme H, the compound represented by the following general formula (5a-12):

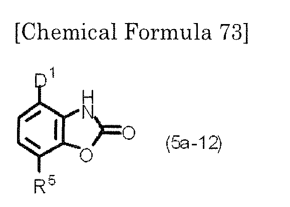

[in the formula, R⁵ and D¹ are the same as those defined above], can be prepared by the use of a compound represented by the following general formula (21), (Step H-1):

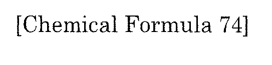

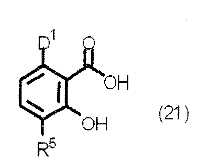

[in the formula, R⁵ and D¹ are the same as those defined above].
This reaction can be carried out by adding diphenylphosphoryl azide to the compound of Formula (21), in a solvent such as toluene or xylene and then subjecting these reagents to a reaction with each other at a temperature ranging from 50°C to 150°C in the presence of a base such as triethylamine or diisopropyl ethylamine.
In the synthetic route scheme H, the compound represented by the following general formula (5b-7):

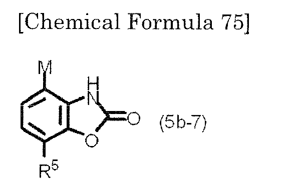

[in the formula, R⁵ and M are the same as those defined above], can be prepared by stannylation or boration of the compound of Formula (5a-12) (Step H-2).
The reaction can be carried out by the same method used in the step C-6.
In the synthetic route schemes A and B, the compounds each having a quinazoline residue as the Heterocycle 2 moiety, among those represented by the general formula (5a) and (5b) can be prepared according to the following synthetic route scheme J:

### [Synthetic Route Scheme J]

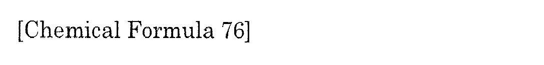

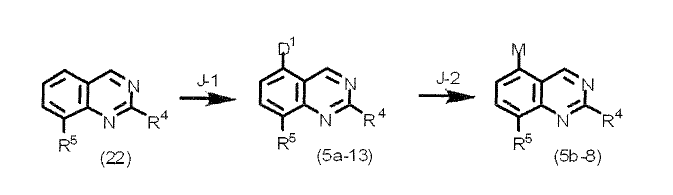

In the synthetic route scheme J, the compound represented by the following general formula (5a-13):

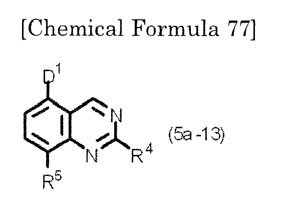

[in the formula, R⁴, R⁵ and D¹ are the same as those defined above], can be prepared by halogenating a compound represented by the following general formula (22):

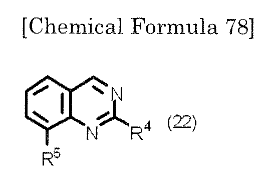

[in the formula, R⁴ and R⁵ are the same as those defined above], (Step J-1).
The reaction can be carried out by the same method used in the step C-3.
In the synthetic route scheme J, the compound represented by the following general formula (5b-8):

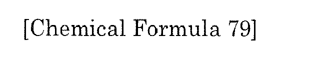

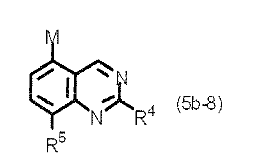

[in the formula, R⁴, R⁵ and M are the same as those defined above], can be prepared by stannylation or boration of the compound of Formula (5a-13) (Step J-2).
The reaction can be carried out by the same method used in the step C-6.
In the synthetic route schemes A and B, the compound having a 2H-benzo[e][1,3]oxazin-4(3H)-one residue as the Heterocycle 2 moiety, among those represented by the general formula (5a) and (5b) can be prepared according to the following synthetic route scheme K:

### [Synthetic Route Scheme K]

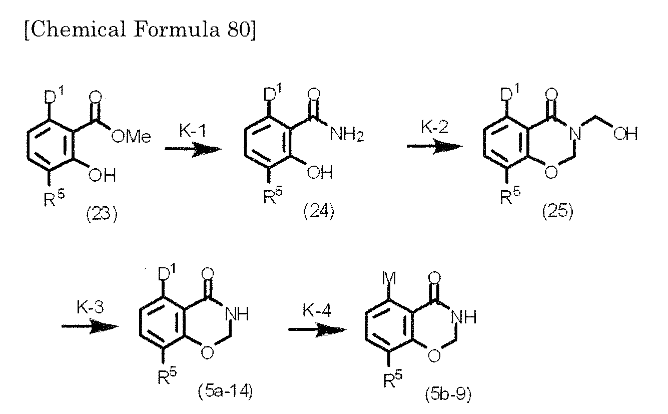

In the synthetic route scheme K, the compound represented by the following general formula (24):

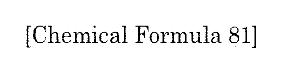

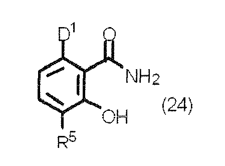

[in the formula, R⁵ and D¹ are the same as those defined above], can be prepared by converting the compound represented by the following general formula (23):

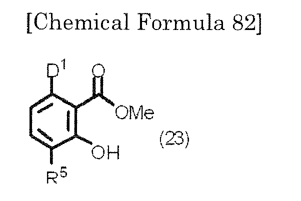

[in the formula, R⁵ and D¹ are the same as those defined above], into its amide derivative (Step K-1).
This reaction can be carried out by acting aqueous ammonia on the compound of Formula (23) at a temperature ranging from ordinary temperature to 100°C.
In the synthetic route scheme K, the compound represented by the following general formula (25):

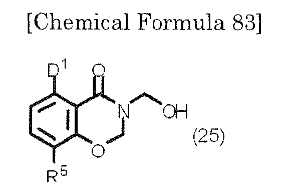

[in the formula, R⁵ and D¹ are the same as those defined above] can be prepared through the reaction of a compound of Formula (24) with formaldehyde (Step K-2).
This reaction is preferably carried out by acting an aqueous formalin solution on the compound of Formula (24) in formic acid while heating the reaction system with refluxing.
In the synthetic route scheme K, the compound represented by the following general formula (5a-14):

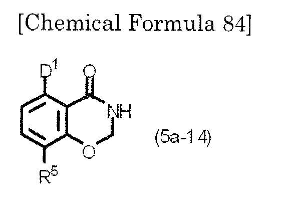

[in the formula, R⁵ and D¹ are the same as those defined above] can be prepared by subjecting a compound of Formula (25) to the treatment for the removal of its hydroxymethyl group (Step K-3).
This reaction can preferably be carried out in a solvent such as toluene or xylene, while heating the reaction system to a temperature ranging from 60°C to 150°C.
In the synthetic route scheme K, the compound represented by the following general formula (5b-9):

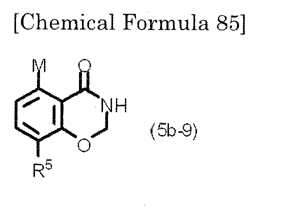

[in the formula, R⁵ and M are the same as those defined above], can be prepared by stannylation or boration of the compound of Formula (5a-14) to a tin- or boron atom-containing derivative thereof (Step K-4).
The reaction can be carried out by the same method used in the step C-6.
In the synthetic route schemes A and B, the compound having a 2H-benzo[b][1,4]oxazin-3(4H)-one residue as the Heterocycle 2 moiety, among those represented by the general formulae (5a) and (5b) can be prepared according to the following synthetic route scheme L:

### [Synthetic Route Scheme L]

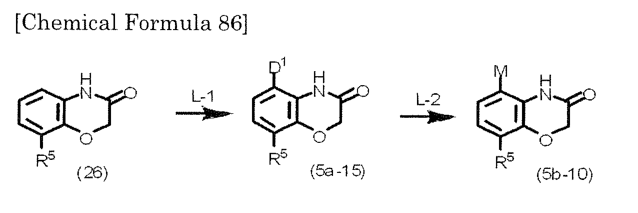

In the synthetic route scheme L, the compound represented by the following general formula (5a-15):

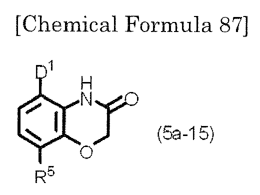

[in the formula, R⁵ and D¹ are the same as those defined above], can be prepared by halogenating a compound of the following general formula (26):

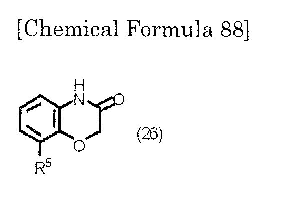

[in the formula, R⁵ is the same as that defined above], (Step L-1).
The reaction can be carried out by the same method used in the step C-3.
In the synthetic route scheme L, the compound represented by the following general formula (5b-10):

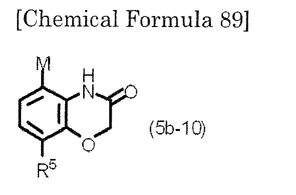

[in the formula, R⁵ and M are the same as those defined above], can be prepared by stannylation or boration the compound of Formula (5a-15) (Step L-2).
The reaction can be carried out by the same method used in the step C-6.
Moreover, among the compounds represented by the general formula (1), those represented by the following general formula (1a), in which R¹ of the Heterocycle 1 is an alkyl group having 1 to 6 carbon atoms, which may have, as a substituent, an alkoxycarbonyl group having 1 to 6 carbon atoms, a carboxyl group, a phenyl group, a halogen atom, an alkylamino group having 1 to 6 carbon atoms or an N-morpholino group:

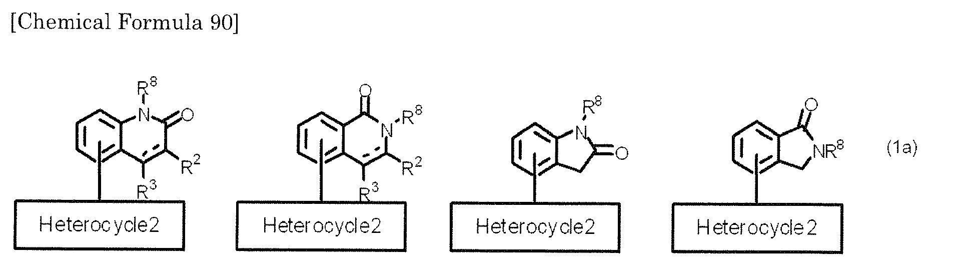

[in the formula, R⁸ represents a group: (CH₂)ₙR⁹ (wherein R⁹ represents an alkoxycarbonyl group having 1 to 6 carbon atoms, a carboxyl group, a phenyl group, a halogen atom, an alkylamino group having 1 to 6 carbon atoms or an N-morpholino group and n is an integer ranging from 1 to 6), and Heterocycle 2, R² and R³ are the same as those defined above], can likewise be prepared according to the following synthetic route scheme M:

### [Synthetic Route Scheme M]

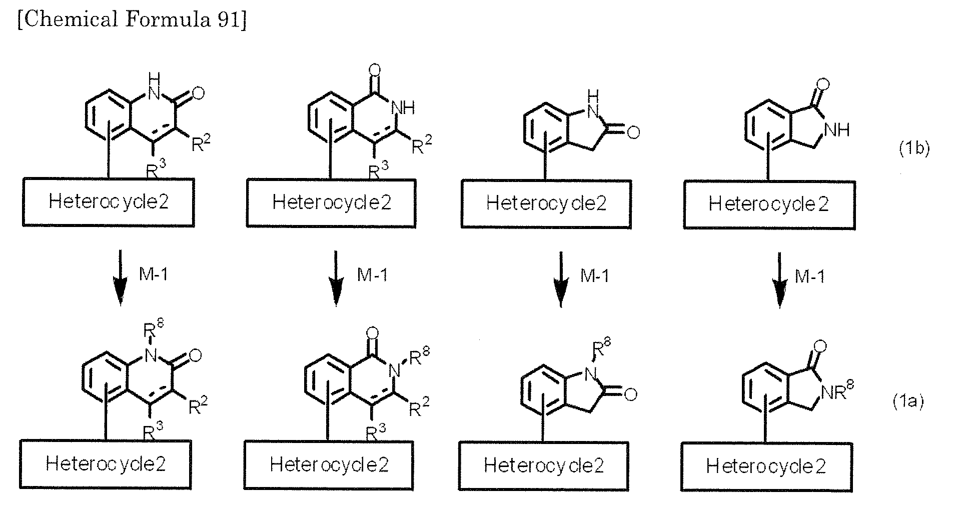

The reaction can be carried out by acting, on a compound whose substituent R¹ is a hydrogen atom and which is represented by the following general formula (1b), among those represented by Formula (1):

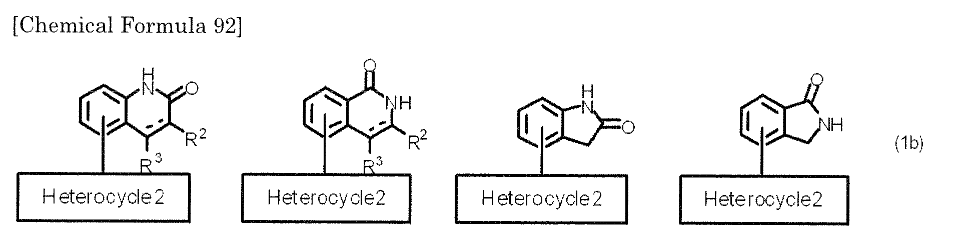

[in the formulas, Heterocycle 2, R² and R³ are the same as those defined above], a compound represented by the following general formula (27):

[Chemical Formula 93] X²(CH₂)ₙR⁹ (27)

[in the formula, X² represents a chlorine atom, a bromine atom, an iodine atom, a methanesulfonyloxy group, a trifluoromethanesulfonyloxy group, a benzene sulfonyloxy group or a p-toluenesulfonyloxy group and R⁹ and n are the same as those defined above], in the presence of a base (Step M-1).
The reaction can be carried out at a reaction temperature ranging from 0°C to 100°C, in a solvent such as toluene, THF, 1,4-dioxane, DMF, DMSO or the like, while using, as a base, lithium carbonate, sodium carbonate, potassium carbonate, lithium hydroxide, sodium hydroxide, potassium hydroxide, lithium hydride, sodium hydride, potassium hydride or the like.

When using the compound or optically active derivative or pharmaceutically acceptable salt thereof according to the present invention as pharmaceutical agents, it can be used, as need arises, in any optimum form selected from, for instance, a solid composition, a liquid composition or other compositions. The pharmaceutical agent according to the present invention can be prepared by incorporating, into the compound of the present invention, a pharmaceutically acceptable carrier. More specifically, additives such as currently used excipients, fillers, binders, disintegrating agents, coating agents, sugar-coating agents, pH-adjusting agents, solubilizing agents, or aqueous or non-aqueous solvents are added to the compound of the present invention and then the resulting mixture can be formed into any dosage form such as tablets, pills, capsules, granules, powders, triturates, liquids, emulsions, suspensions and injections according to the commonly used drug-manufacturing techniques.

The dose of the compound or optically active derivative or pharmaceutically acceptable salt thereof according to the present invention may vary depending on various factors such as the kinds of diseases and symptoms, body weight, age and sexes of patients to be treated and routes of administration, but the dose for the adult preferably ranges from about 0.01 to about 1000 mg/kg (body weight)/day and more preferably about 0.5 to about 200 mg/kg (body weight)/day for oral administration and it can be administered once a day or several times a day.

### Example

The present invention will now be described with reference to the following specific examples, but the scope of the present invention is by no means limited to those specific examples.
Incidentally, the intermediates used for the synthesis and cited in the following Examples were those disclosed in the following patent documents: WO 98/14448; JP-A-10-109988; JP-A-2006-117647; JP-A-2006-169138; WO 2006/095666; JP-A-2007-091597; JP-A-2008-024599; JP-A-2008-063265; JP-A-2008-069144; WO 2008/026687; WO 2008/029829; and WO 2008/029882.

### Example 1: N-Amino-2-amino-3-bromo-6-methoxypyridinium mesitylenesulfonate:

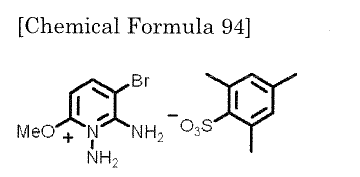

Mesitylsulfonylacetohydroxamic acid ethyl ester (25.3 g) was dissolved in 1,4-dioxane (35 mL), a 70% perchloric acid solution (13 mL) was added to the resulting solution at 0°C and then the mixture was stirred for 30 minutes. After the addition of cold water to the reaction liquid, the solids thus precipitated were collected by filtration and then dissolved in methylene chloride. After the removal of the aqueous phase through the liquid-separatory operation, the remaining methylene chloride phase was washed with saturated brine, followed by the drying of the phase over anhydrous sodium sulfate and the subsequent filtration. The filtrate was added to a solution of 2-amino-3-bromo- 6-methoxypyridine (15.0 mL) in methylene chloride (100 mL) at 0°C and the resulting mixture was stirred at ordinary temperature for one hour. The solvent was distilled off under reduced pressure, diethyl ether was added to the resulting residue and the crystals thus precipitated were collected by filtrationto give a desired product (27.3 g) as a yellow powdery product.

¹H NMR (DMSO-d₆, 400 MHz): δ 2.14 (3H, s), 2.47 (6H, s), 4.06 (3H, s), 5.73 (1H, s), 6.28 (2H, s), 6.45 (1H, d, J = 8.6 Hz), 6.71 (2H, s), 8.20 (1H, d, J = 8.6 Hz), 8.40 (2H, s).

### Example 2: N-Amino-2-methoxypyridinium mesitylenesulfonate:

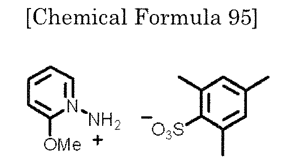

The same procedures as used in Example 1 were carried out except for using 2-methoxypyridine (81.4 g) to give a crude desired product (66.9 g). The product was used in the subsequent process without any further purification.

### Example 3: Ethyl 7-methoxy-2-trifluoromethylpyrazolo[1,5-a]pyridine-3- carboxylate:

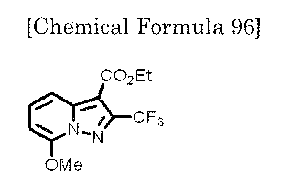

The compound prepared in Example 2 (60.1 g) and 4,4,4-trifluoro-2-butynoic acid ethyl ester (30.8 g) were dissolved in DMF (500 mL), potassium carbonate (51.1 g) was added to the solution, and the resulting mixture was stirred at ordinary temperature for 14 hours. After the solvent was distilled off under reduced pressure, the resulting residue was dissolved in ethyl acetate, the solution was filtered through Celite to remove the insoluble material, water was added to the filtrate and the mixture was extracted three times with ethyl acetate. The extracted phase was washed with saturated brine, dried over anhydrous sodium sulfate and then the solvent was distilled off under reduced pressure. The residue thus obtained was purified by the silica gel column chromatography (hexane: ethyl acetate = 10:1→1.5:1) to give a desired product (15.2 g) as a yellow powdery product.

¹H NMR (CDCl₃, 400 MHz): δ 1.42 (3H, t, J = 7.3Hz), 4.20 (3H, s), 4.42 (2H, q, J = 7.3 Hz), 6.40 (1H, d, J = 7.6 Hz), 7.51 (1H, dd, J = 8.6, 7.6 Hz), 7.91 (1H, d, J = 8.6 Hz).

### Example 4: Ethyl4-bromo-7-methoxy-2-trifluoromethylpyrazolo[1,5-a]pyridine-3-carboxylate:

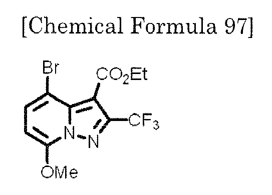

The compound (13.9 g) disclosed in Example 12 of WO 2006/095666 was dissolved in acetonitrile (400 mL), NBS (12.0 g) was added to the solution and the mixture was stirred at 70°C for 5 hours. A 10% sodium thiosulfate aqueous solution was added to the mixture, the acetonitrile was distilled off under reduced pressure, the residue thus obtained was extracted three times with ethyl acetate, the extracted phase was washed with saturated brine, then it was dried over anhydrous sodium sulfate and the solvent was distilled off under reduced pressure. The resulting residue was purified by the silica gel column chromatography (hexane: ethyl acetate = 3:1→1:1) to give a desired product (10.6 g) as a yellow powdery product.

¹H NMR (CDCl₃, 400 MHz) : δ 1.42 (3H, t, J = 7.3 Hz), 4.19 (3H, s), 4.44 (2H, q, J = 7.3 Hz), 6.25 (1H, d, J = 7.9 Hz), 7.59 (1H, d, J = 7.9 Hz).

### Example 5: 4-Bromo-7-methoxy-2-trifluoromethylpyrazolo[1,5-a]pyridine:

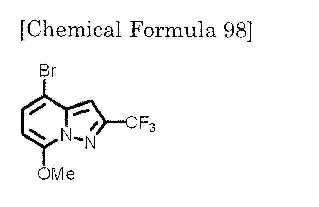

The compound (10.2 g) prepared in Example 4 was dissolved in a methanol (140 mL) and water (70 mL), potassium hydroxide (4.68 g) was added to the solution and the mixture was stirred for 3.5 hours under heated and refluxed conditions. The pH value of the mixture was adjusted to about 2 by the addition of a 1.0 mol/L hydrochloric acid solution, the mixture was then extracted three times with ethyl acetate, the extract was washed with saturated brine, dried over anhydrous sodium sulfate and then the solvent was distilled off under reduced pressure to give a crude carboxylic acid (9.34 g). The resulting carboxylic acid (9.34 g) was dissolved in ethanol (140 mL), concentrated sulfuric acid (7 mL) was added to the solution and then the mixture was stirred for 4 hours under heated and refluxed conditions. A 10% sodium hydroxide aqueous solution was added to the mixture to adjust the pH value thereof to 7 and the ethanol was distilled off under reduced pressure. Further a 10% sodium hydroxide aqueous solution was added to the mixture to adjust the pH value thereof to 8 to 9, the mixture was extracted three times with ethyl acetate, the extracted phase was washed with saturated brine and then dried over anhydrous sodium sulfate. The filtrate was passed through amination-treated silica gel, the solvent was distilled off under reduced pressure and then the residue thus obtained was purified according to the silica gel chromatography (hexane: ethyl acetate = 6:1) to give a desired product (4.54 g) as a colorless powdery product.

¹H NMR (CDCl₃, 400 MHz) : δ 4.18 (3H, s), 6.16 (1H, d, J = 8.6 Hz), 6.93 (1H, s), 7.43 (1H, d, J = 8.6 Hz).

### Example 6: Ethyl 4-bromo-2-ethyl-7-methoxypyrazolo[1,5-a]pyridine- 3-carboxylate:

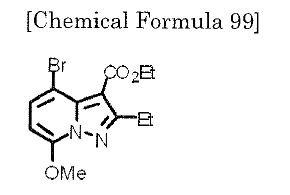

The same procedures used in Example 3 were carried out except for using the compound disclosed in Example 12 of WO 2006/095666 and 2-pentynoic acid ethyl ester to give a desired product as a yellow solid.

¹H NMR (CDCl₃, 400 MHz): δ 1.34 (3H, t, J = 7.6 Hz), 1.42 (3H, t, J = 7.3 Hz), 3.03 (2H, q, J = 7.3 Hz), 4.15 (3H, s), 4.41 (2H, q, J = 7.6 Hz), 6.09 (1H, d, J = 7.9 Hz), 7.50 (1H, d, J = 8.6 Hz).
EIMS (+) : 326 [M]⁺.

### Example 7: Benzyl 4-bromo-2-cyclopropyl-7-methoxypyrazolo[1,5-a] pyridine-3-carboxylate:

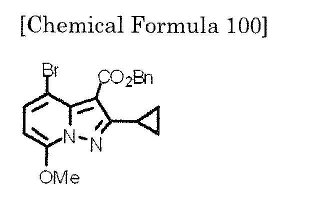

The same procedures used in Example 3 were carried out except for using the compound disclosed in Example 12 of WO 2006/095666 and 3-cyclopropylpropiolic acid benzyl ester to give a desired product as a brown oily product.
¹H NMR (CDCl₃, 400 MHz) : δ 0.94-0.99 (2H, m), 1.07-1.11 (2H, m), 2.35-2.41 (1H, m), 4.11 (3H, s), 5.42 (2H, s), 6.05 (1H, d, J = 7.9 Hz), 7.31-7.40 (3H, m), 7.44-7.50 (3H, m). CIMS (+) : 401 [M+H]⁺.

### Example 8: 4-Bromo-2-ethyl-7-methoxypyrazolo[1,5-a]pyridine

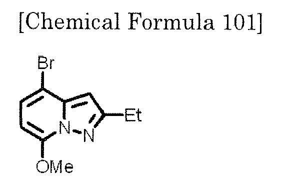

The compound (3.53 g) prepared in Example 6 was dissolved in a mixed solvent comprising dioxane (12.0 mL), methanol (6 mL) and water (9 mL), potassium hydroxide (3.64 g) was added to the solution and then the mixture was stirred for 24 hours under heated and refluxed conditions. After washing the reaction solution with ethyl acetate, the pH value of the aqueous phase was adjusted to 2 to 3 by the addition of a 1 mol/L hydrochloric acid and the aqueous phase was extracted three times with ethyl acetate. The combined extracts were dried over anhydrous sodium sulfate and the solvent was distilled off under reduced pressure to thus obtain a carboxylic acid (2.33 g) as a black solid. The carboxylic acid (2.63 g) was dissolved in ethanol (44 mL), concentrated sulfuric acid (2.20 mL) was added to the solution and then the mixture was stirred for 4.5 hours under heated and refluxed conditions. After the pH value of the reaction solution was adjusted to 7 by the addition of a 10% sodium hydroxide aqueous solution, the solvent was distilled off and the pH of the residue was controlled to 8 to 9 using a 10% sodium hydroxide aqueous solution. The aqueous solution was extracted three times with ethyl acetate, the extracted phase was washed, in order, with a saturated sodium hydrogen carbonate aqueous solution and saturated brine and then dried over anhydrous sodium sulfate. The solvent was distilled off under reduced pressure, then the resulting residue was purified by the amination-treated silica gel column chromatography (ethyl acetate) and then by the silica gel column chromatography (ethyl acetate: hexane = 1:2) to give a desired product (911 mg) as a pale yellow solid.

¹H NMR (CDCl₃, 400 MHz) : δ 1.36 (3H, t, J = 7.3 Hz), 2.90 (2H, q, J = 7.3 Hz), 4.12 (3H, s), 5.92 (1H, d, J = 8.0 Hz), 6.44 (1H, s), 7.24 (1H, d, J = 8.0 Hz).
EIMS (+) : 254 [M]⁺.

### Example 9: 4-Bromo-2-cyclopropyl-7-methoxypyrazolo[1,5-a]pyridine

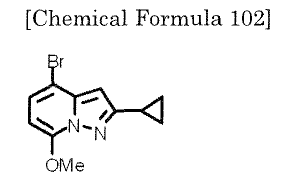

The same procedures used in Example 8 were carried out except for using the compound prepared in Example 7 to give a desired product as a pale yellow solid.

¹H NMR (CDCl₃, 400 MHz) : δ 0.86-0.90 (2H, m), 1.02-1.06 (2H, m), 2.18-2.22 (1H, m), 4.12 (3H, s), 5.90 (1H, d, J = 7.9 Hz), 6.20 (1H, s), 7.23 (1H, d, J = 7.9 Hz).
EIMS (+) : 266 [M]⁺.

### Example 10: 8-Bromo-5-methoxy-2-trifluoromethyl[1,2,4]triazolo[1,5-a]pyridine:

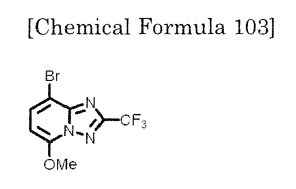

The compound (13.0 g) prepared in Example 1 was dissolved in methanol (100 mL), to the solution were added triethylamine (13.0 mL) and a mixture of trifluoroacetic anhydride (6.6 mL) and methanol (20 mL), and the resulting mixture was stirred at ordinary temperature for 17.5 hours. Water was added to the reaction solution, the resulting mixture was extracted three times with ethyl acetate, the combined extracts were washed with saturated brine and then dried over anhydrous sodium sulfate. The solvent of the filtrate was distilled off under reduced pressure and then the resulting residue was purified by the silica gel column chromatography (hexane: ethyl acetate = 1:1) to give a desired product (6.73 g) as a brown powdery product.

¹H NMR (CDCl₃, 400 MHz): δ 4.23 (3H, s), 6.41 (1H, d, J = 7.9 Hz), 7.87 (1H, d, J = 7.9 Hz).
EIMS (+) : 295 [M]⁺.

### Example 11: 7-Bromo-4-methoxy-2-trifluoromethylbenzothiazole:

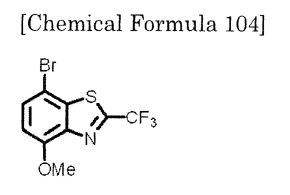

To a solution of 4-methoxy-2-trifluoromethylbenzothiazole (3.45 g) in acetic acid (25 mL), was dropwise added a 1 mol/L solution of bromine in acetic acid (15.6 mL) at ordinary temperature and then the resulting mixture was stirred at 75 °C for 6 hours. After the acetic acid was distilled off under reduced pressure, the residue was dissolved in ethyl acetate, the solution was washed with a saturated sodium hydrogen carbonate aqueous solution and then dried over anhydrous magnesium sulfate. The solvent was distilled off under reduced pressure and then the resulting residue was purified by the silica gel column chromatography (hexane: ethyl acetate = 9:1) to give a desired product (8.43 g) as a colorless powdery product.
¹H NMR (200 MHz, CDCl₃) : δ 4.06 (3H, s), 6.91 (1H, d, J = 8.5 Hz), 7.59 (1H, d, J = 8.5 Hz).

### Example 12: 3-Bromo-2-hydroxymethyl-6-methoxyphenol:

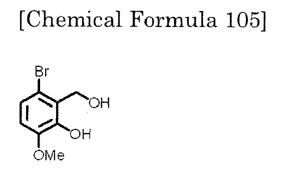

There was dissolved 6-bromo-2-hydroxy-3-methoxybenzaldehyde (1.00 g) in methanol (30 mL), and then sodium boron hydride (164 mg) was added to the solution with stirring under ice-cooled condition. After stirring the mixture at ordinary temperature for 4 hours, a dilute hydrochloric acid solution was added to the mixture and the latter was extracted with ethyl acetate. After washing the extract with water and saturated brine, it was dried over anhydrous sodium sulfate and then the solvent was distilled off under reduced pressure to give a desired product (911 mg) as a pale yellow powdery product.

¹H NMR (CDCl₃, 400 MHz): δ 3.39 (3H, s), 4.91 (2H, s), 6.27 (1H, s), 6.70 (1H, d, J = 8.6 Hz), 7.07 (1H, d, J = 8.6 Hz).

### Example 13: (6-Bromo-2-hydroxy-3-methoxyphenyl)methyltriphenyl phosphonium bromide:

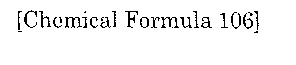

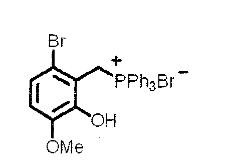

The compound (910 mg) prepared in Example 12 was dissolved in acetonitrile (10 mL), triphenyl phosphonium bromide (1.47 g) was added to the solution and then the mixture was refluxed with heating for 5 hours. The half of the solvent of the mixture was distilled off under reduced pressure, ethyl acetate (50 mL) was added to the remainder, the crystals precipitated were collected by filtration and then they were dried to give an desired product (2.20 g) as a pale yellow powdery product.

¹H NMR (DMSO-d₆, 400 MHz): δ 3.63 (3H, s), 4.81 (2H, d, J = 14.1 Hz), 6.81 (1H, dd, J = 8.6, 1.8 Hz), 6.90 (1H, dd, J = 8.6, 0.6 Hz), 7.52-7.72 (12H, m), 7.80-7.84 (3H, m), 9.80 (1H, s).

### Example 14: 4-Bromo-7-methoxy-2-trifluoromethylbenzofuran:

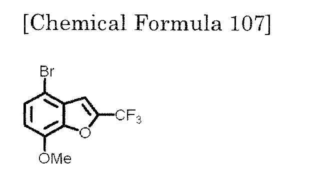

The compound (2.20 g) prepared in Example 13 was suspended in toluene (20 mL) under an argon gas atmosphere, followed by the addition of trifluoroacetic anhydride (0.612 mL) and triethylamine (1.64 mL) to the suspension and then the mixture was refluxed with heating for 5 hours. Water was added to the reaction liquid, the mixture was extracted with ethyl acetate, the extracted phase was washed with saturated brine and then dried over anhydrous sodium sulfate and the solvent was then distilled off under reduced pressure. The resulting residue was purified by the silica gel column chromatography (hexane: ethyl acetate = 10:1) to give a desired product (1.01 g) as pale yellow powder.

¹H NMR (CDCl₃, 400 MHz): δ 4.01 (3H, s), 6.82 (1H, d, J = 8.6 Hz), 7.20-7.21 (1H, m), 7.38 (1H, d, J = 8.6 Hz).

### Example 15: O-(3-Bromo-2-formyl-6-methoxy)phenyl dimethylthiocarbamate:

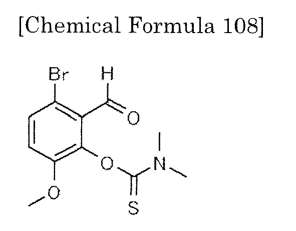

To a solution of 6-bromo-2-hydroxy-3-methoxybenzaldehyde (231 mg) in DMF (4.0 mL), were added triethylenediamine (224 mg) and dimethylthio carbamoyl chloride (247 mg) and the mixture was stirred at ordinary temperature for 12 hours. After the solvent was distilled off under reduced pressure, water was added to the resulting residue, followed by the extraction of the mixture with ethyl acetate. The extracted phase was dried over anhydrous magnesium sulfate and the residue obtained after the removal of the solvent through distillation was washed with diisopropyl ether to give a desired product (258 mg) as pale yellow powder.

¹H NMR (CDCl₃, 400 MHz): δ 3.40 (3H, s), 3.45 (3H, s), 3.86 (3H, s), 7.05 (1H, d, J = 8.6 Hz), 7.51 (1H, d, J = 8.6 Hz), 10.20 (1H, s).
EIMS (+): 317 [M]⁺.

### Example 16: S-(3-Bromo-2-formyl-6-methoxy)phenyl dimethylthiocarbamate:

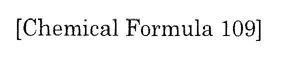

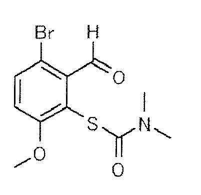

A solution of the compound (5.78 g) of Example 15 in diphenyl ether (57 mL) was stirred at 200°C for 30 minutes. The reaction liquid was cooled and then purified by the silica gel column chromatography (hexane: ethyl acetate = 1:1) to give a desired product (3.28 g) as pale brown powder.

¹H NMR (CDCl₃, 400 MHz): δ 3.00 (3H, brs), 3.16 (3H, brs), 3.89 (3H, s), 6.97 (1H, d, J = 9.2 Hz), 7.64 (1H, d, J = 9.2 Hz), 10.25 (1H, s).
EIMS (+): 317 [M]⁺.

### Example 17: (6-Bromo-2-mercapto-3-methoxy)phenylmethanol:

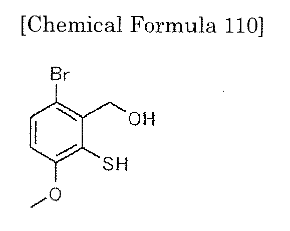

The compound (2.44 g) of Example 16 was suspended in isopropyl alcohol (60 mL), a 1 mol/L aqueous solution of sodium hydroxide (15.3 mL) was added to the suspension and the mixture was stirred at 60°C for 30 minutes. The solvent was evaporated under reduced pressure to concentrate the mixture, the latter was acidified by the addition of a 5% hydrochloric acid solution and then extracted with ethyl acetate. The extracted phase was washed with saturated brine and dried over anhydrous magnesium sulfate and then the solvent was distilled off under reduced pressure. The residue obtained was dissolved in methanol (60 mL), sodium borohydride (580 mg) was added to the solution while ice-cooling the solution and then the mixture was stirred at ordinary temperature for 30 minutes. The solvent was evaporated under reduced pressure to concentrate the mixture, the latter was acidified by the addition of a 5% hydrochloric acid solution and then extracted with ethyl acetate. The extracted phase was washed with saturated brine, dried over anhydrous magnesium sulfate and then the solvent was distilled off under reduced pressure to give a desired product (1.95 g) as a pale purple-colored oily product.

¹H NMR (CDCl₃, 400 MHz): δ 1.99 (1H, brs), 3.90 (3H, s), 4.46 (1H, s), 4.93 (2H, s), 6.71 (1H, d, J = 8.6 Hz), 7.33 (1H, d, J = 8.6 Hz).
EIMS (+): 248 [M]⁺.

### Example 18: 4-Bromo-7-methoxy-2-trifluoromethylbenzo[b]thiophene:

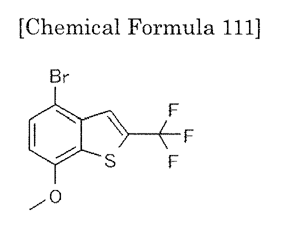

The compound (1.95 g) of Example 17 was dissolved in acetonitrile (15 mL), triphenyl phosphonium bromide (2.90 g) was added to the solution and the mixture was refluxed for 17 hours. The solvent was evaporated under reduced pressure to thus concentrate the reaction system and then the latter was washed with ethyl acetate to give a colorless powdery product (4.39 g). To the resulting solid (4.35 g), were added toluene (60 mL), trifluoroacetic anhydride (1.18 mL) and triethylamine (3.17 mL) and the mixture was refluxed for 3 hours. Water was added to the reaction liquid, the mixture was extracted with ethyl acetate, the extract was then washed with saturated brine and dried over anhydrous magnesium sulfate and the solvent was distilled off under reduced pressure. The residues thus obtained was purified by the silica gel column chromatography (hexane: ethyl acetate = 10:1) to give a desired product (2.00 g) as colorless powder.

¹H NMR (CDCl₃, 400 MHz): δ 4.00 (3H, s), 6.75 (1H, d, J = 8.6 Hz), 7.53 (1H, d, J = 8.6 Hz), 7.79 (1H, q, J = 1.2 Hz).
EIMS (+) : 310 [M]⁺.

### Example 19: 8-Methoxy-2-trifluoromethylimidazo[1,2-a]pyridine:

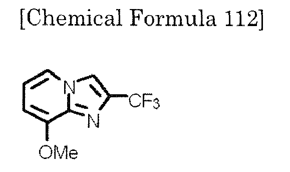

To a solution of 2-amino-3-methoxypyridine (2.17 g) in ethanol (50 mL), was added 3-bromo-1,1,1-trifluoropropan-2-one (5.00 g), and the mixture was stirred at 70°C for 8 hours. After the addition of a saturated aqueous sodium hydrogen carbonate solution (10 mL) to the reaction liquid, the solvent was distilled off under reduced pressure and then the resulting residue was extracted with ethyl acetate. The extracted phase was dried over anhydrous magnesium sulfate, the solvent was distilled off under reduced pressure and the resulting residue was purified according to the silica gel column chromatography (hexane: ethyl acetate = 1:3) to give a desired product (2.57 g) as a white solid.

¹H NMR (CDCl₃, 400 MHz): δ 4.03 (3H, s), 6.55 (1H, d, J = 7.9 Hz), 6.82 (1H, dd, J = 7.9, 6.7 Hz), 7.78 (1H, d, J = 6.7 Hz), 7.86 (1H, s).
EIMS (+): 216 [M]⁺.

### Example 20: 5-Bromo-3-chloro-8-methoxy-2-trifluoromethylimidazo[1,2-a]pyridine:

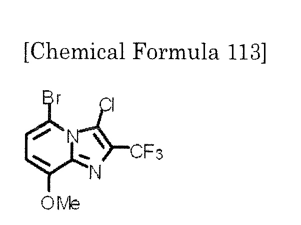

NCS (618 mg) was added to a solution of the compound of Example 19 (1.00 g) in DMF (15 mL) and this mixture was stirred at 70°C for 30 minutes. The temperature of the reaction liquid was cooled back to ordinary temperature, NBS (823 mg) was then added to the liquid and the mixture was stirred at 70°C for one hour. A saturated sodium hydrogen carbonate aqueous solution was added to the reaction liquid, the mixture was extracted with ethyl acetate, the extracted phase was washed, in order, with water and a saturated common salt aqueous solution and then dried over anhydrous magnesium sulfate. The residues obtained after the solvent was distilled off was purified according to the silica gel column chromatography (hexane: ethyl acetate = 3:2) to give a desired product (1.26 g) as pale yellow powder.

¹H NMR (CDCl₃, 400 MHz): δ 4.02 (3H, s), 6.46 (1H, d, J = 7.9 Hz), 7.05 (1H, d, J = 7.9 Hz).
CIMS (+) : 330 [M+H]⁺.

### Example 21: 4-Bromo-N-methyl-2-trifluoromethylpyrazolo[1,5-a]pyridine-7-amine:

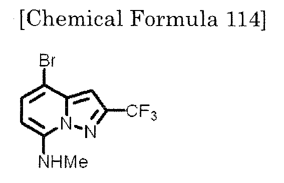

Potassium t-butoxide (1.14 g) was dissolved in DMSO (15 mL) under an argon gas atmosphere, N-methylformamide (0.596 mL) was added to the solution and the mixture was stirred at ordinary temperature for one hour. A solution of the compound of Example 5 (1.00 g) in DMSO (10 mL) was added to the reaction system at ordinary temperature and the mixture was stirred for additional 50 minutes. Ice-water was added to the reaction liquid, the insoluble material precipitated out of the liquid was collected by filtration and washed with water to give a desired product (832 mg) as colorless powder.

¹H NMR (CDCl₃, 400 MHz) : δ 3.08 (3H, d, J = 5.5 Hz), 5.87 (1H, d, J = 8.6 Hz), 5.92 (1H, brs), 6.81 (1H, s), 7.39 (1H, d, J = 8.6 Hz).
EIMS (+): 293 [M]⁺.

### Example 22: 4-Bromo-3-chloro-7-methoxy-2-trifluoromethylpyrazolo[1,5-a]pyridine:

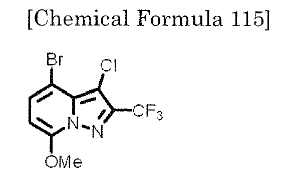

The compound of Example 5 (1.50 g) was dissolved in DMF (50 mL), NCS (1.02 g) was added to the solution and the mixture was stirred at 60°C for 3.5 hours. Water was added to the reaction liquid and the solids precipitated out of the liquid were recovered through filtration. The resulting solids were purified according to the silica gel column chromatography (hexane: ethyl acetate = 4: 1) to give a desired product (825 mg) as colorless powder.

¹H NMR (CDCl₃, 400 MHz) : δ 4.17 (3H, s), 6.14 (1H, d, J = 7.9 Hz), 7.46 (1H, d, J = 7.9 Hz).
EIMS (+): 328 [M]⁺.

### Example 23: 4-Bromo-3-chloro-N-methyl-2-trifluoromethylpyrazolo[1,5-a]pyridine-7-amine:

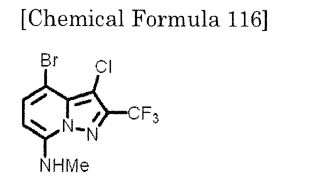

Potassium t-butoxide (842 mg) was dissolved in DMSO (13 mL) under an argon gas atmosphere, N-methylformamide (0.438 mL) was added to the solution and the mixture was stirred at ordinary temperature for one hour. A solution of the compound of Example 22 (825 mg) in DMSO (10 mL) was added to the reaction system at ordinary temperature and the mixture was stirred for additional 35 minutes. Ice-water was added to the reaction liquid, the insoluble material precipitated out of the liquid was collected by filtration to give a desired product (761 mg) as colorless powder.

¹H NMR (CDCl₃, 400 MHz) : δ 3.08 (3H, d, J = 4.9 Hz), 5.87 (1H, d, J = 7.9 Hz), 5.99 (1H, brs), 7.43 (1H, d, J = 7.9 Hz).
EIMS (+): 327 [M]⁺.

### Example 24: 5-Bromo-8-methoxyquinazoline:

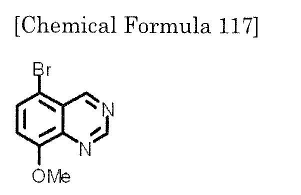

NBS (172 mg) was added to a solution of 8-methoxyquinazoline (155 mg) in DMF (3 mL) and the mixture was allowed to stand at ordinary temperature for 4 days. A 5% aqueous solution of sodium hydrogen carbonate was added to the reaction liquid, the mixture was extracted with ethyl acetate, the extracted phase was washed, in order, with water and saturated brine and then the phase was dried over anhydrous magnesium sulfate. The residue obtained after the solvent was distilled off under reduced pressure was purified according to the silica gel column chromatography (ethyl acetate) to give a desired product (181 mg) as a white solid.

¹H NMR (CDCl₃, 400 MHz) : δ 4.10 (3H, s), 7.13 (1H, d, J = 8.6 Hz), 7.80 (1H, d, J = 8.6 Hz), 9.41 (1H, s), 9.69 (1H, s).
EIMS (+): 238 [M]⁺.

### Example 25: 6-Bromo-4-chloroisoquinolin-1(2H)-one:

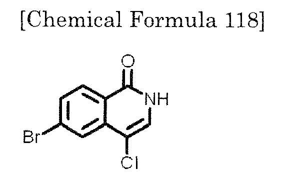

6-Bromoisoquinolone (300 mg) was dissolved in N,N-dimethylacetamide (7.0 mL), NCS (215 mg) was added to the solution and the mixture was stirred at 50°C for 50 minutes. Water was added to the reaction liquid, the insoluble material precipitated out of the liquid was collected by filtration and then washed with isopropyl alcohol to give a desired product (245 mg) as yellow powder.

¹H NMR (CDCl₃, 400 MHz): δ 7.56 (1H, d, J = 6.1 Hz), 7.78 (1H, dd, J = 8.6, 1.8 Hz), 7.92 (1H, d, J = 1.8 Hz), 8.14 (1H, d, J = 8.6 Hz), 11.72 (1H, s).
ESIMS (+): 258 [M+H]⁺.

### Examples 26 and 27: 5-Bromoquinolin-2(1H)-one and 7-bromoquinolin-2(1H)-one:

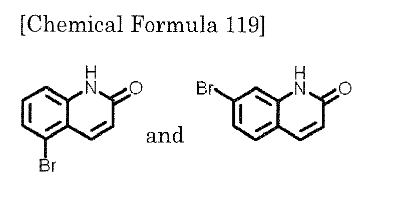

N-(3-bromophenyl)cinnamamide (5.00 g) was dissolved in monochloro benzene (150 mL), aluminum chloride (11.0 g) was added to the solution in small portions at 0°C and then the mixture was stirred at 120°C for 3 hours.
Ice-water was added to the reaction liquid, the insoluble material thus formed was collected by filtration and then washed, in order, with water and diisopropyl ether to give 5-bromoquinolin-2(1H)-one (Example 26) and 7-bromoquinolin-2(1H)-one (Example 27) in the form of a mixture (2.96 g, Ex. 26/Ex. 27 = 1:2), as red-colored powder. The mixture was used in the subsequent reaction without further purification and separation.

### Example 28: 4-Bromo-N,N-diethyl-2-methylbenzamide:

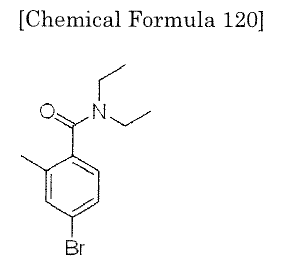

4-Bromo-2-methylbenzoic acid (2.18 g) was dissolved in thionyl chloride (7.37 mL), followed by the stirring of the solution for 3 hours under heated and refluxed conditions. The thionyl chloride was distilled off from the reaction liquid under reduced pressure to obtain a crude acid chloride. Diethylamine (5.22 mL) was dissolved in THF (50 mL) under an argon gas atmosphere, a solution of the crude acid chloride in THF (10 mL) was added to the foregoing solution at 0°C and the resulting mixture was stirred at ordinary temperature for one hour. After water was added to the reaction liquid, the mixture was extracted three times with ethyl acetate. The combined extracts were washed with saturated brine, dried over anhydrous sodium sulfate and then the solvent was distilled off under reduced pressure to thus obtain a desired product (2.64 g) as yellow powder.

¹H NMR (CDCl₃, 400 MHz): δ 1.03 (3H, t, J = 7.3 Hz), 1.25 (3H, t, J = 7.3 Hz), 2.27 (3H, s), 3.11 (2H, q, J = 7.3 Hz), 3.52 (2H, brs), 7.04 (1H, d, J = 7.9 Hz), 7.34 (1H, dd, J = 7.9, 1.8 Hz), 7.38 (1H, d, J = 1.8 Hz).
EIMS (+) : 269 [M]⁺.

### Example 29: 4-Bromo-N,N-diethyl-2-(2-(4-methylphenyl-sulfinamide)propyl) benzamide:

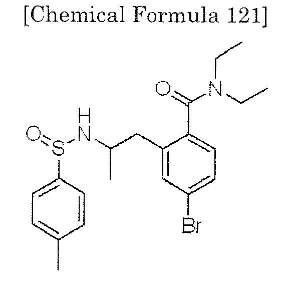

Diisopropylamine (1.87 mL) was dissolved in THF (25 mL) under an argon gas atmosphere, n-butyl lithium (a 1.58 mol/L solution in n-hexane, 7.90 mL) was added to the solution at -78°C and then the mixture was stirred at 0°C for 30 minutes to prepare a solution of LDA in THF. The compound of Example 28 (2.25 g) was dissolved in THF (20 mL) under an argon gas atmosphere, the LDA solution prepared above was added to the resulting solution at 0°C and the mixture was stirred for 30 minutes. A solution of N-ethylidene-4-methylbenzenesulfinamide (755 mg) in THF (30 mL) was added to the mixture at 0°C, followed by the stirring of the mixture for additional one hour. After an aqueous saturated solution of ammonium chloride was added to the reaction liquid, the mixture was extracted three times with ethyl acetate. The combined extracts were washed with saturated brine, dried over anhydrous sodium sulfate and the solvent was then distilled off under reduced pressure. The resulting residue was purified by the silica gel column chromatography (hexane: ethyl acetate = 1:3) to give a desired product (1.90 g) as colorless powder.

EIMS (+) : 450 [M]⁺.

### Example 30: 2-(2-Aminopropyl)-4-bromo-N,N-diethylbennzamide:

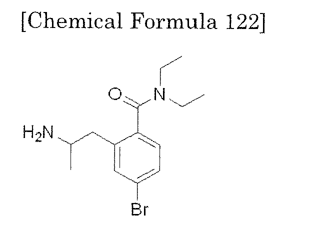

The compound of Example 29 (1.90 g) was dissolved in methanol (40 mL), trifluoroacetic acid (1.56 mL) was added to the solution and the mixture was stirred at ordinary temperature for 3.5 hours. After the solvent was distilled off, from the reaction liquid, under reduced pressure, a 10% aqueous solution of sodium hydroxide (40 mL) was added to the resulting residue, followed by the stirring of the mixture at ordinary temperature for one hour. The reaction liquid was extracted three times with ethyl acetate, the combined organic layers were washed with saturated brine, dried over anhydrous sodium sulfate and then the solvent was removed by the distillation thereof under reduced pressure to give a desired product (1.15 g) as a yellow oily product.

EIMS (+) : 312 [M]⁺.

### Example 31: 6-Bromo-3-methyl-3,4-dihydroisoquinolin-1(2H)-one:

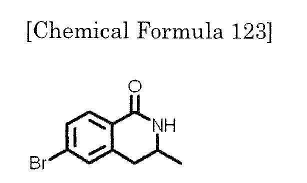

The compound of Example 30 (1.15 g) was dissolved in xylene (30 mL), p-toluenesulfonic acid monohydrate (69.8 mg) was added to the solution and the mixture was stirred for 7 hours under heated and refluxed conditions. After the solvent of the reaction liquid was distilled off under reduced pressure, water was added to the resulting residue and the mixture was extracted three times with ethyl acetate. The combined organic phase was washed with saturated brine, dried over anhydrous sodium sulfate and then the solvent was distilled off under reduced pressure. The resulting residue was washed with diisopropyl ether to give a desired product (602 mg) as yellow powder.

¹H NMR (CDCl₃, 400 MHz) : δ 1.33 (3H, d, J = 6.1 Hz), 2.79 (1H, dd, J = 15.6, 10.7 Hz), 2.92 (1H, dd, J = 15.6, 4.3 Hz), 3.84-3.87 (1H, m), 5.77 (1H, s), 7.37 (1H, s), 7.49 (1H, dd, J = 8.6, 1.5 Hz), 7.92 (1H, d, J = 8.6 Hz).
EIMS (+) : 239 [M]⁺.

### Example 32: 2-Ethyl-4-methoxy-1-(4,4,5,5-tetramethyl[1,3,2]dioxaborolan-2-yl) benzoxazole:

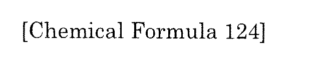

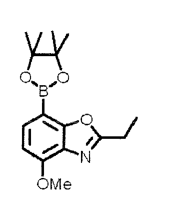

A solution, in 1,4-dioxane (2.0 mL), of 7-bromo-2-ethyl-4- methoxybenzoxazole (100 mg), bis(pinacolato) diboron (109 mg), potassium 2-ethylhexanoate (85.4 mg) and 1,1-bis(diphenylphosphino)ferrocene palladium dichloride dichloromethane complex (15.9 mg) was stirred at 80°C for 1.5 hours, under an argon gas atmosphere. After allowing the solution to cool, water was added to the reaction liquid, the mixture was extracted with ethyl acetate, the extracted phase was dried over anhydrous sodium sulfate and then the solvent was distilled off. The resulting residue was purified by the silica gel column chromatography (hexane: ethyl acetate = 3.5:1) to give a desired product (71.2 mg) as a colorless liquid.

¹H NMR (CDCl₃, 400 MHz): δ 1.37 (12H, s), 1.46 (3H, t, J = 7.6 Hz), 3.00 (2H, q, J = 7.6 Hz), 4.03 (3H, s), 6.77 (1H, d, J = 8.6 Hz), 7.67 (1H, d, J = 8.6 Hz).
EIMS (+) : 303 [M]⁺.

### Example 33: 7-Methoxy-4-(4,4,5,5-tetramethyl[1,3,2]dioxaborolan-2-yl)-2-trifluoro methylpyrazolo[1,5-a]pyridine:

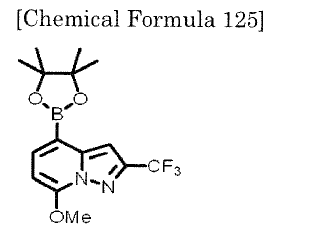

The compound of Example 5 (300 mg) was dissolved in dioxane (10 mL) under an argon gas atmosphere, there were then added, to the resultant solution, bis(pinacolato)diboron (284 mg), 1,1-bis(diphenylphosphino)ferrocene palladium dichloride dichloromethane complex (83.3 mg) and potassium 2-ethylhexanoate (279 mg), and the resulting mixture was stirred at 80°C for 3 hours. After the solvent of the reaction liquid was distilled off under reduced pressure, the resulting residue was purified by the silica gel column chromatography (hexane: ethyl acetate = 3:1) to give a desired product (300 mg) as colorless powder.

¹H NMR (DMSO-d₆, 400 MHz) : δ 1.33 (12H, s), 4.16 (3H, s), 6.66 (1H, d, J = 7.9 Hz), 7.07 (1H, s), 7.77 (1H, d, J = 7.9 Hz).

### Example 34: 5-(4,4,5,5-Tetramethyl-[1,3,2]-dioxaborolan-2-yl)-quinolin-2(1H)-one:

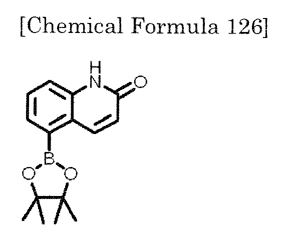

The same procedures used in Example 32 were carried out except for using 5-bromoquinolin-2(1H)-one to give a desired product as brown powder.

¹H NMR (DMSO-d₆, 400 MHz) : δ 1.32 (12H, s), 7.19-7.20 (2H, m), 7.44 (1H, t, J = 7.3 Hz), 8.04 (1H, dd, J = 7.3, 1.8 Hz), 8.30 (1H, dd, J = 7.9, 1.8 Hz), 11.27 (1H, brs).

### Example 35: 6-(4,4,5,5-Tetramethyl[1,3,2]dioxaborolan-2-yl)quinolin-2(1H)-one:

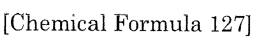

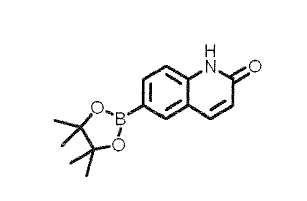

The same procedures used in Example 32 were carried out except for using 6-bromoquinolin-2(1H)-one to give a desired product as yellow powder.

¹H NMR (DMSO-d₆, 400 MHz) : δ 1.32 (12H, s), 6.62 (1H, d, J = 7.3 Hz), 7.16 (1H, dd, J = 7.3, 6.1 Hz), 7.69 (1H, d, J = 7.9 Hz), 7.98 (1H, s), 8.15 (1H, d, J = 7.9 Hz), 11.28 (1H, s).

### Example 36: 7-(4,4,5,5-Tetramethyl[1,3,2]dioxaborolan-2-yl)quinolin-2(1H)-one:

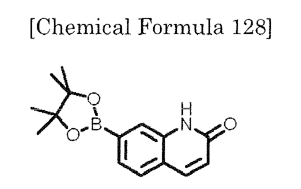

The same procedures used in Example 32 were carried out except for using 7-bromoquinolin-2(1H)-one to give a desired product as brown powder.

¹H NMR, (DMSO-d₆, 400 MHz) : δ 1.31 (14H, s), 6.54 (1H, d, J = 6.7 Hz), 7.22 (1H, t, J = 6.7 Hz), 7.61 (1H, d, J = 7.9 Hz), 7.88 (1H, dd, J = 7.9, 1.2 Hz), 8.53 (1H, s), 11.26 (1H, s).

### Example 37: 8-(4,4,5,5-Tetramethyl[1,3,2]dioxaborolan-2-yl)quinolin-2(1H)-one:

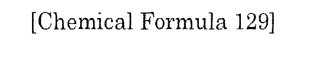

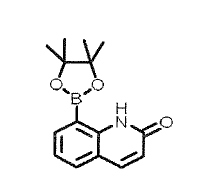

The same procedures used in Example 32 were carried out except for using 8-bromoquinolin-2(1H)-one to give a desired product which was used in the subsequent reaction without any particular purification.

### Example 38: 5-(4,4,5,5-Tetramethyl[1,3,2]dioxaborolan-2-yl)isoquinolin-1(2H)-one:

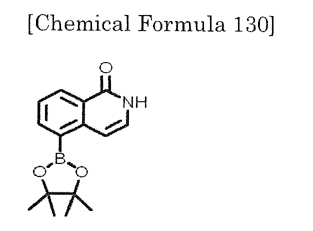

The same procedures used in Example 32 were carried out except for using 5-bromoisoquinolin-1(2H)-one to give a desired product as brown powder.

¹H NMR (DMSO-d₆, 400 MHz) : δ 1.32 (12H, s), 7.19-7.20 (2H, m), 7.44 (1H, t, J = 7.3 Hz), 8.04 (1H, dd, J = 7.3, 1.8 Hz), 8.30 (1H, dd, J = 7.9, 1.8 Hz), 11.27 (1H, brs).

### Example 39: 6-(4,4,5,5-Tetramethyl[1,3,2]dioxaborolan-2-yl)isoquinolin-1(2H)-one:

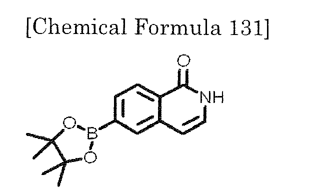

The same procedures used in Example 32 were carried out except for using 6-bromoisoquinolin-1(2H)-one to give a desired product as yellow powder.

¹H NMR (DMSO-d₆, 400 MHz) : δ 1.32 (12H, s), 6.62 (1H, d, J = 7.3 Hz), 7.16 (1H, dd, J = 7.3, 6.1 Hz), 7.69 (1H, d, J = 7.9 Hz), 7.98 (1H, s), 8.15 (1H, d, J = 7.9 Hz), 11.28 (1H, s).

### Example 40: 7-(4,4,5,5-Tetramethyl[1,3,2]dioxaborolan-2-yl)isoquinolin-1(2H)-one:

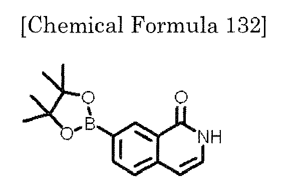

The same procedures used in Example 32 were repeated except for using 7-bromoisoquinolin-1(2H)-one to give a desired product as brown powder.

¹H NMR (DMSO-d₆, 400 MHz) : δ 1.31 (14H, s), 6.54 (1H, d, J = 6.7 Hz), 7.22 (1H, t, J = 6.7 Hz), 7.61 (1H, d, J = 7.9 Hz), 7.88 (1H, dd, J = 7.9, 1.2 Hz), 8.53 (1H, s), 11.26 (1H, s).

### Example 41: 7-(4,4,5,5-Tetramethyl[1,3,2]dioxaborolan-2-yl)-2H-benzo[b][1,4] oxazin-3(4H)-one:

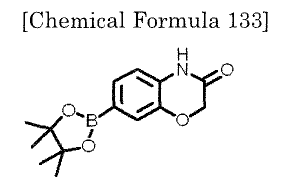

The same procedures used in Example 32 were carried out except for using 7-2H-benzo[b][1,4]-oxazin-3(4H)-one to give a desired product as a white solid.

¹H NMR (CDCl₃, 400 MHz) : δ 4.62 (2H, s), 6.81 (1H, d, J = 8.0 Hz), 7.39-7.43 (2H, m), 8.57 (1H, brs).
EIMS (+): 275 [M]⁺.

### Example 42: 6-Bromo-2-hydroxy-3-methoxybenzamide:

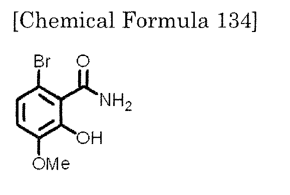

6-Bromo-2-hydroxy-3-methoxybenzoic acid methyl ester (8.30 g) was dissolved in aqueous ammonia (100 mL) and the solution was allowed to stand within a sealed tube at 70°C for 8 hours. The precipitates thus formed were collected by filtration and dissolved in water, a 1 mol/L hydrochloric acid was then added thereto to adjust the pH value thereof to 2 to 3 and the aqueous solution was extracted twice with ethyl acetate. The combined extracted layers were dried over anhydrous sodium sulfate. The solvent was distilled off under reduced pressure to give a desired product (4.34 g) as a white solid.

¹H NMR (DMSO-d₆, 400 MHz): δ 3.78 (3H, s), 6.87 (1H, d, J = 9.2 Hz), 6.95-6.98 (1H, m), 7.40 (2H, s), 9.20 (1H, s).
EIMS (+) : 245 [M]⁺.

### Example 43: 5-Bromo-3-(hydroxymethyl)-8-methoxy-2H-benzo[e][1,3]oxazin-4(3H)-one:

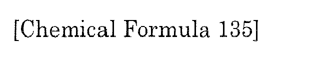

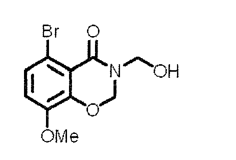

The compound of Example 42 (200 mg) was dissolved in a mixed liquid containing a 37% aqueous solution of formalin (1.0 mL) and formic acid (1.0 mL) and the resulting solution was stirred for 5 hours under heated and refluxed conditions. The reaction liquid was poured into ice-water, neutralized with a saturated sodium carbonate aqueous solution and then extracted three times with ethyl acetate. The combined extracted layers were dried over anhydrous sodium sulfate. After the solvent was distilled off under reduced pressure, the resulting residue was purified by the silica gel column chromatography (ethyl acetate: hexane = 2:1) to give a desired product (125 mg) as a white solid.

¹H NMR (CDCl₃, 400 MHz): δ 3.64 (1H, brt, J = 5.5 Hz), 3.89 (3H, s), 5.08 (2H, d, J = 5.5 Hz), 5.36 (2H, s), 6.87 (1H, d, J = 8.6 Hz), 7.30 (1H, d, J = 8.6 Hz).
EIMS (+): 287 [M]⁺.

### Example 44: 5-Bromo-8-methoxy-2H-benzo[e][1,3]oxazin-4(3H)-one

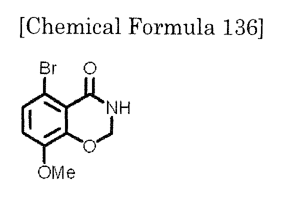

The compound of Example 43 (1.40 g) was dissolved in toluene (48.0 mL) and then the solution was stirred for 1.5 hours under heated and refluxed conditions. After the solvent was distilled off under reduced pressure, the residue was washed with diisopropyl ether to give a desired product (1.15 g) as a white solid.

¹H NMR (CDCl₃, 400 MHz) : δ 3.80 (3H, s), 5.07-5.09 (2H, m), 7.09 (1H, d, J = 8.6 Hz), 7.28 (1H, d, J = 8.6 Hz), 8.87 (1H, brt, J = 4.5 Hz).

### Example 45: 4-Bromo-7-methoxybenzo[d]oxazol-2(3H)-one:

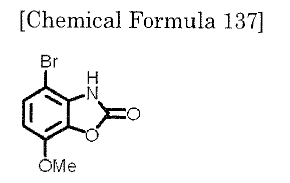

6-Bromo-2-hydroxy-3-methoxybenzoic acid (3.00 g) was dissolved in toluene (120 mL) under an argon gas atmosphere, there were then added, to the solution, diphenylphosphoryl azide (2.89 mL) and triethylamine (1.97 mL) at ordinary temperature and then the resulting mixture was stirred at 80°C for 5 hours. After the solvent was distilled off under reduced pressure, the resulting residue was washed with ethyl acetate to give a desired product (597 mg) as a white solid.

¹H NMR (DMSO-d₆, 400 MHz): δ 3.87 (3H, s), 6.79 (1H, d, J = 9.2 Hz), 7.25 (1H, d, J = 9.2 Hz), 12.10 (1H, s).
EIMS (+): 243 [M]⁺.

### Example 46: 5-Bromo-8-methoxy-2H-benzo[b][1,4]oxazin-3(4H)-one:

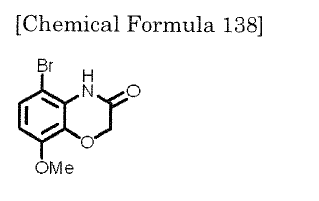

8-Methoxy-2H-benzo[b][1,4]oxazin-3(4H)-one (100 mg) was dissolved in DMF (5.5 mL) under an argon gas atmosphere, NBS (119 mg) was added to the solution at ordinary temperature and the resulting mixture was stirred at that temperature for 1.5 hours. To the reaction liquid, was added a saturated aqueous solution of sodium thiosulfate and the mixture was then extracted twice with ethyl acetate. The combined extracts were washed, in order, with water and saturated brine and then dried over anhydrous sodium sulfate. After the solvent was distilled off under reduced pressure, the resulting residue was purified according to the silica gel column chromatography (ethyl acetate: hexane = 1:2) to give a desired product (59.8 mg) as a white solid.

¹H NMR (CDCl₃, 400 MHz) : δ 3.09 (3H, s), 4.66 (2H, s), 6.57 (1H, d, J = 9.2 Hz), 7.14 (1H, d, J = 9.2 Hz), 7.79 (1H. brs).
EIMS (+): 257 [M]⁺.

### Example 47: 8-(7-Methoxy-2-trifluoromethylpyrazolo[1,5-a]pyridin-4-yl)quinolin-2(1H)-one:

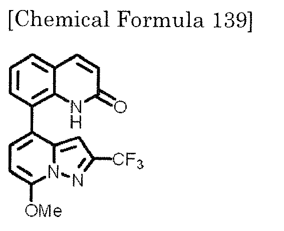

The compound of Example 37 (370 mg) and the compound of Example 5 (200 mg) were dissolved in dimethoxyethane (10 mL) under an argon gas atmosphere, there were then added, to the resulting solution, tetrakis(triphenylphosphine) palladium (103 mg) and a 2.0 mol/L aqueous solution of sodium carbonate (5.0 mL) and then the resulting mixture was stirred at 80°C for 6 hours. After the addition of water to the reaction liquid, the insoluble material precipitated from the liquid was filtered off through Celite and the filtrate was extracted three times with ethyl acetate. The combined extracts were washed with saturated brine and then dried over anhydrous sodium sulfate. After the solvent of the filtrate was distilled off under reduced pressure, the resulting residue was purified according to the silica gel chromatography (ethyl acetate) and then washed with diisopropyl ether to give a desired product (76.8 mg) as yellow powder.

¹H NMR (DMSO-d₆, 400 MHz) : δ 4.39 (3H, s), 6.68 (1H, s), 6.70 (1H, d, J = 9.2 Hz), 6.92 (1H, d, J = 7.9 Hz), 7.49 (1H, t, J = 7.6 Hz), 7.57 (1H, d, J = 7.3 Hz), 7.69 (1H, dd, J = 7.3, 1.2 Hz), 7.97 (1H, dd, J = 7.3, 1.2 Hz), 8.19 (1H, d, J = 9.2 Hz), 10.85 (1H, s).
HREIMS (+): 359.0915 (calculated for C₁₈H₁₂F₃N₃O₂: 359.0882).
Elemental Analysis: Found: C 59.97%, H 3.47%, N 11.25%; Calculated (for C₁₈H₁₂F₃N₃O₂ · 1/10 H₂O): C 59.87%, H 3.41%, N 11.64%.

### Examples 48 and 49: 5-(7-Methoxy-2-trifluoromethylpyrazolo[1,5-a]pyridin-4-yl) quinolin-2(1H)-one and 7-(7-methoxy-2-trifluoromethylpyrazolo[1,5-a]pyridin-4-yl) quinolin-2(1H)-one:

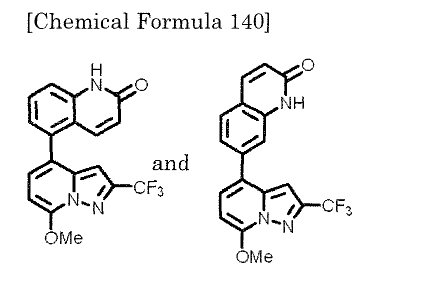

The mixture obtained in Examples 26 and 27 (500 mg, Ex.26:Ex.27 = 1:2) was dissolved in dioxane (20 mL) under an argon gas atmosphere, to the resulting solution, were added bis(pinacolato)diboron (623 mg), 1,1-bis(diphenylphosphino) ferrocene palladium dichloride dichloromethane complex (182 mg) and potassium 2-ethylhexanoate (610 mg) and the resulting mixture was stirred at 80°C for 1.5 hours. The reaction liquid was treated according to the silica gel column chromatography (hexane: ethyl acetate = 1:2) to give a mixture of crude borates (371 mg) corresponding to the mixture of Examples 26 and 27 as a brown oily product. The crude borate mixture (371 mg) and the compound of Example 5 (282 mg) were dissolved in 1,4-dioxane (12 mL) under an argon gas atmosphere, to this solution, were added tetrakis(triphenylphosphine) palladium (146 mg) and a 2.0 mol/L aqueous solution of sodium carbonate (6.3 mL) and the mixture was stirred at 100°C for 7.5 hours. After water was added to the reaction liquid, the insoluble material precipitated out of the liquid was filtered off through Celite and the filtrate was extracted three times with ethyl acetate. The combined extracts were washed with saturated brine and then dried over anhydrous sodium sulfate. After the solvent of the filtrate was distilled off under reduced pressure, the resulting residue was purified by the silica gel column chromatography (ethyl acetate ∼ ethyl acetate:methanol = 20:1) to give a mixture of desired products (236 mg) as yellow powder. This mixture (236 mg) was dissolved in acetonitrile (6.0 mL), followed by the addition of Boc₂O (157 mg) and N,N-dimethylaminopyridine (7.3 mg) to the solution and the mixture was stirred at ordinary temperature for one hour.

Water was added to the reaction liquid and then the mixture was extracted three times with ethyl acetate. The combined extracts were washed with saturated brine and then dried over anhydrous sodium sulfate. The solvent of the filtrate obtained after the filtration of the combined extract was distilled off under reduced pressure and the resulting residue was purified by the silica gel column chromatography (hexane: ethyl acetate = 2:1) to give a Boc-protected compound of Example 48 (94.4 mg) as colorless powder,

¹H NMR (CDCl₃, 400 MHz) : δ 1.59 (9H, s), 4.27 (3H, s), 6.42 (1H, d, J = 7.9 Hz), 6.46 (1H, s), 7.19 (1H, d, J = 8.6 Hz), 7.28 (1H, d, J = 7.3 Hz), 7.61 (1H, dd, J = 7.3, 1.2 Hz), 7.81 (1H, dd, J = 8.6, 7.3 Hz), 8.07 (1H, d, J = 8.6 Hz), 8.12 (1H, dd, J = 8.6, 1.2 Hz), and a Boc-protected compound of Example 49 (80.8 mg) as colorless powder.
: ¹H NMR (CDCl₃, 400 MHz) : δ 1.60 (9H, s), 4.25 (3H, s), 6.41 (1H, d, J = 7.9 Hz), 7.07 (1H, s), 7.31 (1H, d, J = 8.6 Hz), 7.44 (1H, d, J = 7.9 Hz), 7.81 (1H, dd, J = 8.6, 1.5 Hz), 7.97 (1H, d, J = 8.6 Hz), 8.26 (1H, s), 8.30 (1H, d, J = 8.6 Hz).

The Boc-protected compound of Example 48 (94.0 mg) was dissolved in dichloromethane (2.0 mL), trifluoroacetic acid (0.152 mL) was added to the solution and the mixture was stirred at ordinary temperature for one hour. A saturated aqueous solution of sodium hydrogen carbonate was added to the reaction liquid and then the dichloromethane was distilled off under reduced pressure. The insoluble material thus precipitated was filtered off and washed with diisopropyl ether to give a desired product (57.9 mg) as colorless powder (Example 48).

¹H NMR (DMSO-d₆, 400 MHz) : δ 4.20 (3H, s), 6.41 (1H, dd, J = 9.8, 1.8 Hz), 6.62 (1H, s), 6.77 (1H, d, J = 7.9 Hz), 7.22 (1H, d, J = 7.6 Hz), 7.41 (1H, d, J = 7.6 Hz), 7.42 (1H, t, J = 7.9 Hz), 7.52 (1H, d, J = 9.8 Hz), 7.60 (1H, t, J = 7.6 Hz), 11.91 (1H, s).
HREIMS (+): 359.0879 (Calculated for C₁₈H₁₂F₃N₃O₂ : 359.0882).
Elemental Analysis: Found: C 59.39%, H 3.34%, N 11.36%; Calculated (for C₁₈H₁₂F₃N₃O₂ · 1/5 H₂O): C 59.57%, H 3.44%, N 11.58%.

The Boc-protected compound of Example 49 (80.0 mg) was dissolved in dichloromethane (1.5 mL), trifluoroacetic acid (0.129 mL) was added to the solution and the mixture was stirred at ordinary temperature for one hour. A saturated aqueous solution of sodium hydrogen carbonate was added to the reaction liquid and then the dichloromethane was distilled off under reduced pressure. The insoluble material was collected by filtration and washed with diisopropyl ether to give a desired product (57.3 mg) as colorless powder (Example 49).

¹H NMR (DMSO-d₆, 400 MHz) : δ 4.19 (3H, s), 6.53 (1H, dd, J = 9.2, 1.8 Hz), 6.78 (1H, d, J = 7.9 Hz), 7.24 (1H, s), 7.48 (1H, dd, J = 9.2, 1.8 Hz), 7.62 (1H, d, J = 7.9 Hz), 7.66 (1H, d, J = 1.2 Hz), 7.79 (1H, d, J = 9.2 Hz), 7.96 (1H, d, J = 9.2 Hz), 11.72 (1H, s).
HREIMS (+): 359.0919 (Calculated for C₁₈H₁₂F₃N₃O₂ : 359.0882).

### Example 50: 6-(7-Methoxy-2-trifluoromethylpyrazolo[1,5-a]pyridin-4-yl)quinolin-2(1H)-one

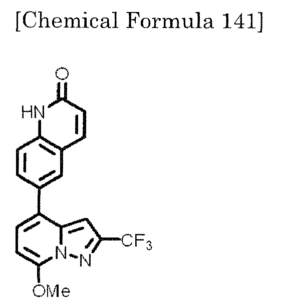

The same procedures used in Example 47 were carried out except for using the compound of Example 5 and the compound of Example 35 to give a desired product as colorless powder.

¹H NMR (CDCl₃, 400 MHz) : δ 4.24 (3H, s), 6.62 (1H, d, J = 9.2 Hz), 6.81 (1H, d, J = 7.9 Hz), 7.30 (1H, s), 7.50 (1H, d, J = 8.6 Hz), 7.60 (1H, d, J = 7.9 Hz), 7.87 (1H, dd, J = 8.6, 1.8 Hz), 8.05 (1H, d, J = 1.8 Hz), 8.08 (1H, d, J = 9.2 Hz), 11.93 (1H, s).
HREIMS (+): 359.0905 (Calculated for C₁₈H₁₂F₃N₃O₂ : 359.0882).

### Example 51: 4-(7-Methoxy-2-trifluoromethylpyrazolo[1,5-a]pyridin-4-yl)quinolin-2(1H)-one:

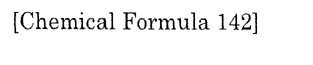

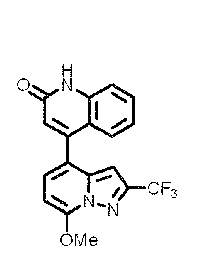

4-Bromoquinolin-2(1H)-one (200 mg) was dissolved in dioxane (9.0 mL) under an argon gas atmosphere, to the resulting solution, were added bis(pinacolato)diboron (272 mg), 1,1-bis(diphenylphosphino)ferrocene palladium dichloride dichloromethane complex (109 mg) and potassium 2-ethylhexanoate (244 mg) and the resulting mixture was stirred at 80°C for 1.5 hours. The reaction liquid was directly treated, without any pretreatment, according to the silica gel column chromatography (ethyl acetate) to give a crude borate (407 mg). The crude borate (407 mg) and the compound of Example 5 (250 mg) were dissolved in dimethoxyethane (10 mL) under an argon gas atmosphere, to this solution were added tetrakis(triphenylphosphine) palladium (129 mg) and a 2.0 mol/L aqueous solution of sodium carbonate (5.6 mL) and the mixture was stirred at 80°C for 9 hours. After water was added to the reaction liquid, the dimethoxyethane was distilled off under reduced pressure and the insoluble material thus formed was collected by filtration. The resulting insoluble material was purified by the silica gel column chromatography (ethyl acetate) and then washed with diisopropyl ether to give a desired product (26.3 mg) as colorless powder.

¹H NMR (DMSO-d₆, 400 MHz): δ 4.28 (3H, s), 6.64 (1H, d, J = 1.2 Hz), 6.85-6.86 (2H, m), 7.14 (1H, dt, J = 7.9, 1.2 Hz), 7.30 (1H, d, J = 7.9 Hz), 7.48 (1H, d, J = 7.9 Hz), 7.59-7.60 (2H, m), 12.03 (1H, s).
HREIMS (+): 359.0870 (Calculated for C₁₈H₁₂F₃N₃O₂: 359.0882).

### Example 52: 5-(7-Methoxy-2-trifluoromethylpyrazolo[1,5-a]pyridin-4-yl)iso quinolin-1(2H)-one:

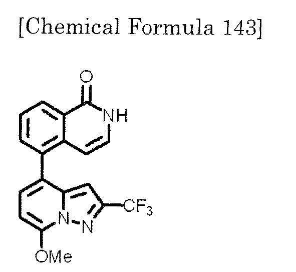

The same procedures used in Example 47 were carried out except for using the compound of Example 38 and the compound of Example 5 to give a desired product as colorless powder.

¹H NMR (DMSO-d₆, 400 MHz) : δ 4.20 (3H, s), 6.10 (1H, d, J = 7.3 Hz), 6.59 (1H, s), 6.77 (1H, d, J = 7.3 Hz), 7.10 (1H, dd, J = 7.3, 5.5 Hz), 7.42 (1H, d, J = 7.9 Hz), 7.59 (1H, t, J = 7.9 Hz), 7.74 (1H, dd, J = 7.3, 1.8 Hz), 8.31 (1H, d, J = 7.9 Hz), 11.35 (1H, d, J = 5.5 Hz). EIMS (+): 359 [M]⁺.
Elemental Analysis: Found: C 60.21%, H 3.51%, N 11.46%; Calculated (for C₁₈H₁₂F₃N₃O₂): C 60.17%, H 3.37%, N 11.69%.

### Example 53: 6-(7-Methoxy-2-trifluoromethylpyrazolo[1,5-a]pyridin-4-yl)iso quinolin-1(2H)-one:

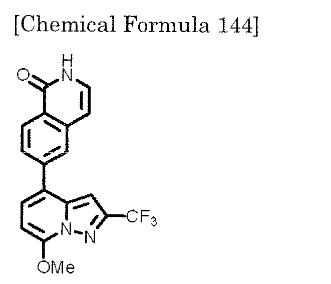

The same procedures used in Example 47 were carried out except for using the compound of Example 39 and the compound of Example 5 to give a desired product as colorless powder.

¹H NMR (DMSO-d₆, 400 MHz) : δ 4.20 (3H, s), 6.67 (1H, d, J = 7.3 Hz), 6.79 (1H, d, J = 7.9 Hz), 7.22 (1H, dd, J = 7.3, 5.5 Hz), 7.25 (1H, s), 7.67 (1H, d, J = 7.9 Hz), 7.77 (1H, dd, J = 8.6, 1.8 Hz), 7.96 (1H, d, J = 1.8 Hz), 8.29 (1H, d, J = 8.6 Hz), 11.29 (1H, s).
HREIMS (+) 359.0888 (Calculated for C₁₈H₁₂F₃N₃O₂: 359.0882).
Elemental Analysis: Found: C 59.64%, H 3.34%, N 11.32%; Calculated (for C₁₈H₁₂F₃N₃O₂ · 1/5H₂O): C 59.57%, H 3.44%, N 11.58%.

### Example 54: 7-(7-Methoxy-2-trifluoromethylpyrazolo[1,5-a]pyridin-4-yl)iso quinolin-1(2H)-one:

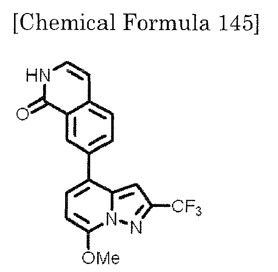

The same procedures used in Example 47 were carried out except for using the compound of Example 40 and the compound of Example 5 to give a desired product as colorless powder.

¹H NMR (DMSO-d₆, 400 MHz) : δ 4.19 (3H, s), 6.63 (1H, d, J = 6.7 Hz), 6.77 (1H, d, J = 7.9 Hz), 7.14 (1H, s), 7.23 (1H, dd, J = 6.7, 5.5 Hz), 7.63 (1H ,d, J = 7.9 Hz), 7.80 (1H, d, J = 7.9 Hz), 8.00 (1H, dd, J = 7.9, 1.8 Hz), 8.41 (1H ,d, J = 1.8 Hz), 11.37 (1H, d, J = 5.5 Hz).
HREIMS (+): 359.0923 (Calculated for C₁₈H₁₂F₃N₃O₂ : 359.0882).
Elemental Analysis: Found: C 59.83%, H 3.50%, N 11.39%; Calculated (for C₁₈H₁₂F₃N₃O₂ · 1/5H₂O): C 59.87%, H 3.41%, N 11.64%.

### Example 55: 4-(7-Methoxy-2-trifluoromethylpyrazolo[1,5-a]pyridin-4-yl)iso quinolin-1(2H)-one:

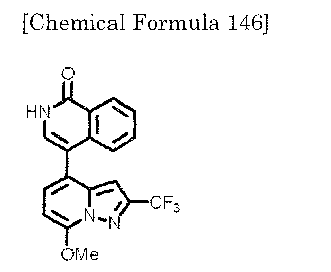

The same procedures used in Example 47 were carried out except for using 4-bromoisoquinolin-1(2H)-one and the compound of Example 33 to give a desired product as white powder.

¹H NMR (DMSO-d₆, 400 MHz) : δ 4.19 (3H, s), 6.69 (1H, d, J = 7.9 Hz), 7.21 (1H, d, J = 7.9 Hz), 7.26 (1H, d, J = 6.1 Hz), 7.44 (1H, d, J = 7.9 Hz), 7.52-7.53 (1H, m), 7.59-7.64 (1H, m), 8.30 (1H, dd, J = 7.9, 1.2), 11.52 (1H, d, J = 6.1 Hz).
HREIMS (+): 359.0920 (Calculated for C₁₈H₁₂F₃N₃O₂ : 359.0882).

### Example 56: 6-(7-Methoxy-2-trifluoromethylpyrazolo[1,5-a]pyridin-4-yl)-3,4-di hydroquinolin-2(1H)-one:

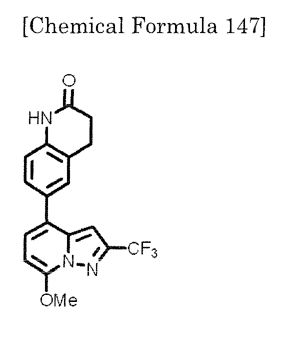

The same procedures used in Example 47 were carried out except for using 6-(4,4,5,5-tetramethyl[1,3,2]dioxaborolan-2-yl)-3,4-dihydroquinolin-2(1H)-one and the compound of Example 5 to give a desired product as colorless powder.

¹H NMR (CDCl₃, 400 MHz) : δ 2.72 (2H, t, J = 7.6 Hz), 3.07 (2H, t, J = 7.6 Hz), 4.22 (3H, s), 6.34 (1H, d, J = 7.3 Hz), 6.86 (1H, d, J = 9.2 Hz), 6.93 (1H, s), 7.23 (1H, d, J = 7.3 Hz), 7.41-7.43 (2H, m), 7.70 (1H, s).
EIMS (+): 361 [M]⁺.
Elemental Analysis: Found: C 59.79%, H 4.06%, N 11.50%; Calculated (for C₁₈H₁₄F₃N₃O₂): C 59.83%, H 4.06%, N 11.63%.

### Example 57: 5-(7-Methoxy-2-trifluoromethylpyrazolo[1,5-a]pyridin-4-yl)indolin-2- one:

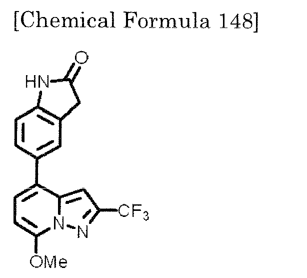

The same procedures used in Example 47 were carried out except for using 5-(4,4,5,5-tetramethyl[1,3,2]dioxaborolan-2-yl)indolin-2-one and the compound of Example 5 to give a desired product as colorless powder.

¹H NMR (DMSO-d₆, 400 MHz) : δ 3.56 (2H, s), 4.16 (3H, s), 6.70 (1H, d, J = 7.9 Hz), 6.94 (1H, d, J = 7.9 Hz), 7.12 (1H, s), 7.42 (1H, d, J = 7.9 Hz), 7.47 (1H, dd, J = 7.9, 1.8 Hz), 7.51 (1H, brs), 10.50 (1H, brs).
EIMS (+): 347 [M]⁺.
Elemental Analysis: Found: C 58.53%, H 3.42%, N 11.99%; Calculated (for C₁₇H₁₂F₃N₃O₂): C 58.79%, H 3.48%, N 12.10%.

### Example 58: 6-(7-Methoxy-2-trifluoromethylpyrazolo[1,5-a]pyridin-4-yl)-3,4-di hydroisoquinolin-1(2H)-one:

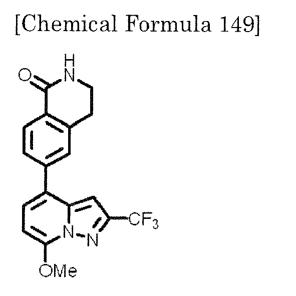

The same procedures used in Example 47 were carried out except for using 6-(4,4,5,5-tetramethyl[1,3,2]dioxaborolan-2-yl)-3,4-dihydroisoquinolin-1(2H)-one and the compound of Example 5 to give a desired product as colorless powder.

¹H NMR (DMSO-d₆, 400 MHz): δ 2.99 (2H, t, J = 6.4 Hz), 3.40-3.41 (2H, m), 4.17 (3H, s), 6.75 (1H, d, J = 7.9 Hz), 7.22 (1H, s), 7.59-7.62 (3H, m), 7.94 (1H, d, J = 7.9 Hz), 7.97 (1H, s).
HREIMS (+): 361.1022 (Calculated for C₁₈H₁₄F₃N₃O₂: 361.1038).
Elemental Analysis: Found: C 59.33%, H 3.86%, N 11.42%; Calculated (for C₁₈H₁₄F₃N₃O₂ · 1/5 H₂O): C 59.24%, H 3.98%, N 11.51%.

### Example 59: 5-(7-Methoxy-2-trifluoromethylpyrazolo[1,5-a]pyridin-4-yl)isoindolin-1-one:

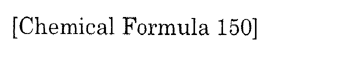

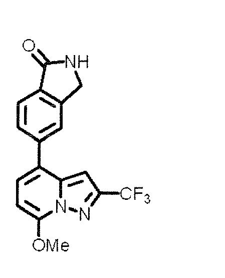

The same procedures used in Example 47 were carried out except for using 5-(4,4,5,5-tetramethyl[1,3,2]dioxaborolan-2-yl)isoindolin-1-one and the compound of Example 5 to give a desired product as colorless powder.

¹H NMR (DMSO-d₆, 400 MHz) : δ 4.19 (3H, s), 4.46 (2H, s), 6.77 (1H, d, J = 7.9 Hz), 7.24 (1H ,s), 7.61 (1H, d, J = 7.9 Hz), 7.76-7.79 (2H, m), 7.88 (1H, s), 8.61 (1H, s).
HREIMS (+): 347.0838 (Calculated for C₁₇H₁₂F₃N₃O₂: 347.0882).
Elemental Analysis: Found: C 58.26%, H 3.57%, N 11.77%; Calculated (for C₁₇H₁₂F₃N₃O₂ · 1/10 H₂O): C 58.49%, H 3.51%, N 12.04%.

### Example 60: 4-Chloro-6-(7-methoxy-2-trifluoromethylpyrazolo[1,5-a]pyridin-4-yl) isoquinolin-1(2H)- one:

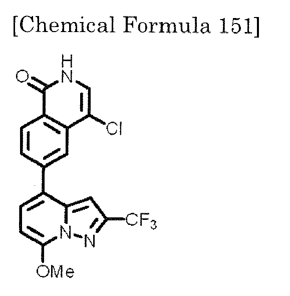

The same procedures used in Example 47 were carried out except for using the compound of Example 25 and the compound of Example 33 to give a desired product as yellow powder.

¹H NMR (CDCl₃, 400 MHz): δ 4.21 (3H, s), 6.80 (1H, d, J = 7.9 Hz), 7.19 (1H, s), 7.56 (1H, s), 7.72 (1H, d, J = 7.9 Hz), 7.92 (1H, dd, J = 7.9, 1.2 Hz), 7.99 (1H, d, J = 1.2 Hz), 8.36 (1H, d, J = 7.9 Hz), 11.63 (1H, s).
HREIMS (+): 393.0453 (Calculated for C₁₈H₁₁ClF₃N₃O₂: 393.0492).

### Example 61: 6-(3-Chloro-7-methoxy-2-trifluoromethylpyrazolo[1,5-a]pyridin-4-yl) quinolin-2(1H)-one:

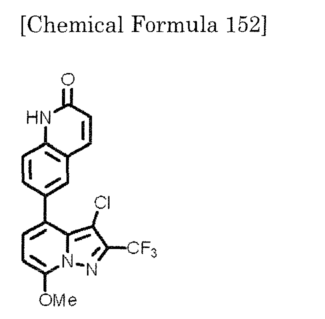

The same procedures used in Example 47 were carried out except for using the compound of Example 35 and the compound of Example 22 to give a desired product as colorless powder.

¹H NMR (CDCl₃, 400 MHz) : δ 4.19 (3H, s), 6.54 (1H, d, J = 9.2 Hz), 6.78 (1H, d, J = 7.9 Hz), 7.36 (1H, d, J = 8.6 Hz), 7.41 (1H, d, J = 7.9 Hz), 7.58 (1H, dd, J = 8.6, 1.8 Hz), 7.75 (1H, d, J = 1.8 Hz), 7.95 (1H, d, J = 9.2 Hz), 11.87 (1H, s).
HRESIMS (+): 394.05756 (Calculated for C₁₈H₁₂ClF₃N₃O₂: 394.05701).
Elemental Analysis: Found: C 54.64%, H 2.90%, N 10.56%; Calculated (for C₁₈H₁₂ClF₃N₃O₂): C 54.91%, H 2.82%, N 10.67%.

### Example 62: 6-(3-Chloro-7-methoxy-2-trifluoromethylpyrazolo[1,5-a]pyridin-4-yl)-3,4-dihydroisoquinolin-1(2H)-one:

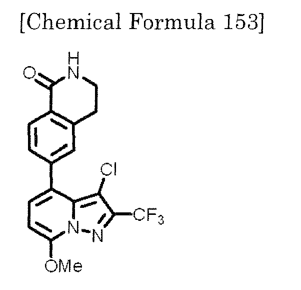

The same procedures used in Example 47 were carried out except for using 6-(4,4,5,5-tetramethyl[1,3,2]dioxaborolan-2-yl)-3,4-dihydroisoquinolin-1(2H)-one and the compound of Example 22 to give a desired product as colorless powder.

¹H NMR (CDCl₃, 400 MHz) : δ 2.96 (2H, t, J = 6.7 Hz), 3.40-3.42 (2H, m), 4.19 (3H, s), 6.79 (1H, d, J = 7.9 Hz), 7.39-7.42 (2H, m), 7.44 (1H, d, J = 7.9 Hz), 7.90 (1H, d, J = 7.3 Hz), 7.99 (1H, s).
HREIMS (+): 395.0605 (Calculated for C₁₈H₁₃ClF₃N₃O₂: 395.0648).
Elemental Analysis: Found: C 54.45%, H 3.44%, N 10.28%; Calculated (for C₁₈H₁₃ClF₃N₃O₂): C 54.63%, H 3.31%, N 10.62%.

### Example 63: 6-(7-Methylamino-2-trifluoromethylpyrazolo[1,5-a]pyridin-4-yl)-3,4-dihydroisoquinolin-1(2H)-one:

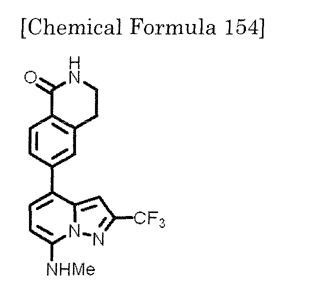

Potassium t-butoxide (93.2 mg) was dissolved in DMSO (1.5 mL) under an argon gas atmosphere, N-methylformamide (0.0486 mL) was added to the solution and the reaction liquid was stirred at ordinary temperature for one hour. A solution of the compound of Example 58 (100 mg) in DMSO (2.5 mL) was added to the liquid at ordinary temperature and the mixture was further stirred for 2 hours. Water was added to the reaction liquid and the mixture was extracted three times with ethyl acetate. The combined extracts were washed with saturated brine, dried over anhydrous sodium sulfate, the solvent was distilled off under reduced pressure, the resulting residue was purified by the silica gel column chromatography (hexane: ethyl acetate = 1:2→ethyl acetate) and then washed with diisopropyl ether to give a desired product (53.7 mg) as yellow powder.

¹H NMR (CDCl₃, 400 MHz) : δ 3.09 (2H, t, J = 6.4 Hz), 3.15 (3H, d, J = 5.5 Hz), 3.63-3.64 (2H, m), 5.91 (1H, s), 6.10 (1H, d, J = 7.9 Hz), 6.11 (1H, s), 6.93 (1H, s), 7.38 (1H, d, J = 7.9 Hz), 7.46 (1H, d, J = 1.8 Hz), 7.59 (1H, dd, J = 7.9, 1.8 Hz), 8.17 (1H, d, J = 7.9 Hz). HREIMS (+): 361.2807 (Calculated for C₁₈H₁₆F₃N₄O: 361.2762).

### Example 64: 6-(7-Methylamino-2-trifluoromethylpyrazolo[1,5-a]pyridin-4-yl) quinolin-2(1H)-one:

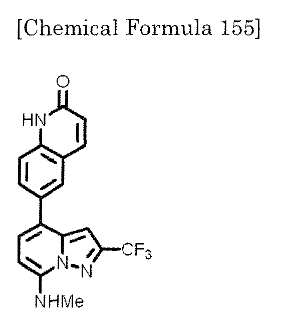

The same procedures used in Example 47 were carried out except for using the compound of Example 35 and the compound of Example 21 to give a desired product as yellow powder.

¹H NMR (CDCl₃, 400 MHz) : δ 2.99 (3H, d, J = 4.9 Hz), 6.24 (1H, d, J = 7.9 Hz), 6.53 (1H, dd, J = 9.8, 1.8 Hz), 7.16 (1H, s), 7.32 (1H, q, J = 4.9 Hz), 7.41 (1H, q, J = 4.9 Hz), 7.50 (1H ,d, J = 7.9 Hz), 7.78 (1H, dd, J = 8.6, 1.8 Hz), 7.94 (1H, d, J = 1.8 Hz), 8.01 (1H, d, J = 9.8 Hz), 11.82 (1H, s).
HREIMS (+): 358.1035 (Calculated for C₁₈H₁₃F₃N₄O: 358.1041).

### Example 65: 6-(3-Chloro-7-methylamino-2-trifluoromethylpyrazolo[1,5-a]pyridin-4-yl)quinolin-2(1H)-one:

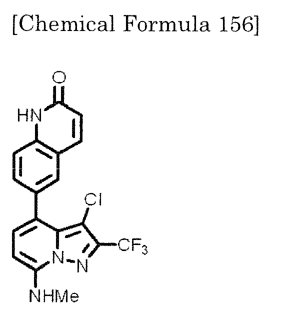

The same procedures used in Example 47 were carried out except for using the compound of Example 35 and the compound of Example 23 to give a desired product as yellow powder.

¹H NMR (CDCl₃, 400 MHz) : δ 2.99 (3H, d, J = 4.9 Hz), 6.27 (1H, d, J = 7.9 Hz), 6.53 (1H, dd, J = 9.2, 1.8 Hz), 7.34 (2H, m), 7.41 (1H, q, J = 4.9 Hz), 7.54 (1H, dd, J = 8.6, 1.8 Hz), 7.70 (1H, d, J = 1.8 Hz), 7.94 (1H, d, J = 9.2 Hz), 11.83 (1H, s).
HREIMS (+): 392.0661 (Calculated for C₁₈H₁₂ClF₃N₄O: 392.0652).

### Example 66: 6-(3-Chloro-7-methylamino-2-trifluoromethylpyrazolo[1,5-a]pyridin-4-yl)-3,4-dihydroisoquinolin-1(2H)-one:

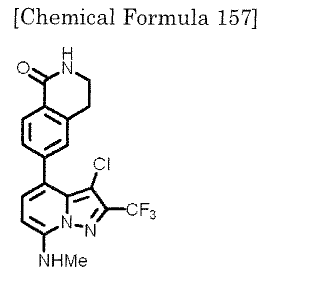

The same procedures used in Example 47 were carried out except for using 6-(4,4,5,5-tetramethyl[1,3,2]dioxaborolan-2-yl)-3,4-dihydroisoquinolin-1(2H)-one and the compound of Example 23 to give a desired product as colorless powder.

¹H NMR (CDCl₃, 400 MHz) : δ 2.95 (2H, t, J = 6.7 Hz), 2.99 (3H, d, J = 4.9 Hz), 3.38-3.40 (2H, m), 6.28 (1H, d, J = 7.9 Hz), 7.36-7.38 (3H, m), 7.48 (1H, q, J = 4.9 Hz), 7.87 (1H, d, J = 7.9 Hz), 7.95 (1H, s).
ESIMS (+): 395 [M+H]⁺.
Elemental Analysis: Found: C 54.80%, H 3.57%, N 14.11%; Calculated (for C₁₈H₁₄ClF₃N₄O): C 54.76%, H 3.57%, N 14.19%.

### Example 67: 2-Ethyl-8-methoxy-5,6'-biquinolin-2'(1'H)-one:

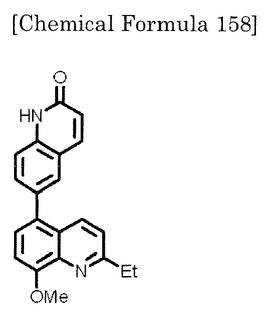

The same procedures used in Example 32 were carried out except for using 5-bromo-2-ethyl-8-methoxyquinoline to obtain a compound and then the same procedures used in Example 47 were carried out except for using the compound prepared above and 6-bromoquinolin-2(1H)-one to give a desired product as yellow powder.

¹H NMR (DMSO-d₆, 400 MHz) : δ 1.29 (3H, t, J = 7.6 Hz), 2.91 (2H, q, J = 7.6 Hz), 3.98 (3H, s), 6.54 (1H, dd, J = 9.5, 1.5 Hz), 7.22 (1H, d, J = 8.6 Hz), 7.41-7.42 (3H, m), 7.56 (1H, dd, J = 8.6, 1.8 Hz), 7.73 (1H, d, J = 1.8 Hz), 7.95 (1H ,d, J = 9.5 Hz), 8.08 (1H, d, J = 8.6 Hz), 11.85 (1H, s).
HREIMS (+): 330.1362 (Calculated for C₂₁H₁₈N₂O₂: 330.1368).

### Example 68: 8-Methoxy-2-methyl-5,6'-biquinolin-2'(1'H)-one:

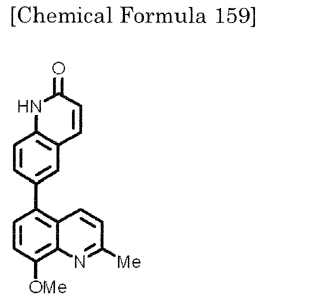

The same procedures used in Example 47 were carried out except for using 5-bromo-8-methoxy-2-methylquinoline and the compound of Example 35 to give a desired product as yellow powder.

¹H NMR (DMSO-d₆, 400 MHz) : δ 2.65 (3H, s), 3.98 (3H, s), 6.55 (1H, dd, J = 9.5, 1.5 Hz), 7.22 (1H, d, J = 7.9 Hz), 7.39-7.43 (3H, m), 7.56 (1H, dd, J = 8.6, 1.8 Hz), 7.73 (1H, d, J = 1.8 Hz), 7.96 (1H, d, J = 9.5 Hz), 8.06 (1H, d, J = 8.6 Hz), 11.86 (1H, s).
HREIMS (+): 316.1183 (Calculated for C₂₀H₁₆N₂O₂: 316.1212).

### Example 69: 8-Methoxy-2-trifluoromethyl-5,6'-biquinolin-2'(1'H)-one:

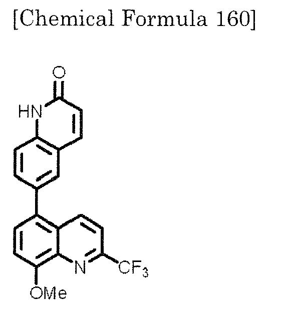

The same procedures used in Example 47 were carried out except for using 5-bromo-8-methoxy-2-trifluoromethylquinoline and the compound of Example 35 to give a desired product as yellow powder.

¹H NMR (DMSO-d₆, 400 MHz) : δ 4.06 (3H, s), 6.57 (1H, dd, J = 9.2, 1.2 Hz), 7.43-7.45 (2H, m), 7.61 (1H, dd, J = 8.6, 1.8 Hz), 7.69 (1H, d, J = 8.6 Hz), 7.79 (1H, d, J = 1.8 Hz), 7.95-7.97 (2H, m), 8.48 (1H, d, J = 8.6 Hz), 11.89 (1H, s).HREIMS (+): 370.0895 (Calculated for C₂₀H₁₃F₃N₂O₂: 370.0929).

### Example 70: 6-(5-Methoxy-2-trifluoromethyl[1,2,4]triazolo[1,5-a]pyridin-8-yl) quinolin-2(1H)-one

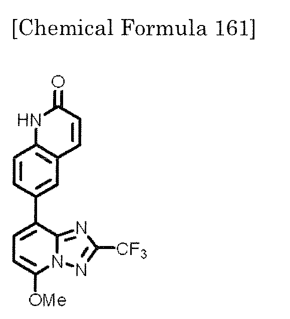

The same procedures used in Example 47 were carried out except for using the compound of Example 35 and the compound of Example 10 to give a desired product as a white solid.

¹H NMR (CDCl₃, 400 MHz) : δ 4.32 (3H, s), 6.55 (1H, dd, J = 9.2, 1.8 Hz), 7.03 (1H, d, J = 8.6 Hz), 7.44 (1H, d, J = 8.6 Hz), 8.00 (1H, d, J = 9.2 Hz), 8.14-8.16 (2H, m), 8.29 (1H, d, J =1.8 Hz), 11.88 (1H, s).
HRESIMS (+): 361.09027 (Calculated for C₁₇H₁₂F₃N₄O₂: 361.09124).

### Example 71: 6-(4-Methoxy-2-trifluoromethylbenzo[d]thiazol-7-yl)quinolin-2(1H)- one

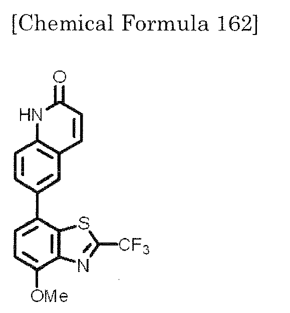

The same procedures used in Example 47 were carried out except for using the compound of Example 35 and the compound of Example 11 to give a desired product as a white solid.

¹H NMR (CDCl₃, 400 MHz) : δ 4.04 (3H, s), 6.56 (1H, d, J = 8.6 Hz), 7.37 (1H, d, J = 8.6 Hz), 7.44 (1H, d, J = 8.6 Hz), 7.75 (1H, d, J = 8.6 Hz), 7.81 (1H, dd, J = 8.6, 2.4 Hz), 7.99-7.99 (2H, m), 11.88 (1H, s).
HREIMS (+): 376.0468 (Calculated for C₁₈H₁₁F₃N₂O₂S: 376.0493).
Elemental Analysis: Found: C 57.71%, H 3.15%, N 7.14%; Calculated (for C₁₈H₁₁F₃N₂O₂S): C 57.44%, H 2.95%, N 7.44%.

### Example 72: 8-Methoxy-5-(2-oxo-1,2-dihydroquinolin-6-yl)-2H-benzo[e][1,3]oxazin-4-(3H)-one

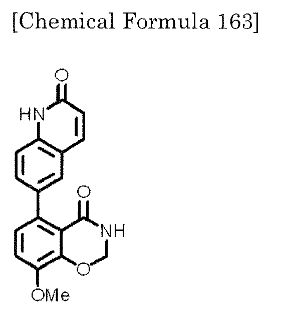

The same procedures used in Example 47 were carried out except for using the compound of Example 35 and the compound of Example 44 to give a desired product as a white solid.

¹H NMR (DMSO-d₆, 400 MHz) : δ 3.83 (3H, s), 5.15 (2H, s), 6.49 (1H, d, J = 9.2 Hz), 6.97 (1H, d, J = 8.0 Hz), 7.22 (1H, d, J = 8.6 Hz), 7.23 (1H, d, J = 8.0 Hz), 7.43 (1H, dd, J = 8.6, 1.8 Hz), 7.58 (1H, d, J = 1.8 Hz), 7.89 (1H, d, J = 9.2 Hz), 8.68 (1H, s), 11.74 (1H, s).
ESIMS (+) : 323 [M+H]⁺.
Elemental Analysis: Found: C 65.91%, H 4.34%, N 8.37%; Calculated (for C₁₈H₁₄N₂O₄ · 0.3H₂O): C 65.97%, H 4.49%, N 8.55%.

### Example 73: 7-Methoxy-4-(2-oxo-1,2-dihydroquinolin-6-yl) -benzo[d]oxazol- 2(3H)-one

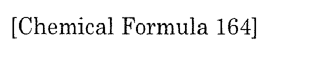

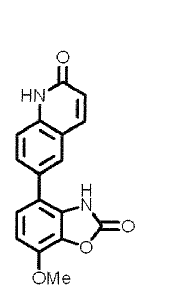

The same procedures used in Example 47 were carried out except for using the compound of Example 35 and the compound of Example 45 to give a desired product as a yellow solid.

¹ H NMR (DMSO-d₆, 400 MHz) : δ 3.81 (3H, s), 6.36 (1H, d, J = 8.6 Hz), 6.45 (1H, d, J = 9.2 Hz), 7.03 (1H, d, J = 9.2 Hz), 7.27 (1H, d, J = 8.6 Hz), 7.90 (1H, d, J = 9.2 Hz), 8.20 (1H, dd, J = 9.2, 1.8 Hz), 8.33 (1H, d, J = 1.8 Hz), 11.65 (1H, s).
HRESIMS (+) : 307.0722 (Calculated for C₁₇H₁₁N₂O₄: 309.0719).

### Example 74: 8-Methoxy-5-(2-oxo-1,2-dihydroquinolin-6-yl)-2H-benzo[b][1,4]oxazin-3(4H)-one:

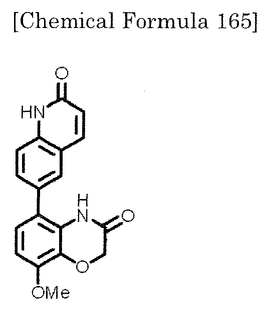

The same procedures used in Example 47 were carried out except for using the compound of Example 35 and the compound of Example 46 to give a desired product as a white solid.

¹ H NMR (DMSO-d₆, 400 MHz) : δ 3.80 (3H, s), 4.52 (2H, s), 6.51 (1H, d, J = 9.8 Hz), 6.79 (1H, d, J = 8.6 Hz), 6.87 (1H, d, J = 8.6 Hz), 7.35 (1H, d, J = 8.6 Hz), 7.47 (1H, dd, J = 8.6, 1.8Hz), 7.64 (1H, d, J = 1.8Hz), 7.93 (1H, d, J = 9.8Hz), 9.78 (1H, brs), 11.79 (1H, brs). EIMS (+) : 322 [M]⁺. Elemental Analysis: Found: C 67.00%, H 4.38%, N 8.63%; Calculated (for C₁₈ H_{1 4} N₂ O₄: C 67.07%, H 4.38%, N 8.69%.

### Example 75: 8-Methoxy-5-(1-oxo-1,2,3,4-tetrahydroisoquinolin-6-yl)-2H-benzo[b] [1,4]oxazin- 3(4H)-one:

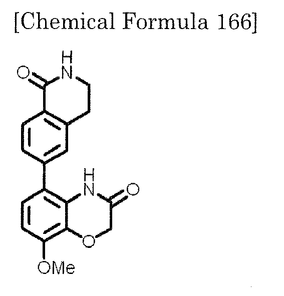

The same procedures used in Example 47 were carried out except for using 6-(4,4,5,5-tetramethyl[1,3,2]dioxaborolan-2-yl)-3,4-dihydroisoquinolin-1(2H)-one and the compound of Example 46 to give a desired product as a white solid.

¹H NMR (DMSO-d₆, 400 MHz) : δ 2.93 (2H, t, J = 6.7 Hz), 3.39 (2H, dt, J = 6.7, 2.4 Hz), 3.81 (3H, s), 4.53 (2H, s), 6.81 (2H, d, J = 8.6 Hz), 6.89 (1H, d, J = 8.6 Hz), 7.29 (1H, s), 7.32 (1H, dd, J = 7.9, 2.4 Hz), 7.87 (1H, d, J = 7.9 Hz), 7.92 (1H, s).
EIMS (+) : 324 [M]⁺.
Elemental Analysis: Found: C 64.94%, H 4.91%, N 8.60%; Calculated (for C₁₈H₁₆ N₂ O₄. 0.5H₂ O): C 64.86%, H 5.14, N 8.40%.

### Example 76: 6-(8-Methoxyquinolin-5-yl) -3,4-dihydroquinolin-2(1H)-one:

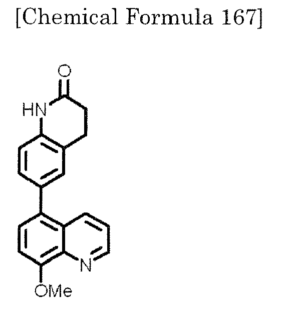

The same procedures used in Example 32 were carried out except for using 5-bromo-8-methoxyquinoline to form a compound and then the same procedures used in Example 47 were carried out except for using the compound obtained above and 6-bromo-3,4-dihydroquinolin-2(1H)-one to give a desired product as a pale yellow solid.

¹H NMR (DMSO-d₆, 400 MHz) : δ 2.49-2.52 (2H, m), 2.94 (2H, t, J = 7.3 Hz), 3.98 (3H, s), 6.98 (1H, d, J = 7.9 Hz), 7.19-7.25 (3H, m), 7.42 (1H, d, J = 7.9 Hz), 7.52 (1H, dd, J = 8.6, 3.6 Hz), 8.19 (1H, dd, J = 8.6, 1.2 Hz), 8.85 (1H, dd, J = 3.6, 1.2 Hz), 10.2 (1H, s).
EIMS (+) : 304 [M]⁺.
Elemental Analysis: Found: C 73.42%, H 5.41%, N 8.55%; Calculated (for C₁₉H₁₆N₂O₂· 0.33H₂O): C 73.55%, H 5.41%, N 9.03%.

### Example 77: 5-(8-Methoxyquinolin-5-yl)indolin-2-one

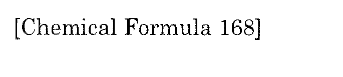

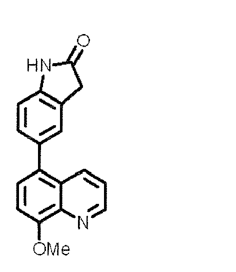

The same procedures used in Example 32 were carried out except for using 5-bromo-8-methoxyquinoline to form a compound and then the same procedures used in Example 47 were carried out except for using the compound obtained above and 5-bromoindolin-2-one to give a desired product as a pale yellow solid.

¹ H NMR (CDCl₃ , 400 MHz): δ 3.64 (2H, s), 4.14 (3H, s), 7.00 (1H, d, J = 7.9 Hz), 7.11 (1H, d, J = 7.9 Hz), 7.27-7.33 (2H, m), 7.41 (2H, dt, J = 8.6, 1.8 Hz), 8.18-8.23 (2H, m), 8.96 (1H, dd, J = 4.4, 1.8 Hz).
EIMS (+) : 290 [M]⁺.
Elemental Analysis: Found: C 72.40%, H 4.94%, N 8.95%; Calculated (for C₁₈H₁₄N₂O₂· 0.5H₂ O): C 72.23%, H 5.05%, N 9.36%.

### Example 78: 7-(8-Methoxyquinolin-5-yl)isoquinolin-1(2H)-one:

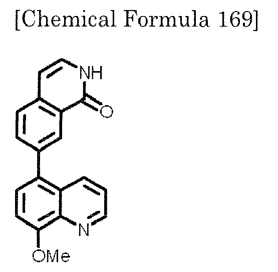

The same procedures used in Example 32 were carried out except for using 5-bromo-8-methoxyquinoline to form a compound and then the same procedures used in Example 47 were carried out except for using the compound obtained above and 7-bromoisoquinolin-1(2H)-one to give a desired product as a pale yellow solid.

¹ H NMR (DMSO-d₆, 400 MHz) : δ 4.02 (3H, s), 6.64 (1H, d, J = 6.7 Hz), 7.23 (1H, t, J = 6.7 Hz), 7.30 (1H, d, J = 7.9 Hz), 7.52-7.57 (2H, m), 7.76-7.80 (2H, m), 8.15-8.20 (2H, m), 8.89 (1H, dd, J = 9.1, 1.2 Hz), 11.4 (1H, brd, J = 4.9 Hz).
EIMS (+) : 302 [M]⁺ .
Elemental Analysis: Found: C 74.74%, H 4.74%, N 9.16%; Calculated (for C₁₉H₁₄ N₂ O₂ · 0.2H₂O): C 74.59%, H 4.74%, N 9.16%.

### Example 79: 5-(8-Methoxyquinolin-5-yl)isoquinolin-1(2H)-one:

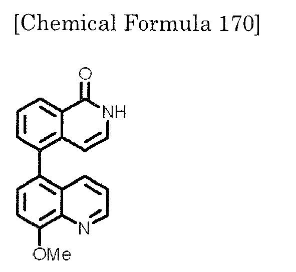

The same procedures used in Example 32 were carried out except for using 5-bromo-8-methoxyquinoline to form a compound and then the same procedures used in Example 47 were carried out except for using the compound obtained above and 5-bromoisoquinolin-1(2H)-one to give a desired product as a white solid.

¹ H NMR (CDCl₃, 400 MHz) : δ 4.18 (3H, s), 6.06 (1H, d, J = 7.3Hz), 7.00 (1H, dd, J = 7.3, 4.3 Hz), 7.17 (1H, d, J = 7.9Hz), 7.34 (1H, dd, J = 8.6, 4.3Hz), 7.45 (1H, d, J = 7.9Hz), 7.60-7.72 (3H, m), 8.55 (1H, dd, J = 7.9, 1.2Hz), 8.97 (1H, dd, J = 4.3, 1.8Hz), 10.7 (1H, brs).
EIMS (+) : 302 [M]⁺.
Elemental Analysis: Found: C 72.79%, H 4.57%, N 8.90%; Calculated (for C₁₉ H₁₄ N₂O₂· 0.6H₂ O): C 72.88%, H 4.89%, N 8.95%.

### Example 80: 6-(8-Methoxyquinolin-5-yl)isoquinolin-1(2H)-one:

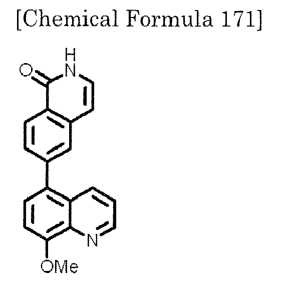

The same procedures used in Example 32 were carried out except for using 5-bromo-8-methoxyquinoline to form a compound and then the same procedures used in Example 47 were carried out except for using the compound obtained above and 6-bromoisoquinolin-1(2H)-one to give a desired product as a pale yellow solid.

¹H NMR (DMSO-d₆, 400 MHz) : δ 4.02 (3H, s), 6.61 (1H, d, J = 7.3 Hz), 7.22 (1H, t, J = 6.1 Hz), 7.30 (1H, d, J = 8.6 Hz), 7.53-7.57 (3H, m), 7.73 (1H, d, J = 1.2 Hz), 8.18 (1H, dd, J = 8.6, 1.8 Hz), 8.28 (1H, d, J = 8.0 Hz), 8.89 (1H, dd, J = 4.3, 1.8 Hz), 11.3 (1H, brs).
EIMS (+) : 302 [M]⁺.
Elemental Analysis: Found: C 73.16%, H 4.63%, N 8.77%; Calculated (for C₁₉ H₁₄N₂O₂· 0.5H₂O): C 73.30%, H 4.86%, N 9.00%.

### Example 81: 6-(2-Ethyl-8-methoxyquinolin-5-yl)-3,4-dihydroquinolin-2(1H)-one:

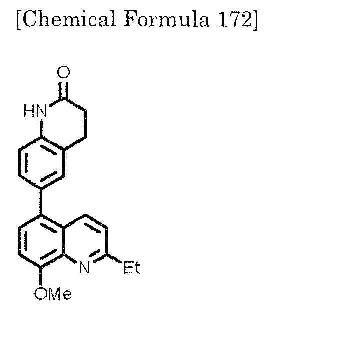

The same procedures used in Example 32 were carried out except for using 5-bromo-2-ethyl-8-methoxyquinoline to form a compound and then the same procedures used in Example 47 were carried out except for using the compound obtained above and 6-bromo-3,4-dihydroquinolin-2(1H)-one to give a desired product as a pale yellow solid.

¹H NMR (CDCl₃ , 400 MHz) : δ 1.41 (3H, t, J = 7.6 Hz), 2.72 (2H, t, J = 7.3 Hz), 3.04-3.12 (4H, m), 4.12 (3H, s), 6.85-6.90 (1H, m), 7.07 (1H, d, J = 7.9 Hz), 7.23-7.26 (2H, m), 7.33 (2H, d, J = 8.6 Hz), 8.14 (1H, d, J = 8.6 Hz).
EIMS (+) : 332 [M]⁺.
Elemental Analysis: Found: C 75.22%, H 6.09%, N 8.19%; Calculated (for C_{2 1}H₂₀ N₂ · 0.2H₂ O): C 75.07%, H 6.12%, N 8.94%.

### Example 82: 5-(2-Ethyl-8-methoxyquinolin-5-yl)indolin-2-one:

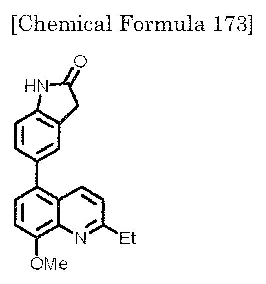

The same procedures used in Example 32 were carried out except for using 5-bromo-2-ethyl-8-methoxyquinoline to form a compound and then the same procedures used in Example 47 were carried out except for using the compound obtained above and 5-bromo-indolin-2-one to give a desired product as a yellow solid.

¹H NMR (DMSO-d₆, 400 MHz) : δ 1.29 (3H, t, J = 7.6 Hz), 2.91 (2H, q, J = 7.6 Hz), 3.54 (2H, s), 3.97 (3H, s), 6.93 (1H, d, J = 7.3 Hz), 7.19 (2H, t, J = 7.3 Hz), 7.24 (1H, s), 7.32 (1H, d, J = 7.9 Hz), 7.41 (1H, d, J = 8.6 Hz), 8.08 (1H, d, J = 9.2 Hz), 10.5 (1H, s).
EIMS (+) : 318 [M]⁺.
Elemental Analysis: Found: C 74.56%, H 5.81%, N 8.28%; Calculated (for C₂₀H₁₈ N₂O₂ · 0.3H₂ O): C 74.19%, H 5.79%, N 8.65%.

### Example 83: 7-(2-Ethyl-8-methoxyquinolin-5-yl)isoquinolin-1(2H)-one:

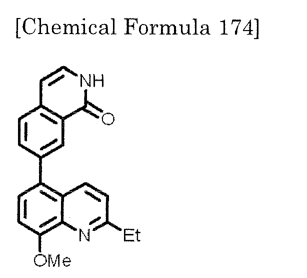

The same procedures used in Example 32 were carried out except for using 5-bromo-2-ethyl-8-methoxyquinoline to form a compound and then the same procedures used in Example 47 were carried out except for using the compound obtained above and 7-bromoisoquinolin-1(2H)-one to give a desired product as a pale yellow solid.

¹H NMR (DMSO-d₆, 400 MHz) : δ 1.30 (3H, t, J = 7.6 Hz), 2.93 (2H, q, J = 7.6 Hz), 4.00 (3H, s), 6.63 (1H, d, J = 7.3 Hz), 7.20-7.27 (2H, m), 7.46 (2H, dd, J = 8.3, 2.4 Hz), 7.78 (2H, dd, J = 10.1, 8.3 Hz), 8.08 (1H, d, J = 8.3 Hz), 8.18 (1H, s), 11.3 (1H brd, J = 4.9 Hz).
EIMS (+) : 330 [M]⁺.
Elemental Analysis: Found: C 75.16%, H 5.53%, N 8.21%; Calculated (for C₂₁ H₁₈ N₂ O₂ · 0.3H₂O): C 75.12%, H 5.58%, N 8.34%.

### Example 84: 5-(2-Ethyl-8-methoxyquiolin-5-yl)isoquinolin-1(2H)-one:

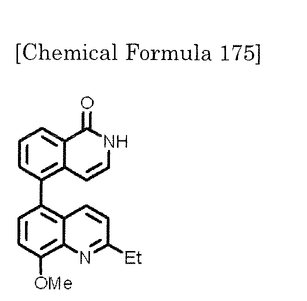

The same procedures used in Example 32 were carried out except for using 5-bromo-2-ethyl-8-methoxyquinoline to form a compound and then the same procedures used in Example 47 were carried out except for using the compound obtained above and 5-bromoisoquinolin-1(2H)-one to give a desired product as a white solid.

¹H NMR (DMSO-d₆, 400 MHz) : δ 1.28 (3H, t, J = 7.6 Hz), 2.91 (2H, q, J = 7.6 Hz), 4.02 (3H, s), 5.57 (1H, d, J = 7.3 Hz), 7.00 (1H, dd, J = 7.3, 6.1 Hz), 7.25 (1H, d, J = 7.9 Hz), 7.36 (2H, dd, J = 10.5, 8.6 Hz), 7.50 (1H, d, J = 8.6 Hz), 7.54-7.64 (2H, m), 8.31 (1H, dd, J = 7.3, 1.2 Hz), 11.3 (1H, brd, J = 5.5 Hz).
EIMS (+) : 330 [M]⁺ .
Elemental Analysis: Found: C 75.76%, H 5.51%, N 8.25%; Calculated (for C₂₁ H₁₈ N₂ O₂· 0.2H₂O): C 75.52%, H 5.55%, N 8.39%.

### Example 85: 6-(2-Ethyl-8-methoxyquinolin-5-yl)isoquinolin- 1(2H)-one:

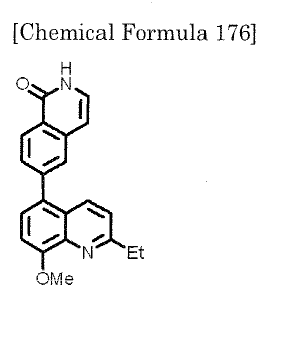

The same procedures used in Example 32 were carried out except for using 5-bromo-2-ethyl-8-methoxyquinoline to thus form a compound and then the same procedures used in Example 47 were carried out except for using the compound obtained above and 6-bromoisoquinolin-1(2H)-one to give a desired product as a yellow solid.

¹ H NMR (DMSO-d₆, 400 MHz) : δ 1.30 (3H, t, J = 7.9 Hz), 2.93 (2H, q, J = 7.9 Hz), 4.04 (3H, s), 6.60 (1H, d, J = 7.3 Hz), 7.21 (1H, t, J = 7.3 Hz), 7,25 (1H, d, J = 8.0 Hz), 7.47 (2H, t, J = 7.3 Hz), 7.53 (1H, dd, J = 8.6, 1.2. Hz), 7.72 (1H, d, J = 1.2 Hz), 8.09 (1H, d, J = 8.6 Hz), 8.27 (1H, d, J = 8.6 Hz), 11.3 (1H, brd, J = 4.3 Hz).
EIMS (+): 330 [M]⁺.
Elemental Analysis: Found: C 75.40%, H 5.53%, N 8.27%; Calculated (for C_{2 1} H₁₈N₂ O₂ · 0.2H₂ O): C 75.52%, H 5.55%, N 8.39%.

### Example 86: 6-(8-Methoxyquinoxalin-5-yl)quinolin-2(1H)-one:

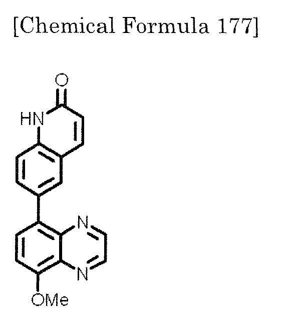

The same procedures used in Example 47 were carried out except for using 5-bromo-8-methoxyquinoxaline and the compound of Example 35 to give a desired product as a yellow solid.

¹H NMR (DMSO-d₆, 400 MHz) : δ 4.03 (3H, s), 6.52 (1H, dd, J = 9.5, 1.8 Hz), 7.38 (1H, dd, J = 8.0, 3.7 Hz), 7.76 (1H, dd, J = 8.6, 1.8 Hz), 7.84 (1H, d, J = 8.6 Hz), 7.89 (1H, d, J = 1.8 Hz), 7.96 (1H, d, J = 9.5 Hz), 8.93 (2H, dd, J = 11.6, 1.8 Hz), 11.8(1H, s).
EIMS (+): 303 [M]⁺.
Elemental Analysis: Found: C 69.77%, H 4.27%, N 13.14%; Calculated (for C₁₈ H₁₃ N₂ O₂ · 0.45H2 O): C 69.42%, H 4.50%, N 13.49%.

### Example 87: 6-(2-Ethyl-7-methoxypyrazolo[1,5-a]pyridin-4-yl)quinolin-2(1H)-one:

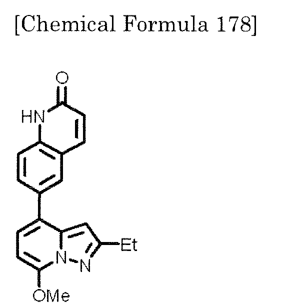

The same procedures used in Example 47 were carried out except for using the compound of Example 8 and the compound of Example 35 to give a desired product as a gray-colored solid.

¹H NMR (DMSO-d₆, 400 MHz) : δ 1.26 (3H, t, J = 7.6 Hz), 2.77 (2H, q, J = 7.6 Hz), 4.09 (3H, s), 6.39 (1H, d, J = 7.9 Hz), 6.54 (1H, d, J = 9.8 Hz), 6.58 (1H, s), 7.27 (1H, d, J = 7.9 Hz), 7.41 (1H, d, J = 8.6 Hz), 7.78 (1H, dd, J = 8.6, 1.8 Hz), 7.94 (1H, d, J = 1.8 Hz), 7.99 (1H, d, J = 9.8 Hz), 11.8 (1H, s).
EIMS (+): 319 [M]⁺.
Elemental Analysis: Found: C 68.60%, H 5.52%, N 12.04%; Calculated (for C₁₉ H₁₇ N₃O₂ · 0.75H2 O): C 68.56%, H 5.60%, N 12.04%.

### Example 88: 6-(8-methoxyquinazolin-5-yl)quinolin-2(1H)-one:

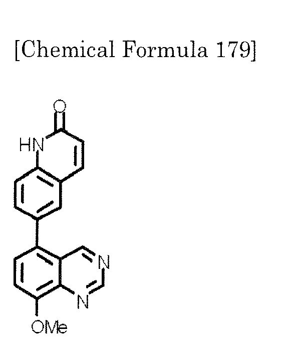

The same procedures used in Example 47 were carried out except for using 5-bromo-8-methoxyquinazoline and the compound of Example 35 to give a desired product as a pale yellow solid.

¹ H NMR (DMSO-d₆, 400 MHz) : δ 4.03 (3H, s), 6.57 (1H, dd, J = 9.2, 1.8 Hz), 7.46 (1H, d, J = 8.6 Hz), 7.54 (1H, d, J = 7.9 Hz), 7.64-7.69 (2H, m), 7.86 (1H, d, J = 1.8 Hz), 7.99 (1H, d, J = 9.2 Hz), 9.29 (1H, s), 9.38 (1H, s), 11.90 (1H, s).
HREIMS (+): 303.3201 (Calculated for C₁₈H₁₃N₃O₂: 303.1008).

### Example 89: 6-(3-Chloro-8-methoxy-2-(trifluoromethyl)imidazo[1,2-a]pyridin-5-yl)quinolin-2(1H)-one:

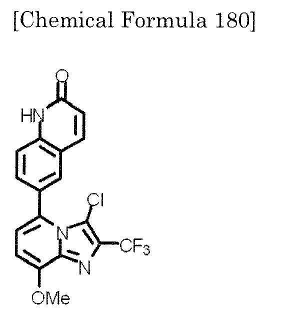

The same procedures used in Example 47 were carried out except for using the compound of Example 20 and the compound of Example 35 to give a desired product as a white solid.

¹H NMR (DMSO-d₆, 400 MHz) : δ 4.00 (3H, s), 6.56 (1H, dd, J = 9.2, 1.8 Hz), 6.93 (1H, d, J = 8.6 Hz), 6.96 (1H, d, J = 7.9 Hz), 7.35 (1H, d, J = 8.6 Hz), 7.64 (1H, dd, J = 8.6, 1.8 Hz), 7.83 (1H, d, J = 1.8 Hz), 7.93 (1H, d, J = 9.2 Hz), 11.94 (1H, s).
Elemental Analysis: Found: C 53.18%, H 2.78%, N 9.93%; Calculated (for C_{1 8} H₁₁ClF₃ N₃ O₂ · 0.75H2 O): C 53.08%, H 3.09%, N 10.32%.

### Example 90: 6-(8-Methoxy-2-trifluoromethylimidazo[1,2-a]pyridin-5-yl)quinolin-2(1H)-one:

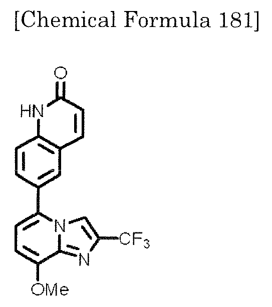

A mixture of the compound of Example 89 (39.4 mg), sodium formate (10.2 mg), palladium acetate (2.25 mg), tri(cyclohexyl) phosphine (5.61 mg) and DMF (1.00 mL) was stirred under an argon gas atmosphere at 80°C for 2 hours and then 120°C for one hour. The reaction liquid was concentrated under reduced pressure and the resulting residue was purified by the silica gel column chromatography (chloroform: methanol = 9: 1) to give a desired product (2.10 mg) as a white solid.

¹ H NMR (DMSO-d₆, 400 MHz) : δ 3.99 (3H, s), 6.58 (1H, dd, J = 9.2, 1.8 Hz), 6.95 (1H, d, J = 8.6 Hz), 6.99 (1H, d, J = 8.6 Hz), 7.46 (1H, d, J = 8.6 Hz), 7.77 (1H, dd, J = 8.6, 1.8 Hz), 7.97-8.00 (2H, m) 8.30 (1H, d, J = 1.8 Hz), 11.97 (1H, s).
HREIMS (+) : 359.0876 (Calculated for C_{1 8} H₁₂ F₃ N₃ O₂: 359.0882).

### Example 91: 6-(7-Methoxy-2-trifluoromethylpyrazolo[1,5-a]pyridin-4-yl)-1-methyl quinolin-2(1H)-one:

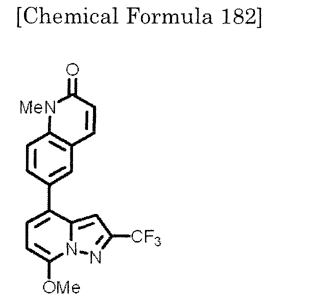

The compound of Example 50 (108 mg) was dissolved in DMF (3.0 mL) under an argon gas atmosphere, 60% sodium hydride (14.4 mg) was added to the solution at 0°C, the resulting mixture was stirred at ordinary temperature for 20 minutes, iodomethane (0.0281 mL) was added to the reaction liquid and the mixture was further stirred for 30 minutes. The reaction liquid was extracted three times with ethyl acetate after the addition of water to the liquid. The combined organic layers were washed with saturated brine, dried over anhydrous sodium sulfate and the solvent was then distilled off under reduced pressure. The resulting residue was purified by the silica gel column chromatography (hexane: ethyl acetate = 1:1→ethyl acetate) to give a desired product (103 mg) as colorless powder.

¹ H NMR (CDCl₃ , 400 MHz): δ 3.79 (3H, s), 4.24 (3H, s), 6.38 (1H, d, J = 7.9 Hz), 6.80 (1H, d, J = 9.2 Hz), 6.94 (1H, s), 7.31 (1H, d, J = 7.3 Hz), 7.51 (1H, d, J = 9.2 Hz), 7.76-7.81 (3H, m).
HREIMS (+): 373.1071 (Calculated for C₁₉H₁₄ F₃ N₃ O₂: 373.1038).
Elemental Analysis: Found: C 60.97%, H 3.75%, N 11.20%; Calculated (for C₁₉H₁₄F₃N₃O₂): C 61.12%, H 3.78%, N11.26%.

### Example 92: Ethyl 2-(6-(7-methoxy-2-trifluoromethylpyrazolo[1,5-a]pyridin-4-yl)-2-oxo- quinolin-2(1H)-yl) acetate:

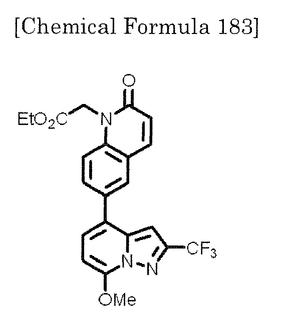

60% Sodium hydride (26.7 mg) was added to a solution of the compound of Example 50 (200 mg) in DMF (5.5 mL) at 0°C under an argon gas atmosphere and the mixture was stirred at ordinary temperature for 30 minutes. Ethyl bromoacetate (0.0931 mL) was added to the mixture at that temperature and the resulting mixture was stirred likewise at that temperature for 30 minutes. Water was added to the reaction liquid and then the mixture was extracted twice with ethyl acetate. The combined extracts were washed, in order, with water and then saturated brine and then dried over anhydrous sodium sulfate. The solvent was then distilled off under reduced pressure and the resulting residue was purified by the silica gel column chromatography (ethyl acetate: hexane = 1:2→1:0) and then washed with diisopropyl ether to give a desired product (175 mg) as a white solid.

¹ H NMR (DMSO-d₆, 400 MHz): δ 1.23 (3H, t, J = 7.3 Hz), 4.13-4.22 (5H, m), 5.14 (2H, s), 6.72 (1H, d, J = 9.8 Hz), 6.77 (1H, d, J = 8.6 Hz), 7.27 (1H, s), 7.54- 7.61 (2H, m), 7.88 (1H, dd, J = 8.4, 2.4 Hz), 8.08-8.15 (2H, m).
EIMS (+): 445 [M]⁺.
Elemental Analysts: Found: C 59.08%, H 3.96%, N 9.01%; Calculated (for C₁₆ H₁₀Cl₂ F₃ N₃ O₃ · 0.2H₂ O): C 58.85%, H 4.13%, N 9.36%.

### Example 93: 2-(6-(Methoxy-2-trifluoromethylpyrazolo[1,5-a]pyridin-4-yl)-2-oxo quinolin-2(1H)-yl) acetic acid:

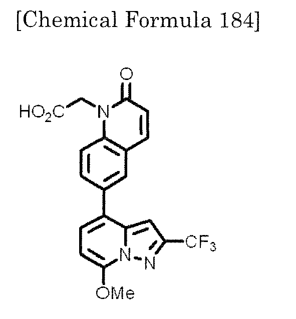

The compound of Example 92 (80.0 mg) was dissolved in a mixture of methanol (1.2 mL) and water (0.6 mL), potassium hydroxide (30.3 mg) was added to the solution and the mixture was stirred at ordinary temperature for 2 hours. Water was added to the reaction solution, the mixture was washed with ethyl acetate, the pH value of the resulting aqueous layer was adjusted to 2 to 3 by the addition of 1 mol/L hydrochloric acid and then the aqueous layer was extracted twice with ethyl acetate. The combined extracts were dried over anhydrous sodium sulfate, the solvent was distilled off under reduced pressure and the resulting residue was washed with diisopropyl ether to give a desired product (40.0 mg) as a brown solid.

¹H NMR (DMSO-d₆, 400 MHz): δ 4.20 (3H, s), 5.06 (2H, s), 6.72 (1H, d, J = 8.6 Hz), 6.78 (1H, d, J = 7.9 Hz), 7.28 (1H, s), 7.56 (1H, d, J = 7.9 Hz), 7.90 (1H, dd, J = 8.6, 2.1 Hz), 8.09-8.13 (2H, m).
HREIMS (+): 417.0972 (Calculated for C₂₀ H₁₄ F₃ N₃ O₄: 417.0936).

### Example 94: 1-Benzy-6-(7-methoxy-2-trifluoromethylpyrazolo[1,5-a]pyridin-4-yl) qumolin-2(1H)-one:

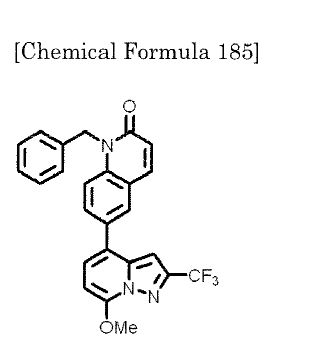

60% Sodium hydride (13.4 mg) was added to a solution of the compound of Example 50 (100 mg) in DMF (3.0 mL) at 0°C under an argon gas atmosphere and the mixture was stirred at ordinary temperature for 40 minutes. Benzyl bromide (0.0496 mL) was added to the mixture at that temperature and the resulting mixture was stirred likewise at that temperature for 40 minutes. Water was added to the reaction liquid and then the mixture was extracted three times with ethyl acetate. The combined extracts were washed, in order, with water and then saturated brine and then dried over anhydrous sodium sulfate. The solvent was then distilled off under reduced pressure and the resulting residue was purified by the silica gel column chromatography (ethyl acetate: hexane = 1:1→ethyl acetate) and then washed with diisopropyl ether to thus give a desired product (52.0 mg) as a white solid.

¹H NMR (DMSO-d₆, 400 MHz): δ 4.18 (3H, s), 5.58 (2H, s), 6.75 (1H, d, J = 8.6 Hz), 6.81 (1H, d, J = 9.8 Hz), 7.22-7.28 (4H, m), 7.31-7.37 (2H, m), 7.52 (1H, d, J = 9.2 Hz), 7.56 (1H, d, J = 8.0 Hz), 7.82 (1H, dd, J = 9.2, 2.4 Hz), 8.11 (1H, d, J = 2.4 Hz), 8.15 (1H, d, J = 9.8 Hz).
EIMS (+): 449 [M]⁺.
Elemental Analysis: Found: C 66.54%, H 4.13%, N 8.98%; Calculated (for C_{2 5} H₁₈F₃ N₃ O₂ 0.1H₂ O): C 66.54%, H 4.07%, N 9.31%.

### Example 95: 1-(3-Bromopropyl)-6-(7-methoxy-2-trifluoromethylpyrazolo[1,5-a] pyridin-4-yl)quinolin-2(1H)-one:

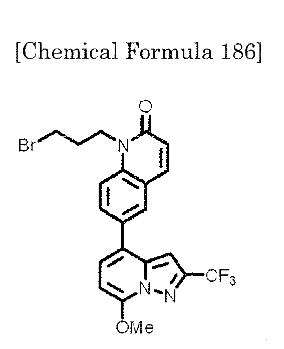

60% Sodium hydride (36.8 mg) was added to a solution of the compound of Example 50 (300 mg) in DMF (8.0 mL) at 0°C and the mixture was stirred at ordinary temperature for 30 minutes. 1,3-Dibromopropane (0.0424 mL) was added to the mixture at 0°C and the resulting mixture was stirred at ordinary temperature for 3 hours. A saturated aqueous ammonium chloride solution was added to the reaction liquid and then the mixture was extracted three times with ethyl acetate. The combined extracts were washed, in order, with water and then with saturated brine and then dried over anhydrous sodium sulfate. The solvent was then distilled off under reduced pressure and the resulting residue was purified by the silica gel column chromatography (ethyl acetate: hexane = 2:3→1:0) to give a desired product (177 mg) as a yellow solid.

¹H NMR (DMSO-d₆, 400 MHz): δ 2.16-2.23 (2H, m), 3.68 (2H, t, J = 6.7 Hz), 4.18 (3H, s), 4.34-4.42 (2H, m), 6.68 (1H, d, J = 9.8 Hz), 6.76 (1H, d, J = 7.9 Hz), 7.27 (1H, s), 7.59 (1H, d, J = 7.9 Hz), 7.72 (1H, d, J = 8.9 Hz), 7.92 (1H, dd, J = 8.9, 2.1 Hz), 8.05 (1H, d, J = 9.8 Hz), 8.08 (1H, d, J = 2.1 Hz).
EIMS (+): 479 [M]⁺.

### Example 96: 1-(3-(Ethylamino)propyl)-6-(7-methoxy-2-trifluoromethylpyrazolo [1,5-a]pyridin-4-yl)quinolin-2(1H)-one:

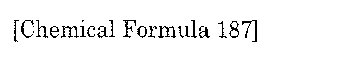

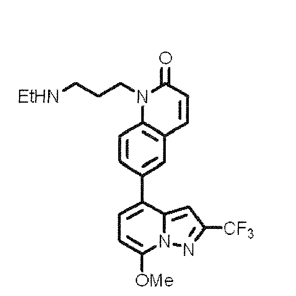

To a solution of the compound of Example 95 (80.0 mg) in THF (1.0 mL) was added a 2.0 mol/L solution of ethylamine in THF (0.835 mL) at ordinary temperature and the resulting mixture was allowed to stand within a sealed tube at 50°C for 6 hours. The ethylamine and the THF were distilled off under reduced pressure, the resulting residue was purified by the aminated silica gel column chromatography (ethyl acetate: methanol = 20:1) and then washed with diisopropyl ether to give a desired product (60.6 mg) as a yellow solid.

¹H NMR (DM80-d₆, 400 MHz) : δ 1.02 (3H, t, J = 7.3 Hz), 1.75-1.82 (2H, m), 2.51-2.60 (4H, m), 4.18 (3H, s), 4.32 (2H, t, J = 7.3 Hz), 6.67 (1H, d, J = 9.8 Hz), 6.76 (1H, d, J = 7.9 Hz), 7.26 (1H, s), 7.58 (1H, d, J = 7.9 Hz), 7.74 (1H, d, J = 9.2 Hz), 7.91 (1H, dd, J = 9.2, 2.1 Hz), 8.03 (1H, d, J = 9.8 Hz), 8.07 (1H, d, J = 2.1 Hz).
HREIMS (+) : 444.1757 (Calculated for C₂₃ H₂₃ F₃ N₄ O₂: 444.1773).

### Example 97: 6-(7-Methoxy-2-trifluoromethylpyrazolo[1,5-a]pyridin-4-yl)-1-(3-morpholin-4-ylpropyl)quinolin-2(1H)-one:

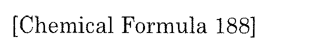

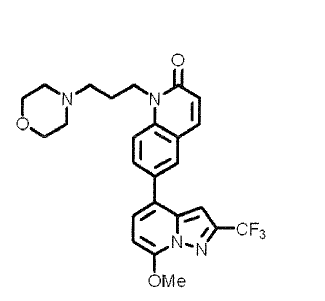

Morpholine (0.0728 mL) was added to a solution of the compound of Example 95 (80.0 mg) in THF (1.7 mL) at ordinary temperature under an argon gas atmosphere and the mixture was stirred at 50°C for 6 hours. Water was added to the reaction liquid and the mixture was extracted twice with ethyl acetate. The combined extracts were washed, in order, with water and then with saturated brine and then dried over anhydrous sodium sulfate. The solvent was then distilled off under reduced pressure and the resulting residue was purified by the aminated silica gel column chromatography (ethyl acetate: methanol = 3:1) and then washed with diisopropyl ether to give a desired product (66.0 mg) as a white solid.

¹ H NMR (DMSO-d₆, 400 MHz): δ 1.81 (2H, t, J = 7.3 Hz), 2.30-2.42 (6H, m), 3.57 (2H, t, J = 4.3 Hz), 4.19 (3H, s), 4.31 (2H, t, J = 7.3 Hz), 6.66 (1H, d, J = 9.2 Hz), 6.76 (1H, d, J = 7.9 Hz), 7.26 (1H, s), 7.59 (1H, d, J = 7.9 Hz), 7.76 (1H, d, J = 9.2 Hz), 7.91 (1H, dd, J = 9.2, 2.1 Hz), 8.04 (1H, d, J = 9.2 Hz), 8.07 (1H, d, J = 2.1 Hz).
HREIMS (+): 486.1900 (Calculated for C₂₅ H₂₅ F₃ N₄O₃: 486.1879).

### Example 98: 1-(3-(Dimethylamino)propyl)-6-(7-methoxy-2-trifluoromethylpyrazolo [1,5-a]pyridin-4-yl)quinolin-2(1H)-one

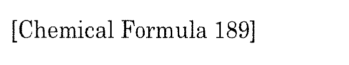

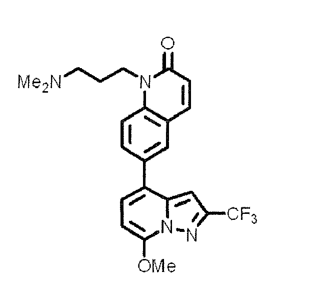

To a solution of the compound of Example 95 (185 mg) in THF (1.0 mL) was added a 2.0 mol/L solution of dimethylamine in THF (1.93 mL) at ordinary temperature and the resulting mixture was allowed to stand within a sealed tube at 50°C for 2 hours, at 60°C for 2 hours and at 75°C for one hour. The dimethylamine and the THF were distilled off under reduced pressure, the resulting residue was purified by the aminated silica gel column chromatography (ethyl acetate: methanol = 3:1) and then washed with diisopropyl ether to give a desired product (125 mg) as a yellowish green-colored solid.

¹ H NMR (DMSO-d₆, 400 MHz): δ 1.81-1.88 (2H, m), 2.25 (6H, s), 2.43 (2H, t, J = 7.0 Hz), 4.25 (3H, s), 4.35 (2H, t, J = 7.0 Hz), 6.73 (1H, d, J = 9.8 Hz), 6.83 (1H, d, J = 7.9 Hz), 7.33 (1H, s), 7.65 (1H, d, J = 7.9 Hz), 7.79 (1H, d, J = 9.2 Hz), 7.98 (1H, dd, J = 9.2, 2.1 Hz), 8.10 (1H, d, J = 9.8 Hz), 8.13 (1H, d, J = 2.1 Hz).
HRESIMS (+): 445.1849 (Calculated for C₂₃ H₂₄ F₃ N₄ O₂: 445.1851).

### Example 99: 3-(6-(7-Methoxy-2-trifluoromethylpyrazolo[1,5-a]pyridin-4-yl)-2-oxo-quinolin-2(1H)-yl)-N,N,N-trimethylpropane-1-ammonium iodide:

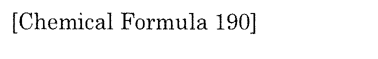

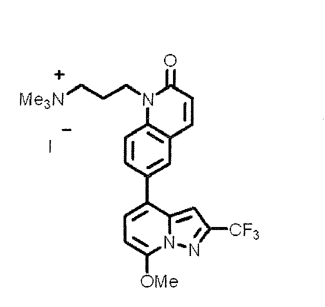

To a solution of the compound of Example 98 (50.0 mg) in acetone (0.2 mL) was added iodomethane (0.0700 mL) at ordinary temperature and the mixture was allowed to stand at that temperature for one hour. The insoluble material thus formed was collected by filtration and then washed, in order, with acetone and diisopropyl ether to give a desired product (42.0 mg) as a white solid.

¹H NMR (DMSO-d₆, 400 MHz): δ 2.10-2.17 (2H, m), 3.06 (9H, s), 3.48-3.52 (2H, m), 4.19 (3H, s), 4.34 (2H, t, J = 7.0 Hz), 6.71 (1H, d, J = 9.2 Hz), 7.22 (1H, s), 7.60 (1H, d, J = 7.9 Hz), 7.78 (1H, d, J = 9.2 Hz), 7.93 (1H, dd, J = 9.2, 2.1 Hz), 8.09 (1H, d, J = 9.2 Hz), 8.12 (1H, d, J = 2.1 Hz).
ESIMS (+): 459 [M-HI]⁺.
Elemental Analysis: Found: C 48.72%, H 4.27%, N 9.11%; Calculated (for C₂₄ H₂₆ F₃ IN₄ O₂ · 0.4H₂O): C 48.56%, H 4.55%, N 9.44%.

### Example 100: 6-(7-Methoxy-2-trifluoromethylpyrazolo[1,5-a]pyridin-4-yl)-2-methyl-3,4-dihydroisoquinolin-1(2H)-one:

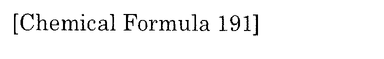

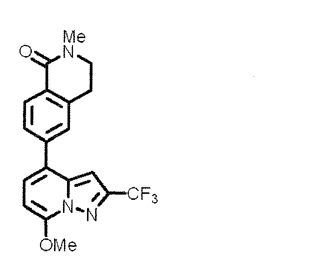

The same procedures used in Example 91 were carried out except for using the compound prepared in Example 58 to give a desired product as a white solid.

¹ H NMR (DMSO-d₆, 400 MHz): δ 3.05 (3H, s), 3.08 (2H, t, J = 6.7 Hz), 3.59 (2H, t, J = 6.7 Hz), 4.18 (3H, s), 6.76 (1H, d, J = 7.9 Hz), 7.24 (1H, s), 7.58-7.61 (2H, m), 7.64 (1H, dd, J = 7.9, 1.8 Hz), 7.98 (1H, d, J = 7.9 Hz).
EIMS (+): 375 [M]⁺.
Elemental Analysis: Found: C 60.53%, H 4.24%, N 11.08%; Calculated (for C₁₉ H₁₆ F₃ N₃ O₂: C 60.80%, H 4.30%, N 11.20%.

### Example 101: 2-Benzyl-6-(7-methoxy-2-trifluoromethylpyrazolo[1,5-a]pyridin-4-yl)-3,4- dihydroisoquinolin-1 (2H)-one:

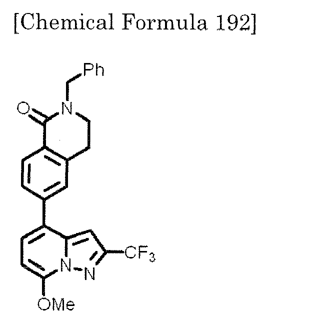

The same procedures used in Example 94 were carried out except for using the compound prepared in Example 58 to give a desired product as a white solid.

¹H NMR (DMSO-d₆, 400 MHz): δ 3.06 (2H, t, J = 6.7 Hz), 3.54 (2H, t, J = 6.7 Hz), 4.18 (3H, s), 6.76 (1H, d, J = 7.9 Hz), 7.24-7.38 (6H, m), 7.57-7.61 (2H, m), 7.67 (1H, dd, J = 7.9, 1.8 Hz), 8.04 (1H, d, J = 7.9 Hz).
EIMS (+): 451 [M]⁺ .
Elemental Analysis: Found: C 65.25%, H 4.37%, N 8.99%; Calculated (for C₂₅H₂₀F₃N₃O₂ · 0.5H₂O): C 65.21%, H 4.60%, N 9.13%.

### Example 102: 6-(7-Methoxy-2-trifluoromethylpyrazolo[1,5-a]pyridin-4-yl)-1-methyl-3,4-dihydroquinolin-2(1H)-one:

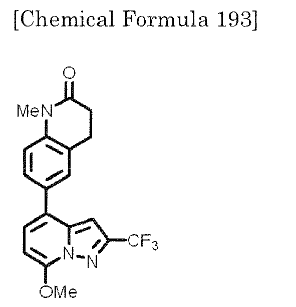

The same procedures used in Example 91 were carried out except for using the compound prepared in Example 56 to give a desired product as a pale yellow solid.

¹H NMR (DMSO-d₆, 400 MHz): δ 2.66 (2H, t, J = 7.3 Hz), 3.04 (2H, t, J = 7.3 Hz), 3.38 (3H, s), 4.24 (3H, s), 6.79 (1H, d, J = 7.9 Hz), 7.24 (1H, s), 7.28 (1H, d, J = 7.9 Hz), 7.56 (1H, d, J = 7.9 Hz), 7.60-7.65 (2H, m).
EIMS (+): 375 [M]⁺ .
Elemental Analysis: Found: C 60.40%, H 4.35%, N 10.90%; Calculated (for C₁₉H₁₆ F₃ N₃ O₂ · 0.15H₂ O): C 60.36%, H 4.35%, N 11.12%.

### Example 103: 1-Benzyl-6-(7-methoxy-2-trifluoromethylpyrazolo[1,5-a]pyridin-4-yl)-3,4-dihydroquinolin-2(1H)-one:

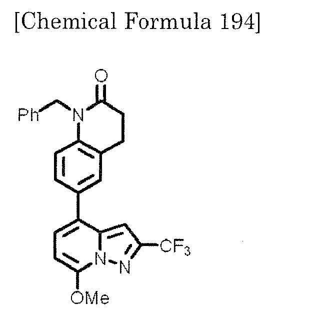

The same procedures used in Example 94 were carried out except for using the compound prepared in Example 56 to give a desired product as a white solid.

¹H NMR (DMSO-d₆, 400 MHz): δ 2.73-2.78 (2H, m), 3.06 (2H, t, J = 7.3 Hz), 4.15 (3H, s), 5.18 (2H, s), 6.69 (1H, d, J = 7.9 Hz), 7.03 (1H, s), 7.19-7.35 (5H, m), 7.39-7.46 (2H, m), 7.56 (1H, d, J = 1.8 Hz).
EIMS (+): 451 [M]⁺.
Elemental Analysis: Found: C 65.93%, H 4.50%, N 9.11%; Calculated (for C_{2 5} H₂₀F₃ N₃ O₂ · 0.2H₂ O): C 65.99%, H 4.52%, N 9.23%.

### Example 104: Ethyl 2-(6-(7-methoxy-2-trifluoromethylpyrazolo[1,5-a]pyridin-4-yl)-1-oxo-3,4-dihydroisoquinolin-1(2H)-yl)acetate:

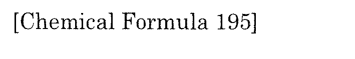

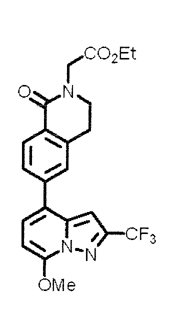

The same procedures used in Example 92 were carried out except for using the compound prepared in Example 56 to give a desired product as a white solid.

¹ H NMR (CDCl₃, 400 MHz): δ 1.31 (3H, t, J = 7.0 Hz), 3.16 (2H, t, J = 6.4 Hz), 3.74 (2H, t, J = 6.4 Hz), 4.23 (3H, s), 4.24 (2H, q, J = 7.0 Hz), 4.38 (2H, s), 6.37 (1H, d, J = 7.9 Hz), 6.96 (1H, s), 7.33 (1H, d, J = 7.9 Hz), 7.44 (1H, d, J = 1.8 Hz), 7.58 (1H, dd, J = 7.9, 1.8 Hz), 8.21 (1H, d, J = 7.9 Hz).
EIMS (+): 448 [M]⁺ .
Elemental Analysis: Found: C 59.06%, H 4.51%, N 9.39%; Calculated (for C₂₂H₂₀F₃N₃O₄): C 58.84%, H 4.74%, N 9.16%.

### Example 105: 2-(6-(7-Methoxy-2-trifluoromethylpyrazolo[1,5-a]pyridin-4-yl)-1-oxo-3,4-dihydroisoquinolin-1(2H)-yl) acetic acid

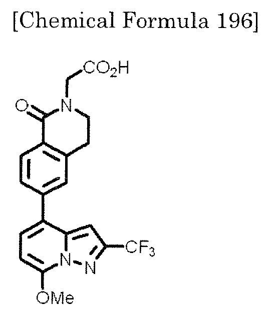

The same procedures used in Example 93 were carried out except for using the compound prepared in Example 104 to give a desired product as a white solid.

¹H NMR (CDCl₃, 400 MHz): δ 3.17 (2H, t, J = 6.4 Hz), 3.76 (2H, t, J = 6.4 Hz), 4.23 (3H, s), 4.42 (2H, s), 6.36 (1H, d, J = 7.9 Hz), 6.95 (1H, s), 7.31 (1H, d, J = 7.9 Hz), 7.44 (1H, d, J = 1.5 Hz), 7.58 (1H, dd, J = 7.9, 1.5 Hz), 8.20 (1H, d, J = 7.9 Hz).
EIMS (+): 419 [M]⁺.
Elemental Analysis: Found: C 55.36%, H 4.01%, N 9.42%; Calculated (for C₂₀H₁₆ F₃ N₃ O₄ · 0.8H2 O): C 55.38%, H 4.09%, N 9.69%.

### Example 106: 4-(7-Methoxy-2-trifloromethylpyrazolo[1,5-a]pyridin-4-yl)-1-methylquinolin-2(1H)-one:

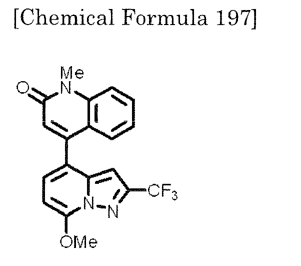

The same procedures used in Example 91 were carried out except for using the compound prepared in Example 51 to give a desired product as colorless powder.

¹H NMR (CDCl₃, 400 MHz): δ 3.82 (3H, s), 4.27 (3H, s), 6.41 (1H, d, J = 7.9 Hz), 6.56 (1H, s), 6.82 (1H, s), 7.14-7.18 (1H, m), 7.31 (1H, d, J = 7.9 Hz), 7.37 (1H, dd, J = 7.9, 1.2 Hz), 7.49 (1H, d, J = 7.9 Hz), 7.62-7.64 (1H, m).
HREIMS (+): 373.1011 (Calculated for C₁₉H₁₄ F₃ N₃ O₂: 373.1038).
Elemental Analysis: Found: C 60.98%, H 3.79%, N 11.18%; Calculated (for C₁₉ H₁₄ F₃ N₃ O₂): C 61.13%, H 3.78%, N 11.26%.

### Example 107: 1-Benzyl-4-(7-methoxy-2-trifluoromethylpyrazolo[1,5-a]pyridin-4-yl) quinolin-2(1H)-one:

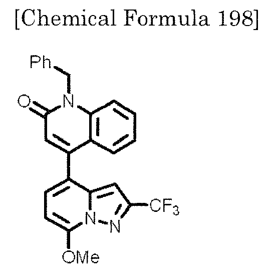

The same procedures used in Example 94 were carried out except for using the compound prepared in Example 51 to give a desired product as colorless powder.

¹H NMR (CDCl₃, 400 MHz): δ 4.28 (3H, s), 5.58 (1H, brd, J = 17.1 Hz), 5.72 (1H, brd, J = 17.1 Hz), 6.42 (1H ,d, J = 7.9 Hz), 6.62 (1H, s), 6.90 (1H, s), 7.09-7.11 (1H, m), 7.31-7.37 (8H, m), 7.45-7.50 (1H, m).
HREIMS (+): 449.1337 (Calculated for C_{2 5} H₁₈F₃ N₃ O₂: 449.1351).
Elemental Analysis: Found: C 66.81%, H 4.04%, N 9.35%; Calculated (for C₂₅ H₁₈ F₃ N₃ O₂): C 66.75%, H 4.05%, N 9.30%.

### Example 108: 5-(7-Methoxy-2-trifluoromethylpyrazolo[1,5-a]pyridin-4-yl)-2-methylisoindolin-1-one

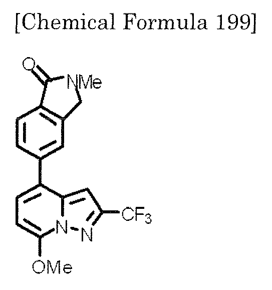

The same procedures used in Example 91 were carried out except for using the compound prepared in Example 59 to give a desired product as colorless powder.

¹ H NMR (CDCl₃, 400 MHz): δ 3.25 (3H, s), 4.24 (3H, s), 4.48 (2H, s), 6.38 (1H, d, J = 7.9 Hz), 6.94 (1H ,s), 7.33 (1H, d, J = 7.9 Hz), 7.68-7.69 (2H, m), 7.96 (1H, d, J = 9.2 Hz). HREIMS (+): 361.1061 (Calculated for C₁₈H₄₈F₃N₃O₂: 361.1038).

### Example 109: 2-Benzyl-5-(7-methoxy-2-trifluoromethylpyrazolo[1,5-a]pyridin-4-yl) isoindolin-1-one

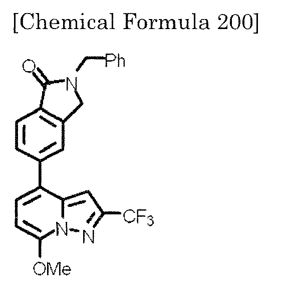

The same procedures used in Example 94 were carried out except for using the compound prepared in Example 59 to give a desired product as yellow powder.

¹H NMR (CDCl₃, 400 MHz) : δ 4.23 (3H, s), 4.36 (2H, s), 4.85 (2H, s), 6.37 (1H, d, J = 7.9 Hz), 6.91 (1H, s), 7.29-7.36 (6H, m), 7.62 (1H, s), 7.69 (1H, d, J = 7.9 Hz), 8.02 (1H, d, J = 7.9 Hz).
EIMS (+): 437 [M]⁺.
Elemental Analysis: Found: C 65.67%, H 4.38%, N 9.43%; Calculated (for C₂₄H₁₈F₃N₃O₂): C 65.90%, H 4.15%, N 9.61%.

### Example 110: 2-(7-Methoxy-2-(trifluoromethyl)benzo[b]thiophen-4-yl)-4,4,5,5-tetramethyl-1,3,2-dioxaborolane:

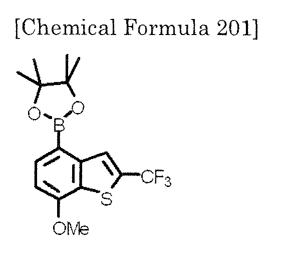

The same procedures used in Example 32 were carried out except for using the compound prepared in Example 18 to give a desired product as colorless powder.

¹ H NMR (CDCl₃, 400 MHz): δ 1.39 (12H, s), 4.03 (3H, s), 6.87 (1H, d, J = 8.0 Hz), 7.93 (1H, d, J = 8.0 Hz), 8.31 (1H, d, J = 1.2 Hz).

### Example 111: 2-(7-Methoxy-2-(trifluoromethyl)benzo[b]thiophen-4-yl)quinolin-2(1H)-one:

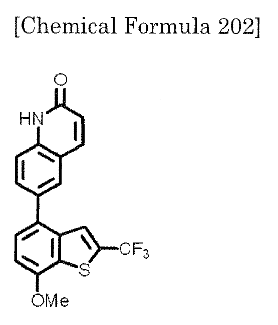

The same procedures used in Example 47 were carried out except for using the compound prepared in Example 110 and 6-bromoquinolin-2(1H)-one to give a desired product as colorless powder.

¹H NMR (CDCl₃, 400 MHz): 6 4.07 (3H, s), 6.79 (1H, d, J = 9.2 Hz), 6.98 (1H, d, J = 8.6 Hz), 7.66-7.72 (3H, m), 7.88 (1H, d, J = 9.2 Hz), 11.30 (1H, brs).
HREIMS (+): 375.0517 (Calculated for C₁₉ H₁₂ F₃ NO₂ S: 375.0541).

### Example 112: 6-(2-Ethyl-4-methoxybenzo[d]oxazol-7-yl)quinolin-2(1H)-one:

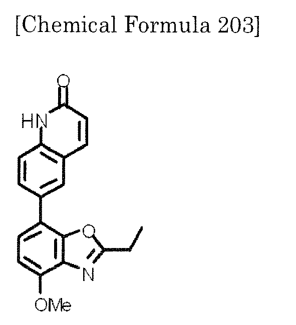

The same procedures used in Example 47 were carried out except for using the compound prepared in Example 35 and 7-bromo-2-ethyl-4-methoxybenzo[d]oxazole to give a desired product as pale brown powder.

¹H NMR (DMSO-d₆, 400 MHz): δ 1.36 (3H, t, J = 7.6 Hz), 2.97 (2H, q, J = 7.6 Hz), 3.98 (3H, s), 6.53 (1H, dd, J = 8.6, 1.8 Hz), 7.01 (1H, d, J = 8.6 Hz), 7.42 (1H, d, J = 8.6 Hz), 7.56 (1H, d, J = 8.6 Hz), 7.95-8.00 (2H, m), 8.09 (1H, d, J = 1.8 Hz), 11.84(1H, s).
HREIMS (+): 320.1186 (Calculated for C₁₉ H₁₆ N₂ O₃: 320.1161).

### Example 113: 8-Methoxy-5,6'-biqumolin-2,2'(1H,1'H)-dione:

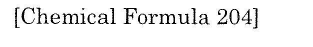

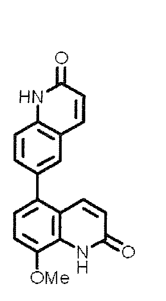

The same procedures used in Example 47 were carried out except for using the compound prepared in Example 35 and 5-bromo-8-methoxyquinolin-2(1H)-one to give a desired product as yellow powder.

¹H NMR (DMSO-d₆, 400 MHz): δ 3.92 (3H, s), 6.47-6.54 (2H, m), 7.09 (1H, d, J = 7.9 Hz), 7.21 (1H, d, J = 7.9 Hz), 7.39 (1H, d, J = 8.6 Hz), 7.50 (1H, dd, J = 8.6, 1.8 Hz), 7.68-7.70 (2H, m), 7.94 (1H, d, J = 9.8 Hz), 10.84 (1H, s), 11.84 (1H, s).
HREIMS (+): 318.1050 (Calculated for C₁₉ H₁₄ N₂ O₃: 318.1004).

### Example 114: 6-(2-Cyclopropyl-7-methoxypyrazolo[1,5-a]pyridin-4-yl)quinolin-2(1H)-one:

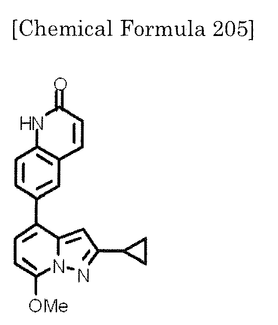

The same procedures used in Example 47 were carried out except for using the compound prepared in Example 35 and the compound prepared in Example 9 to give a desired product as a pale yellow solid.

¹H NMR (DMSO-d₆ , 400 MHz): δ 0.82-0.88 (2H, m), 0.89-0.99 (2H, m), 2.05-2.09 (1H, m), 4.06 (3H, s), 6.37 (1H, d, J = 7.9 Hz), 6.49 (1H, s), 6.54 (1H, d, J = 9.8 Hz), 7.25 (1H, d, J = 7.9 Hz), 7.40 (1H, d, J = 8.6 Hz), 7.77 (1H, dd, J = 8.6, 1.8 Hz), 7.93 (1H, s), 7.99 (1H, d, J = 9.8 Hz), 11.83 (1H, s).
Elemental Analysis: Found: C 72.15%, H 5.17%, N 12.41%; Calculated (for C₂₄H₁₈F₃N₃O₂): C 72.49%, H 5.17%, N 12.68%.

### Example 115: 6-(3-Chloro-7-methoxy-2-trifluoromethylpyrazolo[1,5-a]pyridin-4-yl)-1-methylquinolin-2(1H)-one:

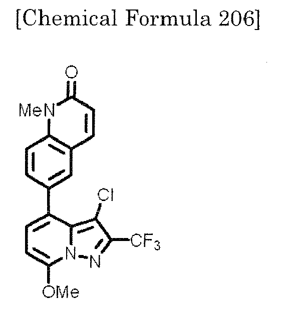

The same procedures used in Example 91 were carried out except for using the compound prepared in Example 61 to give a desired product as a white solid.

¹ H NMR (CDCl₃ , 400 MHz): δ 3.79 (3H, s), 4.24 (3H, s), 6.36 (1H, d, J = 7.9 Hz), 6.78 (1H, d, J = 9.8 Hz), 7.20 (1H, d, J = 7.3 Hz), 7.44 (1H, d, J = 7.9 Hz), 7.60-7.65 (2H, m), 7.72 (1H, d, J = 9.8 Hz).
EIMS (+): 407 [M]⁺.
Elemental Analysis: Found: C 55.74%, H 3.35%, N 10.16%; Calculated (for C₁₉ H₁₃ ClF₃ N₃ O₂): C 55.96%, H 3.21%, N 10.30%.

### Example 116: 6-(3-Chloro-7-methoxy-2-trifluoromethylpyrazolo[1,5-a]pyridin-4-yl)-3,4-dihydroquinolin-2(1H)-one:

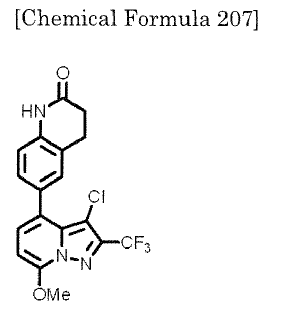

The same procedures used in Example 47 were carried out except for using 6-(4,4,5,5-tetramethyl[1,3,2]dioxaborolan-2-yl)-3,4-dihydroquinolin-2(1H)-one and the compound prepared in Example 22 to give a desired product as a white solid.

¹ H NMR (CDCl₃, 400 MHz): δ 2.68-2.73 (2H, m), 3.01-3.07 (2H, m), 4.22 (3H, s), 6.33 (1H, d, J = 7.9 Hz), 6.83 (1H, d, J = 4.3 Hz), 7.14 (1H, d, J = 7.9 Hz), 7.20-7.25 (2H, m), 8.01 (1H, s).
EIMS (+): 395 [M]⁺.
Elemental Analysis: Found: C 54.68%, H 3.50%, N 10.24%; Calculated (for C₁₈ H₁₃ ClF₃ N₃ O₂): C 54.63%, H 3.31%, N 10.62%.

### Example 117: 6-(3-Chloro-7-methoxy-2-trifluoromethylpyrazolo[1,5-a]pyridin-4-yl)-1-methyl-3,4-dihydroquinolin-2(1H)-one:

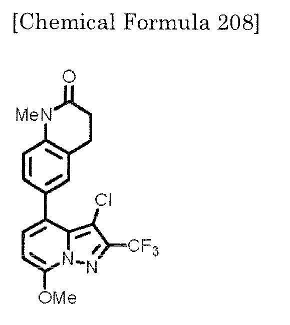

The same procedures used in Example 91 were carried out except for using the compound prepared in Example 116 to give a desired product as a white solid.

¹H NMR (CDCl₃, 400 MHz): δ 2.69-2.74 (2H, m), 2.94-3.00 (2H, m), 3.42 (3H, s), 4.22 (3H, s), 6.33 (1H, d, J = 7.9 Hz), 7.05 (1H, d, J = 8.6 Hz), 7.15 (1H, d, J = 7.9 Hz), 7.20-7.23 (1H, m), 7.31 (1H, dd, J = 8.6, 2.1 Hz).
EIMS (+): 409 [M]⁺.
Elemental Analysis: Found: C 55.43%, H 3.65%, N 10.19%; Calculated (for C₁₉H₁₅ ClF₃ N₃ O₂): C 55.69%, H 3.69%, N 10.25%.

### Example 118: 6-(4-Methoxy-2-trifluoromethyl-1H-benzo[d]imidazol-7-yl)quinolin-2(1H)-one; or 6-(7-methoxy-2-trifluoromethyl-1H-benzo[d]imidazol-4-yl)quinolin-2(1H)-one:

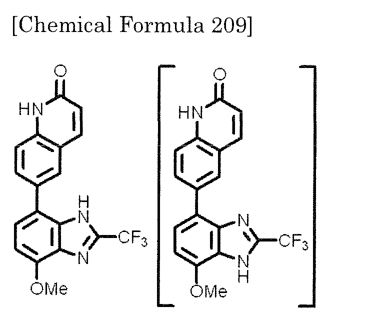

The same procedures used in Example 32 were carried out except for using 7-bromo-4-methoxy-2-trifluoromethyl-1H-benzo[d]imidazole (or 4-bromo-7-methoxy-2-trifluoromethyl-1H-benzo[d]imidazole) to thus form a compound and then the same procedures used in Example 47 were carried out except for using the compound obtained above and 6-bromo-quinolin-2(1H)-one to give a desired product as pale yellow powder.

¹H NMR (CDCl₃, 400 MHz): δ 4.05 (3H, s), 6.65 (1H, d, J = 9.2 Hz), 7.00 (1H, d, J = 8.6 Hz), 7.46 (2H, d, J = 8.6 Hz), 7.55-7.66 (3H, m), 8.05 (1H, d, J = 9.2 Hz), 8.13 (1H, brs). HREIMS(+): 360.09714 (Calculated for C₁₈ H₁₃ F₃ N₃ O₂: 360.09599).

### Example 119:6-(7-Methoxy-2-trifluoromethylbenzofuran-4-yl)quinolin-2(1H)-one:

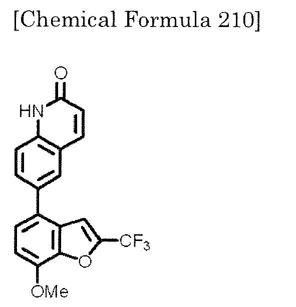

The same procedures used in Example 32 were carried out except for using the compound of Example 14 to thus form a compound and then the same procedures used in Example 47 were carried out except for using the resulting compound and 6-bromoquinolin-2(1H)-one to give a desired product as colorless powder.

¹H NMR (CDCl₃, 400 MHz): δ 4.09 (3H, s), 6.78 (1H, d, J = 9.2 Hz), 7.03 (1H, d, J = 8.0 Hz), 7.30 (1H, d, J = 1.2 Hz), 7.35 (1H, d, J = 8.6 Hz), 7.48 (1H, d, J = 8.0 Hz), 7.71 (1H, dd, J = 8.6, 1.2 Hz), 7.72 (1H, s), 7.89 (1H, d, J = 9.2 Hz), 11.38 (1H, brs).
HRESIMS(+): 359.0768 (Calculated for C₁₉ H₁₂ F₃ NO₃: 359.0769).

### Example 120: 6-(8-Methoxyquinolin-5-yl)quinolin-2(1H)-one:

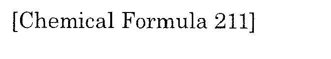

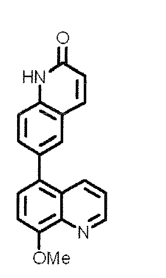

The same procedures used in Example 47 were carried out except for using 5-bromo-8-methoxyquinoline and the compound prepared in Example 35 to give a desired product as a white solid.

¹H NMR (DMSO-d₆, 400 MHz): δ 4.00 (3H, s), 6.55 (1H, dd, J = 9.2, 1.8 Hz), 7.27 (1H, d, J = 7.9 Hz), 7.43 (1H, d, J = 7.9 Hz), 7.49 (1H, d, J = 7.9 Hz), 7.53 (1H, dd, J = 8.6, 4.3 Hz), 7.58 (1H, dd, J = 8.6, 1.8 Hz), 7.75 (1H, d, J = 1.8 Hz), 7.97 (1H, d, J = 9.2 Hz), 8.18 (1H, dd, J = 9.2, 1.8 Hz), 8.87 (1H, dd, J = 4.3, 1.8 Hz), 11.87 (1H, s).
EIMS (+): 302 [M]⁺.
Elemental Analysis: Found: C 75.11%, H 4.70%, N 9.18%; Calculated (for C₁₉H₁₄N₂O₂): C 75.48%, H 4.67%, N 9.27%.

### Example 121: 6-(2-Cyclopropyl-7-methoxypyrazolo[1,5-a]pyridin-4-yl)-3,4-dihydro isoqionolin-1(2H)-one:

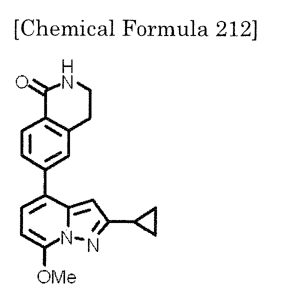

The same procedures used in Example 47 were carried out except for using 6-(4,4,5,5-tetramethyl[1,3,2]dioxaborolan-2-yl)-3,4-dihydroisoquinolin-1(2H)-one and the compound prepared in Example 9 to give a desired product as a yellowish brown-colored solid.

¹H NMR (DMSO-d₆, 400 MHz): δ 0.82-0.86 (2H, m), 0.95-0.99 (2H, m), 2.05-2.11 (1H, m), 2.98 (2H, t, J = 6.4 Hz), 3.39-3.42 (2H, m), 4.09 (3H, s), 6.38 (1H, d, J = 7.9 Hz), 6.50 (1H, s), 7.31(1H, d, J = 7.9 Hz), 7.56 (1H, d, J = 1.8 Hz), 7.60 (1H, dd, J = 7.9, 1.8 Hz), 7.92 (1H, d, J = 7.9 Hz), 7.94 (1H, brs).
Elemental Analysis: Found: C 71.30%, H 5.72%, N 12.36%; Calculated (for C_{2 0} H₁₉ N₃ O₂ · 0.2H2 O): C 71.28%, H 5.80%, N 12.47%.

### Example 122: 6-(7-Methoxy-2-trifluoromethylpyrazolo[1,5-a]pyridin-4-yl)-3-methyl-3,4-dihydroisoqionolin-1(2H)-one:

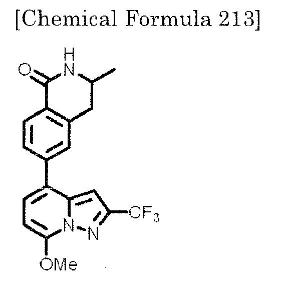

The same procedures used in Example 47 were carried out except for using the compounds prepared in Examples 33 and 31 to give a desired product as colorless powder.

¹H NMR (CDCl₃, 400 MHz): δ 1.22 (3H, d, J = 6.7 Hz), 2.75 (1H, dd, J = 15.9, 10.4 Hz), 3.06 (1H, dd, J = 15.9, 4.3 Hz), 3.72-3.75 (1H, m), 4.18 (3H, s), 6.76 (1H, d, J = 7.9 Hz), 7.59-7.60 (2H, m), 7.64 (1H, dd, J = 7.0, 1.8 Hz), 7.96 (1H, d, J = 7.9 Hz), 7.97 (1H, brs). EIMS (+): 375 [M]⁺.
Elemental Analysis: Found: C 60.81%, H 4.29%, N 10.97%; Calculated (for C₁₉ H₁₀F₃ N₃ O₂): C 60.80%, H 4.30%, N 11.20%.

### Example 123: 6-(3-Chloro-7-methoxy-2-trifluoromethylpyrazolo[1,5-a]pyridin-4-yl)-2-methyl-3,4-dihydroisoqionolin-1(2H)-one:

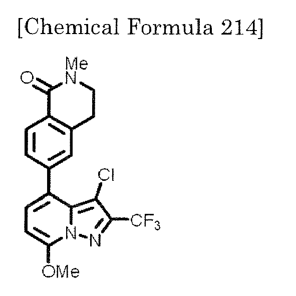

The same procedures used in Example 91 were carried out except for using the compound prepared in Example 62 to give a desired product as a white solid.

¹H NMR (CDCl₃, 400 MHz): δ 3.07 (2H, t, J = 6.7 Hz), 3.20 (3H, s), 3.63 (2H, t, J = 6.7 Hz), 4.23 (3H, s), 6.34 (1H, d, J = 7.9 Hz), 7.19 (1H, d, J = 7.9 Hz), 7.22 (1H, d, J = 1.2 Hz), 7.38 (1H, dd, J = 7.9, 1.2 Hz), 8.16 (1H, d, J = 7.9 Hz).
EIMS (+): 409 [M]⁺.
Elemental Analysis: Found: C 55.61%, H 3.72%, N 10.20%; Calculated (for C_{1 9} H₁₅ ClF₃ N₃ O₂ ): C 55.69%, H 3.69%, N 10.25%.

### Example 124: 4-Chloro-6-(4,4,5,5-tetramethyl[1,3,2]dioxaborolan-2-yl)iso quinolin-1(2H)-one:

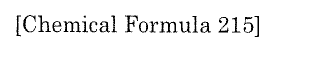

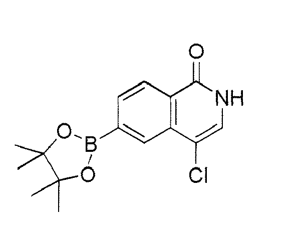

The same procedures used in Example 32 were carried out except for using the compound prepared in Example 25 to give a desired product as yellow powder.

¹ H NMR (CDCl₃, 400 MHz): δ 1.39 (12H, s), 7.30 (1H, s), 8.00 (1H, d, J = 7.9 Hz), 8.37 (1H, s), 8.42 (1H, d, J = 7.9 Hz), 11.05 (1H, s).
EIMS (+): 305 [M]⁺.

### Example 125: 4-Chloro-6-(3-chloro-7-methoxy-2-trifluoromethylpyrazolo[1,5-a] pyridin-4-yl)isoqionolin-1(2H)-one:

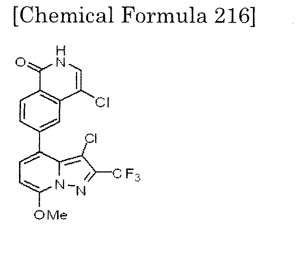

The same procedures used in Example 47 were carried out except for using the compound prepared in Example 22 and the compound prepared in Example 124 to give a desired product as a white solid.

¹H NMR (DMSO-d₆, 400 MHz): δ 4.21 (3H, s), 6.82 (1H, d, J = 7.9 Hz), 7.52-7.56 (2H, m), 7.69 (1H, dd, J = 7.9, 1.8 Hz), 7.82 (1H, d, J = 1.8 Hz), 8.31 (1H, d, J = 7.9 Hz), 11.65 (1H, brd, J = 5.5Hz).
ESIMS (+): 428 [M+H]⁺.
Elemental Analysis: Found: C 50.29%, H 2.35%, N 9.59%; Calculated (for C₁₈ H₁₀Cl₂ F₃ N₃ O₂): C 50.49%, H 2.35%, N 9.81%.

### Example 126: 4-Chloro-6-(3-chloro-8-methoxy-2-trifluoromethylimidazo[1,2-a]pyridin-5-yl)isoqionolin-1(2H)-one:

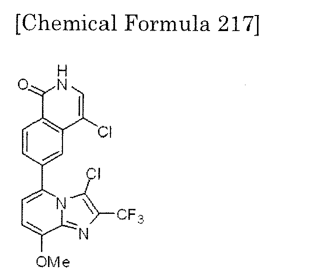

The same procedures used in Example 47 were carried out except for using the compound prepared in Example 20 and the compound prepared in Example 124 to give a desired product as a white solid.

¹H NMR (DMSO-d₆, 400 MHz): δ 4.02 (3H, s), 6.99 (1H, d, J = 7.9 Hz), 7.05 (1H, d, J = 7.9 Hz), 7.57 (1H, d, J = 5.5 Hz), 7.76 (1H, dd, J = 7.9, 1.8 Hz), 7.94 (1H, d, J = 1.8 Hz), 8.29 (1H, d, J = 7.9 Hz), 11.71 (1H, brd, J = 5.5 Hz).
EIMS (+): 427 [M]⁺.
Elemental Analysis: Found: C 50.09%, H 2.54%, N 9.53%; Calculated (for C₁₈ H₁₀ Cl₂ F₃ N₃ O₂ · 0.2H₂ O): C 50.07%, H 2.54%, N 9.73%.

### Example 127: 6-Bromo-1-methylquinolin-2(1H)-one:

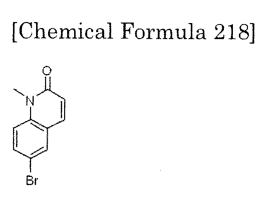

The same procedures used in Example 91 were carried out except for using 6-bromoquinolin-2(1H)-one to give a desired product as yellow powder.

¹H NMR (CDCl₃, 400 MHz): δ 3.70 (3H, s), 6.74 (1H, d, J = 9.8 Hz), 7.24 (1H, d, J = 9.8 Hz), 7.59 (1H, d, J = 9.8 Hz), 7.65 (1H ,dd, J = 9.8, 2.4 Hz), 7.69 (1H, d, J = 2.4 Hz).
EIMS (+): 237 [M]⁺.

### Example 128: 4-Bromo-2-trifluoromethylpyrazolo[1,5-a]pyridine-7-amine:

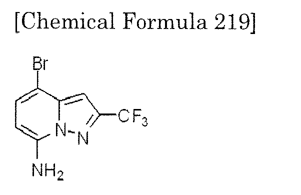

Potassium t-butoxide (570 mg) was dissolved in DMSO (10 mL) under an argon gas atmosphere, formamide (0.202 mL) was added to the solution and the mixture was stirred at ordinary temperature for one hour. A solution of the compound of Example 5 (500 mg) in DMSO (5.0 mL) was added and the resulting mixture was stirred at 60°C for 2.5 hours. A saturated aqueous ammonium chloride solution was added to the reaction liquid and the mixture was extracted three times with ethyl acetate. The combined organic layers were washed with saturated brine and dried over anhydrous sodium sulfate and then the solvent was distilled off under reduced pressure. The resulting residue was dissolved in methanol (10 mL), a solution of sodium hydroxide (203 mg) in water (5.0 mL) was added, and the mixture was stirred at ordinary temperature for 2.5 hours. Water was added to the reaction liquid and the mixture was extracted three times with ethyl acetate. The combined organic layers were washed with saturated brine and dried over anhydrous sodium sulfate and then the solvent was distilled off under reduced pressure. The resulting residue was purified by the silica gel column chromatography (hexane: ethyl acetate = 4:1) to thus give a desired product (315 mg) as yellow powder.

¹H NMR (CDCl₃, 400 MHz): δ 5.26 (2H, s), 6.10 (1H, d, J = 7.9 Hz), 6.84 (1H, s), 7.34 (1H, d, J = 7.9 Hz).
EIMS (+): 279 [M]⁺.

### Example 129: 2-Trifluoromethylimidazo[1,2-a]pyridine-8-amine:

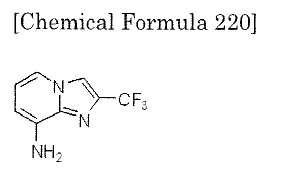

2,3-Diaminopyridine (14.1 g) was suspended in ethanol (260 mL), 3-bromo-1,1,1-trifluoropropan-2-one (24.7 g) was added to this solution and the resulting mixture was stirred for 8 hours under heated and refluxed conditions. The solvent of the reaction liquid was distilled off under reduced pressure, a saturated aqueous solution of sodium hydrogen carbonate was added to the residue and then it was extracted three times with ethyl acetate. The combined organic layers were washed with saturated brine and dried over anhydrous sodium sulfate and then the solvent was distilled off. The resulting residue was purified by the silica gel column chromatography (hexane: ethyl acetate = 3:1) to give a desired product (15.7 g) as a yellow powdery product.

¹H NMR (CDCl₃, 400 MHz): δ 4.59 (2H, s), 6.39 (1H, dd, J = 7.3, 1.2 Hz), 6.72 (1H, t, J = 7.3 Hz), 7.59 (1H, d, J = 7.3 Hz), 7.81 (1H, d, J = 1.2 Hz).
EIMS (+): 201 [M]⁺.

### Example 130: 5-Bromo-2-trifluoromethylimidazo[1,2-a]pyridine-8-amine:

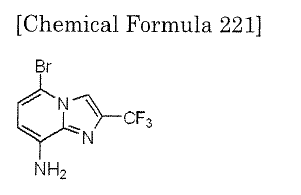

The compound of Example 129 (3.0 g) was dissolved in DMF (100 mL), NBS (2.67 g) was added to this solution at 0°C and the mixture was stirred at 0°C for one hour. Water was added to the reaction liquid and the mixture was extracted three times with ethyl acetate. The combined organic phases were washed with saturated brine and dried over anhydrous sodium sulfate and then the solvent was distilled off under reduced pressure. The resulting residue was purified by the silica gel column chromatography (hexane: ethyl acetate = 4:1) to give a desired product (1.96 g) as yellow powder.

¹H NMR (CDCl₃, 400 MHz): δ 4.61 (2H, s), 6.38 (1H, d, J = 7.9 Hz), 6.93 (1H, d, J =7.9 Hz), 8.02 (1H, d, J = 1.2 Hz).
EIMS (+): 279 [M]⁺.

### Example 131: t-Butyl 2-trifluoromethylimidazo[1,2-a]pyridin-8-yl carbamate:

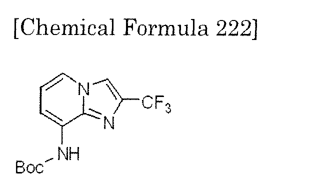

The compound of Example 129 (5.63 g) was dissolved in THF (26 mL) under an argon gas atmosphere, a solution of sodium hexamethyldisilazane in THF (1.0 mol/L, 53.6 mL) was then added to this solution and the mixture was stirred at ordinary temperature for 30 minutes. A solution of t-butyl dicarbonate (5.85 g) in THF (26 mL) was slowly dropwise added to the mixture and then the resulting mixture was stirred at ordinary temperature for 3 hours. A saturated aqueous ammonium chloride solution was added to the reaction liquid and the mixture was extracted three times with ethyl acetate. The combined organic layers were washed with saturated brine and dried over anhydrous sodium sulfate and the solvent was then distilled off under reduced pressure. The resulting residue was purified by the silica gel column chromatography (hexane: ethyl acetate = 9:1) to give a desired product (5.16 g) as yellow powder.

¹H NMR (CDCl₃, 400 MHz): δ 1.54 (9H, s), 6.88 (1H, t, J = 7.3 Hz), 7.79 (1H, d, J = 7.3 Hz), 7.82 (1H, s), 7.86 (1H, s), 7.91 (1H, d, J = 7.3 Hz).
ESIMS (+): 301 [M+H]⁺.

### Example 132: t-Butyl 2-trifluoromethylimidazo[1,2-a]pyridin-8-yl (methyl) carbamate:

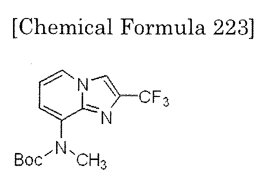

The compound of Example 131 (5.12 g) was dissolved in DMF (100 mL) under an argon gas atmosphere, 60% sodium hydride (884 mg) was added to this solution at 0°C and then the mixture was stirred at ordinary temperature for 30 minutes. Methyl iodide (1.38 mL) was added to the mixture at 0°C and the mixture was stirred at ordinary temperature for one hour. The reaction liquid was poured into ice-water and the resulting precipitates were collected by filtration to give a desired product (5.38 g) as colorless powder.

¹H NMR (CDCl₃, 400 MHz): δ 1.41 (9H, s), 3.41 (3H, s), 6.88 (1H, t, J = 7.3 Hz), 7.20 (1H, d, J = 7.3 Hz), 7.90 (1H, s), 8.03 (1H, dd, J = 7.3, 1.2 Hz).
CIMS (+): 316 [M+H]⁺.

### Example 133: N-Methyl-2-trifluoromethylimidazo[1,2-a]pyridine-8-amine:

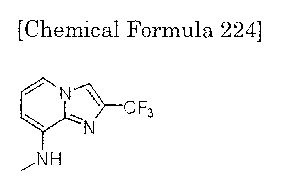

The compound of Example 132 (3.59 g) was dissolved in dichloromethane (60 mL), trifluoroacetic acid (10 mL) was added to this solution and the mixture was stirred at ordinary temperature for 30 minutes. After the solvent of the reaction liquid was distilled off under reduced pressure, a saturated aqueous sodium hydrogen carbonate was added to the liquid and the mixture was extracted three times with ethyl acetate. The combined extracts were washed with saturated brine and dried over anhydrous sodium sulfate and the solvent was then distilled off under reduced pressure. The resulting residue was purified by the silica gel column chromatography (ethyl acetate) to give a desired product (2.56 g) as a green powdery product.

¹H NMR (CDCl₃, 400 MHz): δ 2.98 (3H, d, J = 4.9 Hz), 5.21 (1H, brs), 6.12 (1H, d, J = 7.3 Hz), 6.77 (1H, t, J = 7.3 Hz), 7.50 (1H, d, J = 7.3 Hz), 7.78 (1H, s).
EIMS (+): 215 [M]⁺.

### Example 134: 5-Bromo-N-methyl-2-trifluoromethylimidazo[1,2-a]pyridine-8-amine:

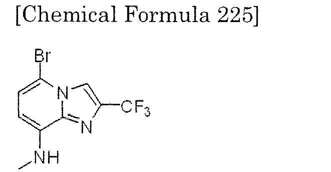

The compound of Example 133 (2.39 g) was dissolved in DMF (80 mL), NBS (2.07 g) was added to this solution and the mixture was stirred at 0°C for 25 minutes. Water was added to this reaction liquid and the mixture was extracted three times with ethyl acetate. The combined extracts were washed with saturated brine and dried over anhydrous sodium sulfate and the solvent was then distilled off under reduced pressure. The resulting residue was purified by the silica gel column chromatography (hexane: ethyl acetate = 20:1) to give a desired product (2.60 g) as colorless powder.

¹H NMR (CDCl₃, 400 MHz): δ 2.97 (3H, d, J = 4.9 Hz), 5.24 (1H, brs), 6.09 (1H, d, J = 7.9 Hz), 6.97 (1H, d, J = 7.9 Hz), 7.99 (1H, s).
EIMS (+): 293 [M]⁺.

### Example 135: t-Butyl 3-chloro-2-(trifluoromethyl)imadazo[1,2-a]pyridin-8-yl (methyl)carbamate:

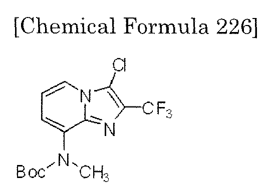

The compound of Example 132 (5.38 g) was dissolved in DMF (100 mL), NCS (2.50 g) was added to this solution and the mixture was stirred at 60°C for one hour. Water was added to this reaction liquid and the mixture was extracted three times with ethyl acetate. The combined organic layers were washed with saturated brine and dried over anhydrous sodium sulfate and the solvent was then distilled off under reduced pressure. The resulting residue was purified by the silica gel column chromatography (hexane: ethyl acetate = 3:1) to give a desired product (5.25 g) as yellow powder.

¹H NMR (CDCl₃, 400 MHz): δ 1.42 (9H, s), 3.40 (3H, s), 7.03 (1H, t, J = 7.3 Hz), 7.27 (1H, d, J = 7.3 Hz), 8.03 (1H, dd, J = 7.3, 1.2 Hz).
CIMS (+): 350[M+H]⁺.

### Example 136: t-Butyl 5-bromo-3-chloro-2-trifluoromethylimidazo[1,2-a]pyridin-8-yl (methyl)carbamate:

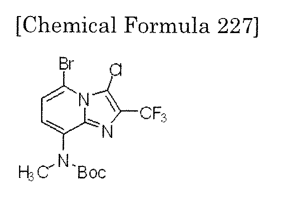

The compound of Example 135 (4.74 g) was dissolved in DMF (100 mL), NBS (3.15 g) was added to this solution and the mixture was stirred at 60°C for 1.5 hours. Water was added to this reaction liquid and the mixture was extracted three times with ethyl acetate. The combined organic layers were washed with saturated brine and dried over anhydrous sodium sulfate and the solvent was then distilled off under reduced pressure. The resulting residue was purified by the silica gel column chromatography (hexane: ethyl acetate = 4:1) to give a desired product (5.32 g) as colorless powder.

¹H NMR (CDCl₃, 400 MHz): δ 1.41 (9H, s), 3.34 (3H, s), 7.06 (1H, d, J = 7.9 Hz), 7.13 (1H, d, J = 7.9 Hz).
CIMS (+): 428 [M+H]⁺.

### Example 137: 6-(3-Chloro-(7-(methylamino)-2-trifluoromethylpyrazolo[1,5-a] pyridin-4-yl)-1-methylquinolin-2(1H)-one:

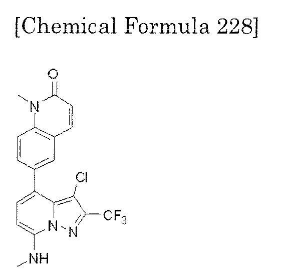

The compound of Example 127 (488 mg), bis(pinacolato) diboron (573 mg), 1,1-bis(diphenylphosphino)ferrocene palladium dichloride dichloromethane complex (167 mg) and potassium 2-ethyl-hexanoate (563 mg) was dissolved in 1,4-dioxane (20 mL) and the resulting mixture was stirred at 80°C for 2 hours. The dioxane of the reaction liquid was distilled off under reduced pressure, water was then added to the resulting residue and the mixture was extracted three times with ethyl acetate. The combined organic layers were washed with saturated brine and dried over anhydrous sodium sulfate. Then the solvent was distilled off under reduced pressure and the resulting residue was purified by the silica gel column chromatography (hexane: ethyl acetate = 1:2) to obtain a crude borate (631 mg).
The compound of Example 23 (200 mg), the crude borate (347 mg) and tetrakis(triphenylphosphine) palladium (70.4 mg) were dissolved in dioxane (6.0 mL) under an argon gas atmosphere, a 2.0 mol/mL aqueous solution of sodium carbonate (1.22 mL) was added and the resulting mixture was stirred at 100°C for 5.5 hours. After water was added to the reaction liquid, the mixture was extracted three times with ethyl acetate. The combined extracts were washed with saturated brine and then dried over anhydrous sodium sulfate and the solvent was distilled off under reduced pressure. The resulting residue was purified by the silica gel column chromatography (hexane: ethyl acetate = 1:1 → 1:2) and then washed with diisopropyl ether to give a desired product (150 mg) as yellow powder.

¹H NMR (CDCl₃, 400 MHz): δ 3.15 (3H, d, J = 4.9 Hz), 3.78 (3H, s), 6.08 (1H, brs), 6.09 (1H, d, J = 7.9 Hz), 6.76 (1H, d, J = 9.2 Hz), 7.21 (1H, d, J = 7.3 Hz), 7.42 (1H, d, J = 7.9 Hz), 7.61-7.64 (2H, m), 7.71 (1H, d, J = 9.2 Hz).
ESIMS(+): 407 [M+H]⁺.
Elemental Analysis: Found: C 55.99%, H 3.45%, N 13.59%; Calculated (for C₁₉ H₁₄ ClF₃ N₄ O): C 56.10%, H 3.47%, N 13.77%.

### Example 138: 4-Chloro-6-(7-(methylamino)-2-trifluoromethylpyrazolo[1,5-a] pyridin-4-yl)isoquinolin-1(2H)-one:

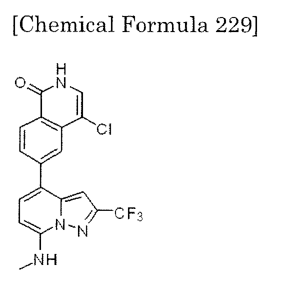

The same procedures used in Example 47 were carried out except for using the compound of Example 21 and the compound of Example 124 to give a desired product as a white solid.

¹H NMR (DMSO-d₆, 400 MHz): δ 3.02 (3H, d, J = 4.9 Hz), 6.30 (1H, d, J = 8.6 Hz), 7.14 (1H, s), 7.52-7.57 (2H, m), 7.69 (1H, d, J = 8.6 Hz), 7.90 (1H, dd, J = 8.6, 1.8 Hz), 7.96 (1H, d, J = 1.8 Hz), 8.32 (1H, d, J = 8.6 Hz), 11.56 (1H, brd, J = 5.5 Hz).
ESIMS (+): 393 [M+H]⁺.
Elemental Analysis: Found: C 54.81%, H 3.11%, N 13.98%; Calculated (for C₁₈ H₁₂ ClF₃ N₄ O): C 55.04%, H 3.08%, N 14.26%.

### Example 139: 4-Chloro-6-(3-chloro-7-(methylamino)-2-trifluoromethylpyrazolo [1,5-a]pyridin-4-yl)isoquinolin-1(2H)-one:

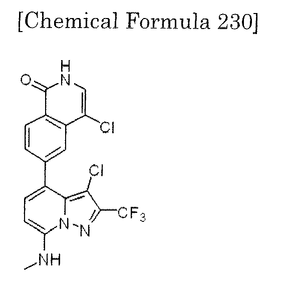

The same procedures used in Example 47 were carried out except for using the compound of Example 23 and the compound of Example 124 to give a desired product as a pale yellow solid.

¹H NMR (DMSO-d₆, 400 MHz): δ 3.01 (3H, d, J = 5.5 Hz), 6.32 (1H, d, J = 7.9 Hz), 7.49 (1H, d, J = 7.9 Hz), 7.52 (1H, s), 7.58 (1H, q, J = 5.5 Hz), 7.66 (1H, dd, J = 8.6, 1.8 Hz), 7.78 (1H, d, J = 1.8 Hz), 8.27 (1H, d, J = 8.6 Hz), 11.59 (1H, s).
ESIMS (+): 427 [M+H]⁺.
Elemental Analysis: Found: C 50.82%, H 2.60%, N 13.05%; Calculated (for C₁₈ H₁₁Cl₂ F₃ N₄O): C 50.61%, H 2.60%, N 13.11%.

### Example 140: 6-(7-Amino-2-trifluoromethylpyrazolo[1,5-a]pyridin-4-yl)quinolin-2(1H)-one:

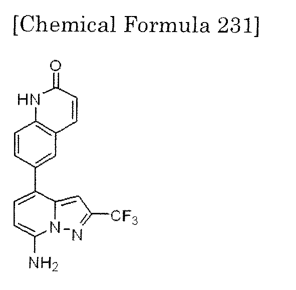

The same procedures used in Example 47 were carried out except for using the compound of Example 130 and the compound of Example 35 to give a desired product as a yellow solid.

¹H NMR (DMSO-d₆, 400 MHz): δ 6.35 (1H, d, J = 7.9 Hz), 6.53 (1H, d, J = 9.8 Hz), 7.06 (2H, brs), 7.12 (1H, s), 7.40-7.43 (2H, m), 7.76 (1H, dd, J = 8.6, 1.8 Hz), 7.92 (1H, s), 8.00 (1H, d, J = 9.8 Hz), 11.81 (1H, brs).
HRESIMS (+): 345.09687 (Calculated for C₁₇ H₁₂ F₃ N₄ O: 345.09632).

### Example 141: 6-(7-Amino-2-trifluoromethylpyrazolo[1,5-a]pyridin-4-yl)-4-chloro isoquinolin-1(2H)-one:

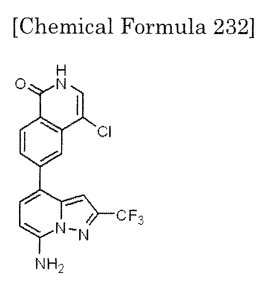

The same procedures used in Example 47 were carried out except for using the compound of Example 130 and the compound of Example 124 to give a desired product as yellow powder.

¹H NMR (DMSO-d₆, 400 MHz): δ 6.41 (1H, d, J = 7.9 Hz), 7.11 (1H, s), 7.32 (2H, s), 7.52 (1H, d, J = 6.1 Hz), 7.63 (1H, d, J = 7.9 Hz), 7.89 (1H, dd, J = 7.9, 1.8 Hz), 7.95 (1H, d, J = 1.8 Hz), 8.32 (1H, d, J = 7.9 Hz), 11.55 (1H, d, J = 6.1 Hz).
HRESIMS (+): 379.05792 (Calculated for C₁₇H₁₁ClF₃N₄O: 379.05735).

### Example 142: 6-(3-Chloro-8-methylamino-2-trifluoromethylimidazo[1,2-a]pyridin-5-yl)quinolin-2(1H)-one:

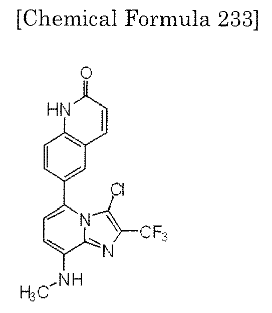

The compound of Example 136 (300 mg) and the compound of Example 35 (190 mg) were dissolved in 1,4-dioxane (7.0 mL) under an argon gas atmosphere, then to this solution were added tetrakis(triphenylphosphine) palladium (80.9 mg) and a 2.0 mol/L aqueous solution of sodium carbonate (1.4 mL), and the resulting mixture was stirred at 100°C for 10 hours. Water was added to the reaction liquid and the mixture was extracted three times with ethyl acetate. The combined organic layers were washed with saturated brine and dried over anhydrous sodium sulfate and the solvent was then distilled off under reduced pressure. The resulting residue was purified by the silica gel column chromatography (hexane: ethyl acetate = 1:4), the resulting amorphous substance was dissolved in dichloromethane (5.0 mL), trifluoroacetic acid (2.0 mL) was added to the solution and the mixture was stirred at ordinary temperature for one hour. The solvent of the reaction liquid was distilled off under reduced pressure, a saturated aqueous sodium hydrogen carbonate solution was added to the resulting residue and the mixture was extracted three times with ethyl acetate. The combined organic layers were washed with saturated brine and dried over anhydrous sodium sulfate and the solvent was then distilled off under reduced pressure. The resulting residue was purified by the aminated silica gel column chromatography (ethyl acetate) and then recrystallized (ethyl acetate) to give a desired product (63.0 mg) as colorless powder.

¹H NMR (DMSO-d₆, 400 MHz): δ 2.86 (3H, d, J = 4.9 Hz), 6.29 (1H, d, J = 7.9 Hz), 6.55 (1H, dd, J = 9.8, 1.8Hz), 6.62 (1H, q, J = 4.9 Hz), 6.83 (1H, d, J = 7.9 Hz), 7.32 (1H, d, J = 7.9Hz), 7.59 (1H, dd, J = 7.9, 1.8Hz), 7.77 (1H, s), 7.93 (1H, d, J = 9.8Hz), 11.89 (1H, s).
HRESIMS (+): 393.07244 (Calculated for C₁₈ H₁₃ ClF₃ N₄ O: 393.07300).
Elemental Analysis: Found: C 54.94 %, H 3.12 %, N 14.10 %; Calculated (for C₁₈H₁₂ClF₃N₄O): C 55.04 %, H 3.08 %, N 14.26 %.

### Example 143: 6-(3-Chloro-8-methylamino-2-trifluoromethylimidazo[1,2-a]pyridin-5-yl)-3,4-dihydroisoquinolin-1(2H)-one:

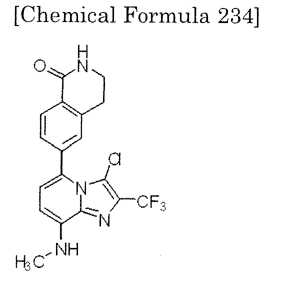

The same procedures used in Example 142 were carried out except for using 6-(4,4,5,5-tetramethyl[1,3,2]dioxaborolan-2-yl)-3,4-dihydroisoquinolin-1(2H)-one and the compound of Example 136 to give a desired product as white powder.

¹H NMR (DMSO-d₆, 400 MHz): δ 2.86 (3H, d, J = 4.9 Hz), 2.93-2.95 (2H, m), 3.39-3.41 (2H, m), 6.30 (1H, d, J = 7.9 Hz), 6.70 (1H, q, J = 4.9 Hz), 6.87 (1H, d, J = 7.9 Hz), 7.42-7.43 (2H, m), 7.87 (1H, d, J = 7.9 Hz), 8.00 (1H, s).
HRESIMS (+): 395.08888 (Calculated for C₁₈ H₁₃ ClF₃ N₄ O: 395.08865).
Elemental Analysis: Found: C 54.19 %, H 3.59 %, N 13.63 %; Calculated (for C₁₈ H₁₂ ClF₃ N₄ O · 0.3H2 O): C 54.02 %, H 3.68 %, N 14.00 %.

### Example 144: 4-Chloro-6-(8-(methylamino)-2-trifluoromethylimidazo[1,2-a] pyridin-5-yl)isoquinolin-1(2H)-one:

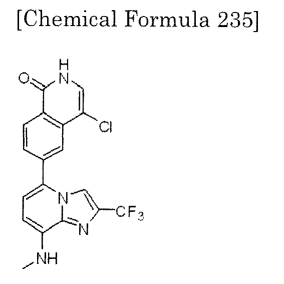

The same procedures used in Example 47 were carried out except for using the compound of Example 134 and the compound of Example 124 to give a desired product as a white solid.

¹H NMR (DMSO-d₆, 400 MHz): δ 2.88 (3H, d, J = 4.9 Hz), 6.31 (1H, d, J = 7.9 Hz), 6.77 (1H, q, J = 4.9 Hz), 7.09 (1H, d, J = 7.9 Hz), 7.56 (1H, s), 7.89 (1H, dd, J = 8.6, 1.8 Hz), 7.94 (1H, d, J = 1.8 Hz), 8.35-8.37 (2H, m), 11.63 (1H, s).
HRESIMS (+): 393.07368 (Calculated for C₁₈ H₁₃ ClF₃ N₄ O: 393.07300).
Elemental Analysis: Found: C 54.83%, H 3.15%, N 14.08%; Calculated (for C₁₈ H₁₂ ClF₃ N₄ O): C 55.04%, H 3.08%, N 14.26%.

### Example 145: 4-Chloro-6-(3-chloro-8-(methylamino)-2-trifluoromethylimidazo [1,2-a]pyridin-5-yl)isoquinolin-1(2H)-one:

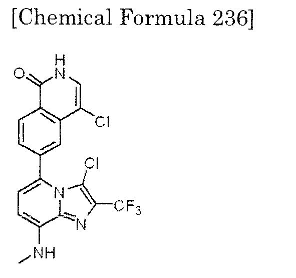

The same procedures used in Example 142 were carried out except for using the compound of Example 136 and the compound of Example 124 to give a desired product as yellow powder.

¹H NMR (DMSO-d₆, 400 MHz): δ 2.88 (3H, d, J = 4.9 Hz), 6.33 (1H, d, J = 7.9 Hz), 6.78 (1H, q, J = 4.9 Hz), 6.98 (1H, d, J = 7.9 Hz), 7.55 (1H, s), 7.70 (1H, dd, J = 8.6, 1.8 Hz), 7.85 (1H, d, J = 1.8 Hz), 8.26 (1H, d, J = 8.6 Hz), 11.65 (1H, s).
HRESIMS (+): 427.03451 (Calculated for C₁₈ H₁₂ Cl₂ F₃ N₄ O: 427.03403).
Elemental Analysis: Found: C 50.13%, H 2.59%, N 12.93%; Calculated (for C₁₈ H₁₁ Cl₂ F₃ N₄ O · 1/5H₂ O): C 50.18%, H 2.67%, N 13.00%.

### Example 146: 6-(8-Amino-2-trifluoromethylimidazo[1,2-a]pyridin-5-yl)-4-chloro-isoquinolin-1(2H)-one:

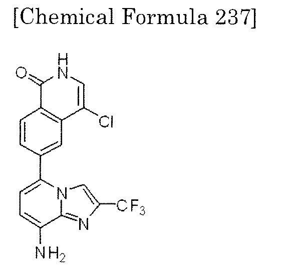

The same procedures used in Example 47 were carried out except for using the compound of Example 130 and the compound of Example 124 to give a desired product as yellow powder.

¹H NMR (DMSO-d₆, 400 MHz): δ 6.22 (2H, s), 6.53 (1H, d, J = 7.9 Hz), 7.02 (1H, d, J = 7.9 Hz), 7.56 (1H, s), 7.88 (1H, dd, J = 7.9, 1.8 Hz), 7.93 (1H, d, J = 1.8 Hz), 8.34-8.36 (2H, m), 11.63 (1H, s).
ESIMS (+): 379 [M+H]⁺.
Elemental Analysis: Found: C 53.78%, H 2.79%, N 14.30%; Calculated (for C₁₇ H₁₀ ClF₃ N₄ O · 1/5H₂ O): C 53.40%, H 2.74%, N 14.65%.

### Test Example 1: Phosphodiesterase 4 Inhibitory Activity:

The cDNA of the PDE4B catalytic zone (hereunder referred to as "PDE4Bcat") was isolated from the human-derived RNA using the RT-PCR technique. The isolated cDNA fragment was introduced into insect cells Sf9 using Gateway system (available from Invitrogen Company) and Bac-to-Bac™ Baculovirus Expression system (available from Invitrogen Company) to thus make the cells express the target PDE protein. This recombinant PDE4Bcat was obtained by cultivating the Sf9 cells which could highly expressed this PDE protein and subjecting the resulting culture supernatant or cell-extract to the purification by the ion-exchange chromatography and the resulting purified PDE4Bcat was used in the following experiments.
The compound to be examined was stepwise diluted 4 times with a 15% DMSO solution, starting from a solution of the compound having an initial concentration of 4 mmol/L to thus prepare solutions thereof having a concentration ranging from 15 nmol/L to 4mmol/L (in the following experiments, the final concentration of the compound ranged from 1.5 nmol/L to 400 µ mol/L). To the wells of a 96-well plate were added 10 µL each of these solutions of the compound as an analyte, [³H]-cAMP diluted with a buffer solution (40 mmol/L Tris-HCl (pH 7.4) and 10 mmol/L of MgCl₂) and 40µL of the human-derived recombinant PDE protein in an amount of 2x10⁻⁶ units (wherein one unit means the amount of PDE required for decomposing 1 µmol/L of cAMP, within one minute, at a pH value of 7.5 and a temperature of 30°C) and they were reacted with each other at 30°C for 20 minutes. After they were reacted at 65°C for additional 2 minutes, 25 µL each of a 1 mg/mL 5' nucleotidase solution (Crotalus atrox venom available from Sigma Company) was added to each well and the reaction was continued at 30°C for 10 minutes. After the completion of the reaction, 200 µL each of Dowex solution (300 mg/mL Dowex 1x8-400 (available from Sigma Aldrich Company), 33% Ethanol) was added to each well, followed by the oscillation mixing thereof at 4°C for 20 minutes, the addition of 200 µL each of MicroScint 20 (available from Packard Company) and the measurement using a scintillation counter (Topcount, available from Packard Company). The IC₅₀ value of each test compound was calculated by the use of GraphPad Prism v3. 03 (available from GraphPad Software Company).

In this connection, the results thus obtained are expressed as follows: IC₅₀ value ≧1 µmol/L (-); 1 µmol/L> IC₅₀ value≧0.1 µmol/L (+); 0.1 µmol/L> IC₅₀ value≧0.01 µ mol/L (++); 0.01 µmol/L> IC₅₀ value (+++).
The results obtained are summarized in the following Table 1.

**Table 1**

| Ex. No | IC₅₀(µmol/L) PDE4 | Ex. No | IC₅₀(µmol/L) PDE4 | Ex. No | IC₅₀(µmol/L) PDE4 |
|---|---|---|---|---|---|
| 50 | +++ | 71 | +++ | 116 | ++ |
| 51 | ++ | 72 | ++ | 117 | ++ |
| 52 | ++ | 74 | ++ | 118 | ++ |
| 54 | +++ | 80 | ++ | 119 | +++ |
| 56 | ++ | 83 | ++ | 120 | ++ |
| 57 | ++ | 86 | +++ | 121 | ++ |
| 58 | ++ | 87 | ++ | 122 | ++ |
| 59 | ++ | 88 | ++ | 125 | +++ |
| 60 | +++ | 89 | +++ | 126 | +++ |
| 61 | +++ | 90 | ++ | 137 | +++ |
| 62 | +++ | 91 | +++ | 138 | +++ |
| 63 | +++ | 92 | ++ | 139 | +++ |
| 64 | +++ | 93 | ++ | 140 | +++ |
| 65 | +++ | 94 | +++ | 141 | ++ |
| 66 | +++ | 102 | ++ | 142 | +++ |
| 67 | ++ | 111 | ++ | 143 | +++ |
| 68 | ++ | 112 | +++ | 144 | +++ |
| 69 | ++ | 114 | +++ | 145 | +++ |
| 70 | +++ | 115 | +++ | 146 | ++ |

### Test Example 2: Phosphodiesterase 3-Inhibitory Activity:

The cDNA of the PDE3A catalytic zone (hereunder referred to as "PDE3Acat") was isolated from the human-derived RNA using the RT-PCR technique. Each cDNA fragment thus isolated was introduced into insect cells Sf9 using Gateway system (available from Invitrogen Company) and Bac-to-Bac™ Baculovirus Expression system (available from Invitrogen Company) to thus make the cells express the target PDE protein. This recombinant PDE3Acat was obtained by cultivating the Sf9 cells which could highly expressed the PDE protein and subjecting the resulting culture supernatant or cell-extract to the purification according to the ion-exchange chromatography technique and the resulting purified PDE3Acat was used in the following experiments.

The compound to be examined was stepwise diluted 4 times with a 15% DMSO solution, starting from a solution of the compound having an initial concentration of 4 mmol/L to thus prepare solutions thereof having a concentration ranging from 15 nmol/L to 4mmol/L (in the following experiments, the final concentration of the compound ranged from 1.5 nmol/L to 400 µmol/L). To the wells of a 96-well plate, there were added 10 µL each of these solutions of the compound as an analyte, [³H]-cAMP diluted with a buffer (40 mmol/L Tris-HCl (pH 7.4) and 10 mmol/L of MgCl₂) and 40 µL of the human-derived recombinant PDE protein in an amount of 2x10⁻⁶ units (wherein one unit means the amount of PDE required for decomposing 1µ mol/L of cAMP, within one minute, at a pH value of 7.5 and a temperature of 30°C) and they were reacted with each other at 30°C for 20 minutes. After they were reacted at 65°C for additional 2 minutes, 25 µL of a 1 mg/mL 5' nucleotidase solution (Crotalus atrox venom available from Sigma Company) was added to the well and the reaction was continued at 30°C for 10 minutes.
After the completion of the reaction, 200 µL of Dowex solution (300 mg/mL Dowex 1x8-400 (available from Sigma Aldrich Company), 33% Ethanol) was added to the well, followed by the oscillation mixing thereof at 4°C for 20 minutes, the addition of 200 µL each of MicroScint 20 (available from Packard Company) and the measurement using a scintillation counter (Topcount, available from Packard Company). The IC₅₀ value of each test compound was calculated by the use of GraphPad Prism v3. 03 (available from GraphPad Software Company).
As a result of the measurements according to the foregoing method, it was found that the compounds prepared in, for instance, Examples 58, 59, 60, 63, 64, 89, 102, 115, 121, 142 and 143 showed the IC₅₀ values of >1µmol/L.

### Test Example 3: Histamine-Induced Broncoconstrictive Reaction in Guinea Pig

Guinea pigs were anesthetized with pentobarbital (30 mg/kg, i.p.), and there were inserted a cannula for the intravenous administration into the left external jugular vein; a cannula for the blood collection and for the blood pressure measurement into the right internal jugular vein; and a tracheal cannula into thetrachea, respectively. Then the artificial respiration was practiced on the guinea pigs under the conditions of 60 times/min and 10 mL/kg/stroke, the amount of the air overflowing through the collateral of the tracheal cannula (air flow) was determined by the bronchospasm transducer (Ugo-Basile) and the data thus obtained were stored in a computer through Power Lab (AD Instruments Japan). After the animals were immobilized with gallamine (10 mg/kg, i.v.), histamine (12.5 µg/kg, i.v.) was administered to each test animal every 10 minutes. After the stabilization of the broncoconstriction induced by histamine, a candidate compound (0.3 mg/kg, through the i.v. route) was administered to each of the test animals, the broncoconstriction reaction due to the action of histamine after 30 seconds from the administration of the compound was determined to thus examine the inhibitory effect of the compound on the broncoconstriction. The broncoconstriction was recorded in terms of the measured air flow value and the result was expressed in terms of the ratio of the maximum of the air flow due to the action of histamine observed after 30 seconds from the administration of the compound to the maximum thereof observed before the administration thereof. Incidentally, each candidate compound was used in the form of a solution in DMSO.
As a result of the determination according to the foregoing method, it was found that the compounds prepared in, for instance, Examples 50, 52, 53, 54, 56, 57, 59, 67, 68, 69, 88, 90, 91, 112, 118 and 120 showed such an inhibitory effect of not less than 70%.

### Test Example 4: LPS-induced Pulmonary Inflammation in Rats:

One hour before the inhalation of lipopolysaccharide from E. coli serotype 055:B5 (LPS), each candidate compound was orally administered to each test rat in a dose of 3 mg/kg, and then the test animal was allowed to inhale an LPS solution (50 mL) over 30 minutes, while the solution was atomized using a nebulizer. After 3 hours from the LPS-inhalation, the rats were euthanized by the administration of a 20% urethane solution (5 ml/rat, i.p.). The bronchial cannula was inserted through the trachea. Broncoalveolar lavage was performed by injecting 5 ml physiological saline, three consecutive times using a 5 mL volume syringe. The operations were repeated twice and these physiological saline samples were recovered as broncoalveolar lavage fluid (BALF). Each of the BALF thus recovered was centrifuged at 1200 rpm and 4°C for 10 minutes (Hirtachi; himac CR 5 DL), the resulting residue was again suspended in 10 mL of 0.1% bovine serum albumin-containing physiological saline, an equivalent volume of Turk's diluting fluid was added to the suspension to thus stain the leukocytes present therein and the total number of leukocytes was determined under the microscopic observation to thus calculate the rate of inhibition.
As a result of the determination according to the foregoing method, it was found that the compounds prepared in, for instance, Examples 58, 59, 60, 62, 63, 64, 89, 102, 115, 121, 142 and 143 showed such an inhibitory effect of not less than 60%.

As has been described above, the compounds represented by the general formula (1) according to the present invention exhibit a PDE-inhibitory activity and the effectiveness thereof has been confirmed in a variety of animal test models.

### Industrial Applicability:

As has been described above in detail, according to the present invention, it has been found that a novel heterocyclic biaryl derivative and an addition salt thereof exhibit excellent PDE inhibitory activity. Such a compound having a PDE inhibitory activity is useful as an agent for treating angina, heart failure and hypertension; a platelet aggregation inhibitor; an agent for preventing or treating bronchial asthma, chronic obstructive pulmonary diseases (COPD), interstitial pneumonia, interstitial cystitis, allergic conjunctivitis, allergic rhinitis, atopic dermatitis, osteoporosis, osteoarthritis of knee, rheumatoid arthritis, non-alcoholic fatty liver, multiple sclerosis, Crohn's disease, inflammatory colitis, a variety of mental disorders such as Huntington's disease, Alzheimer's disease, dementia, Parkinson's disease, depression and schizophrenia, obesity and metabolic syndromes; as well as an agent for the treatment of the male impotence.

## Claims

1. A heterocyclic biaryl derivative represented by the following general formula (1), wherein the Heterocycle 1 and the Heterocycle 2 are directly bonded together, an optically active derivative thereof or a pharmaceutically acceptable salt or hydrate thereof: 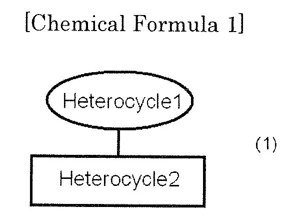 [wherein the Heterocycle 1 is a hetero ring represented by the following general formula (2): 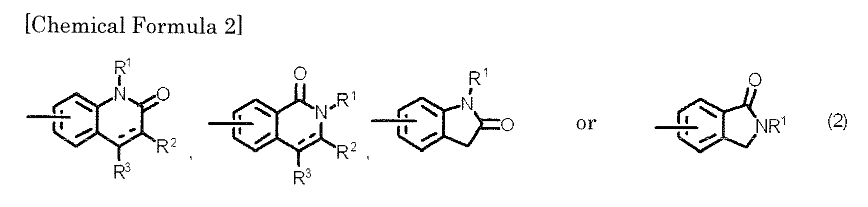 (in the formula (2), R¹ represents a hydrogen atom or an alkyl group having 1 to 6 carbon atoms which may have a substituent; R² and R³ may be the same or different and each represents a hydrogen atom, an alkyl group having 1 to 6 carbon atoms or a halogen atom;
[Chemical Formula 3] - - - - - -
represents a single bond or a double bond); and wherein the Heterocycle 2 is a hetero ring represented by the following general formula (3): 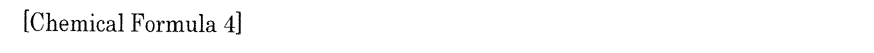 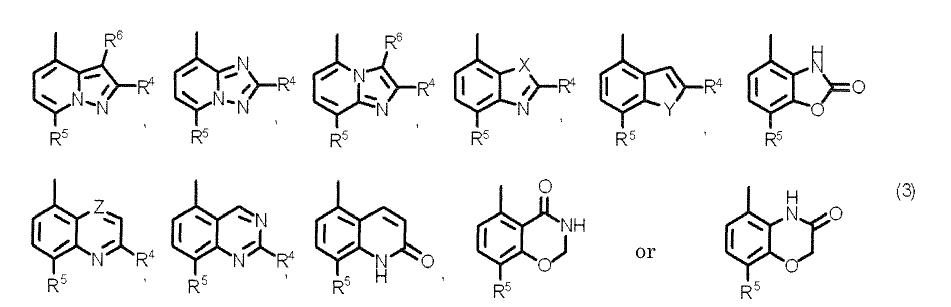 (in the formula (3), R⁴ represents a hydrogen atom, an alkyl group which may be substituted with a halogen atom and which has 1 to 6 carbon atoms or a cycloalkyl group having 3 to 8 carbon atoms, R⁵ represents an alkoxy group having 1 to 6 carbon atoms, an amino group or an alkylamino group having 1 to 6 carbon atoms, R⁶ represents a hydrogen atom or a halogen atom, X represents NH, O or S, Y represents O or S, and Z represents CH or N)].

2. The heterocyclic biaryl derivative, an optically active derivative thereof or a pharmaceutically acceptable salt or hydrate thereof as set forth in claim 1, wherein the Heterocycle 1 of the compound represented by the general formula (1) is a group represented by the following general formula (2a): 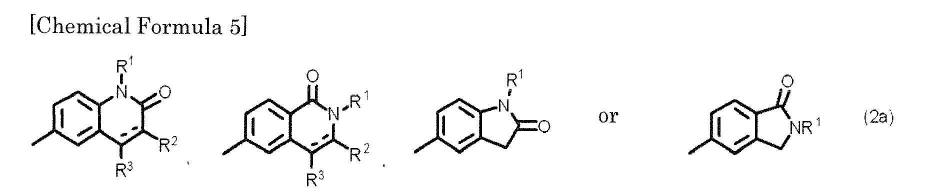 [wherein R¹, R², R³ and
[Chemical Formula 6] - - - - - -
are the same as those defined above].

3. The heterocyclic biaryl derivative, an optically active derivative thereof or a pharmaceutically acceptable salt or hydrate thereof as set forth in claim 1, wherein the Heterocycle 1 of the compound represented by the general formula (1) is a group represented by the following general formula: 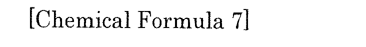 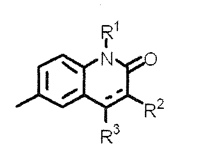 [wherein R¹, R², R³ and
[Chemical Formula 8] - - - - - -
are the same as those defined above].

4. The heterocyclic biaryl derivative, an optically active derivative thereof or a pharmaceutically acceptable salt or hydrate thereof as set forth in claim 1, wherein the Heterocycle 1 of the compound represented by the general formula (1) is a group represented by the following general formula: 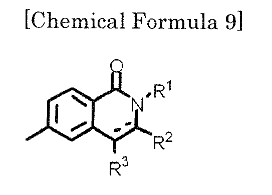 [wherein R¹, R², R³ and
[Chemical Formula 10] - - - - - -
are the same as those defined above].

5. The heterocyclic biaryl derivative, an optically active derivative thereof or a pharmaceutically acceptable salt or hydrate thereof as set forth in claim 1, wherein the Heterocycle 1 of the compound represented by the general formula (1) is a group represented by the following general formula: 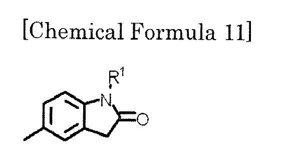 [wherein R¹ is the same as that defined above].

6. The heterocyclic biaryl derivative, an optically active derivative thereof or a pharmaceutically acceptable salt or hydrate thereof as set forth in claim 1, wherein the
Heterocycle 1 of the compound represented by the general formula (1) is a group represented by the following general formula: 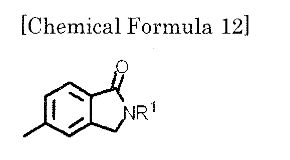 [wherein R¹ is the same as that defined above].

7. The heterocyclic biaryl derivative, an optically active derivative thereof or a pharmaceutically acceptable salt or hydrate thereof as set forth in any one of claims 1 to 6, wherein the Heterocycle 2 of the compound represented by the general formula (1) is a hetero ring represented by the following general formula (3a): 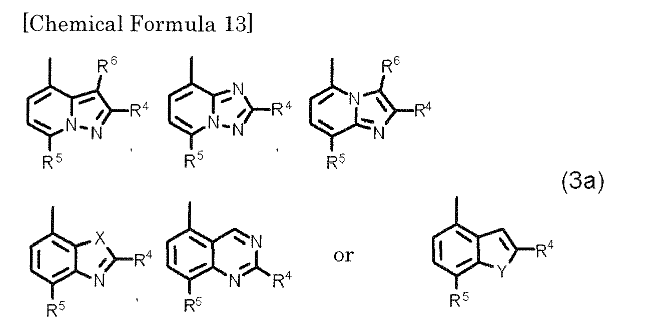 (wherein R⁴, R⁵, R⁶, X and Y are the same as those defined above).

8. The heterocyclic biaryl derivative, an optically active derivative thereof or a pharmaceutically acceptable salt or hydrate thereof as set forth in claim 1, wherein the compound of the formula (1) is one represented by the following general formula: 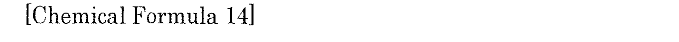 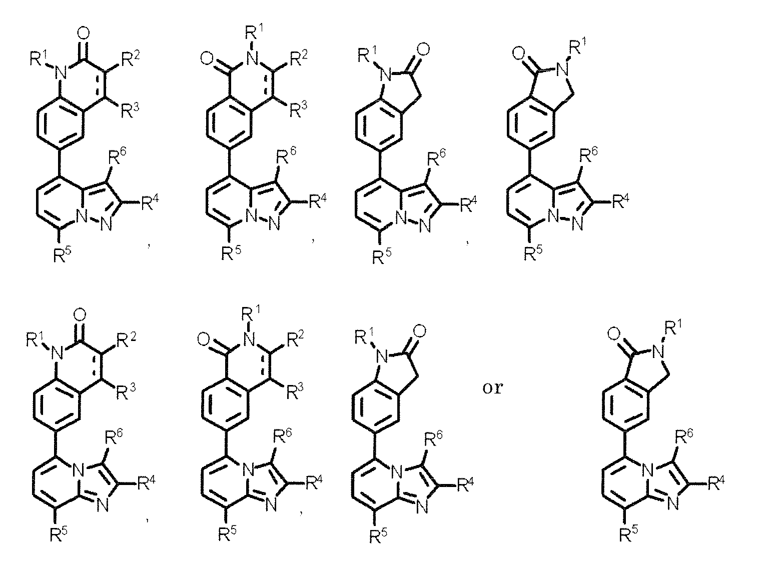 [In the formula, R¹, R², R³, R⁴, R⁵, R⁶, and
[Chemical Formula 15] - - - - - -
are the same as those defined above].

9. The heterocyclic biaryl derivative, an optically active derivative thereof or a pharmaceutically acceptable salt or hydrate thereof as set forth in any one of claims 1 to 8, wherein the substituent R⁵ of the compound represented by the general formula (1) is an alkoxy group having 1 to 6 carbon atoms.

10. The heterocyclic biaryl derivative, an optically active derivative thereof or a pharmaceutically acceptable salt or hydrate thereof as set forth in any one of claims 1 to 8, wherein the substituent R⁵ of the compound represented by the general formula (1) is an alkylamino group having 1 to 6 carbon atoms.

11. The heterocyclic biaryl derivative, optically active derivatives thereof or pharmaceutically acceptable salts or hydrates thereof as set forth in claim 1, wherein the compound of the formula (1) is:
• 6-(7-Methoxy-2-trifluoromethylpyrazolo[1,5-a]pyridin-4-yl)quinolin-2(1H)-one;
• 6-(7-Methoxy-2-trifluoromethylpyrazolo[1,5-a]pyridin-4-yl)-3,4-dihydroiso-quinolin-1(2H)-one;
• 5-(7-Methoxy-2-trifluoromethylpyrazolo[1,5-a]pyridin-4-yl)isoindolin-1-one;
• 4-Chloro-6-(7-methoxy-2-trifluoromethylpyrazolo[1,5-a]pyridin-4-yl)isoquinolin-1(2H)-one;
• 6-(3-Chloro-7-methoxy-2-trifluoromethylpyrazolo[1,5-a]pyridin-4-yl)-3,4-dihydro isoquinolin-1(2H)-one;
• 6-(7-Methylamino-2-trifluoromethylpyrazolo[1,5-a]pyridin-4-yl)-3,4-dihydroiso quinolin-1(2H)-one;
• 6-(7-Methylamino-2-trifluoromethylpyrazolo[1,5-a]pyridin-4-yl)quinolin-2(1H)- one;
• 6-(3-Chloro-8-methoxy-2-trifluoromethylimidazo[1,2-a]pyridin-5-yl)-quinolin-2(1H)-one;
• 6-(7-Methoxy-2-trifluoromethylpyrazolo[1,5-a]pyridin-4-yl)-1-methyl-3,4-dihydro quinolin-2(1H)-one;
• 6-(3-Chloro-7-methoxy-2-trifluoromethylpyrazolo[1,5-a]pyridin-4-yl)-1-methyl quinolin-2(1H)-one;
• 6-(2-Cyclopropyl-7-methoxypyrazolo[1,5-a]pyridin-4-yl)-3,4-dihydroisoquinolin-1(2H)-one;
• 6-(3-Chloro-(8-methylamino-2-trifluoromethylimidazo[1,2-a]pyridin-5-yl) quinolin-2(1H)-one; or
• 6-(3-Chloro-(8-methylamino-2-trifluoromethylimidazo[1,2-a]pyridin-5-yl)-3,4-di hydroisoquinolin- 1(2H) -one.

12. A phosphodiesterase (PDE) inhibitor comprising, as an effective component, a heterocyclic biaryl derivative, an optically active derivative thereof, or a pharmaceutically acceptable salt or hydrate thereof as set forth in any one of claims 1 to 11.

13. A pharmaceutical agent comprising, as an effective component, a heterocyclic biaryl derivative, an optically active derivative thereof, or a pharmaceutically acceptable salt or hydrate thereof as set forth in any one of claims 1 to 11.

14. The pharmaceutical agent as set forth in claim 13 for preventing or treating angina, heart failure, hypertension, bronchial asthma, chronic obstructive pulmonary diseases (COPD), interstitial pneumonia, interstitial cystitis, allergic conjunctivitis, allergic rhinitis, atopic dermatitis, osteoporosis, rheumatoid arthritis, osteoarthritis of knee, non-alcoholic fatty liver, multiple sclerosis, Crohn's disease, inflammatory colitis, Huntington's disease, Alzheimer's disease, dementia, Parkinson's disease, depression, schizophrenia, obesity and metabolic syndromes.
